# EUROPÄISCHE PATENTSCHRIFT

(11) **EP 1 472 354 B1**
(45) Veröffentlichungstag und Bekanntmachung des Hinweises auf die Patenterteilung: **19.09.2012**
(21) Anmeldenummer: 03701537.7
(22) Anmeldetag: 22.01.2003
(51) Int. Cl.: C12N 15/52, C12N 15/81, C12N 9/90, C12N 9/02, C12P 33/00

(54) **VERFAHREN ZUR HERSTELLUNG VON 7-DEHYDROCHOLESTEROL IN TRANSGENEN ORGANISMEN**
METHOD FOR THE PRODUCTION OF 7-DEHYDROCHOLESTEROL IN TRANSGENIC ORGANISMS
PROCEDE DE PRODUCTION DE 7-DEHYDROCHOLESTEROL DANS DES ORGANISMES TRANSGENIQUES

(30) Priorität: 29.01.2002 DE 10203352
(43) Veröffentlichungstag der Anmeldung: 03.11.2004
(73) Patentinhaber: Organo Balance GmbH, 13355 Berlin (DE)
(72) Erfinder: LANG, Christine, 13355 Berlin (DE); VEEN, Markus, 13407 Berlin (DE)
(74) Vertreter: Jungblut, Bernhard Jakob
(86) Internationale Anmeldenummer: PCT/EP2003/000592
(87) Internationale Veröffentlichungsnummer: WO 2003/064650

(56) Entgegenhaltungen:
- WO-A-98/45457
- WO-A-02/061072
- US-A- 5 480 805
- SILVE S. ET AL.: "Emopamil binding Protein, a Mammalian Protein that Binds a Series of Structurally Diverse Neuroprotective Agents, Exhibits delta8-delta7 Sterol Isomerase Activity in Yeast" THE JOURNAL OF BIOLOGICAL CHEMISTRY, Bd. 271, Nr. 37, 13. September 1996 (1996-09-13), Seiten 22434-22440, XP002245941
- HUSSELSTEIN T. ET AL. : "delat7-Sterol-C5-desaturase: molecular characterization and functional expression of wild-type and mutant alleles" PLANT MOLECULAR BIOLOGY, Bd. 39, 1999, Seiten 891-906, XP002245942
- LEES N D ET AL: "Cloning of the late genes in the ergosterol Biosynthetic pathway of Saccharomyces cerevisiae" LIPIDS, CHAMPAIGN, IL, US, Bd. 30, Nr. 3, 1. März 1995 (1995-03-01), Seiten 221-226, XP002094332 ISSN: 0024-4201
- NISHI S.: "cDNA cloning of the mammalian sterol C5-desaturase and the expression in yeast mutant" BIOCHIMICA ET BIOPHYSICA ACTA, Bd. 1490, 2000, Seiten 106-108, XP002245943 in der Anmeldung erwähnt

## Beschreibung

Die vorliegende Erfindung betrifft ein Verfahren zur Herstellung von 7-Dehydrocholesterol durch Kultivierung von Organismen, insbesondere Hefen. Ferner betrifft die Erfindung die zur Herstellung der genetisch veränderten Organismen benötigten Nukleinsäurekonstrukte, sowie die genetisch veränderten Organismen, insbesondere Hefen selbst.

7-Dehydrocholesterol, auch als Cholesta-5,7-dienol oder Provitamin D3 bezeichnet, dessen biosynthetische Zwischenprodukte des Sterolstoffwechsels, wie beispielsweise Zymosterol, Farnesol, Geraniol, Squalen, Lanosterol, Cholesta 5,7,24-trienol und Cholesta-5,7,22,24-tetraenol sowie dessen biosynthetischen Folgeprodukte des Sterolstoffwechsels, wie Vitamin D₃ und Cholesterol sind Verbindungen mit hohem wirtschaftlichen Wert.

Die wirtschaftliche Bedeutung von 7-Dehydrocholesterol liegt vor allem in der Gewinnung von Vitamin D3 aus 7-Dehydrocholesterol über UV-Bestrahlung.

Ein wirtschaftliches Verfahren zur Herstellung von 7-Dehydrocholesterol ist daher von großer Bedeutung.

Besonders wirtschaftliche Verfahren sind biotechnologische Verfahren unter Ausnutzung von durch genetische Veränderung optimierter Organismen, die 7-Dehydrocholesterol herstellen.

Während der Sterolstoffwechsel in Bakterien, Pilzen, Hefen und einigen Insekten im wesentlichen von Zymosterol über Fecosterol, Episterol, Ergosta-5,7-dienol und Ergosta-5,7,22,24-tetraen-3β-ol zu Ergosterol (Provitamin D₂) führt, führt der Sterolstoffwechsel in Säugern im wesentlichen von Zymosterol über Cholesta-7,24-dienol, Lathosterol zu 7-Dehydro-Cholsterol (Provitamin D₃).

7-Dehydro-Cholsterol (Provitamin D₃) wird durch die 7-Dehydro-Cholesterolreduktase in Cholesterol und Cholesterol in Steroidhormone, Corticoide und Gallensäuren wie Progesteron, Testosteron, Estradiol, Aldosteron, Cortison und Cholat überführt.

Einige Gene des 7-Dehydrocholsterol-Stoffwechsels in Säugern sind bekannt und kloniert, wie beispielsweise

Nukleinsäuren kodierend eine humane Δ8-Δ7-Isomerase (auch Emopamil Binding Protein (EBP) genannt), ACCESSION NM_006579 und eine murine Δ8-Δ7-Isomerase (Braverman,N. et al.,(1999): Mutations in the gene encoding 3beta-hydroxysteroid-delta8,delta7-isomerase cause X-linked dominant Conradi-Hunermann syndrome.Nat.Genet.22(3),291-294.,

Nukleinsäuren kodierend eine humane Δ5-Desaturase (auch Sterol-C5-Desaturase genannt), ACCESSION AB016247 und eine murine Δ5-Desaturase (Nishi,S. et al., (2000):cDNA cloning of the mammalian sterol C5-desaturase and the expression in yeast mutant. Biochim.Biophys.Acta 1490(1-2),106-108),

Nukleinsäuren kodierend eine humane Δ24-Reduktase (auch 24-Dehydrocholesterol Reduktase (DHCR24) genannt), ACCESSION NM_014762 und eine murine Δ24-Reduktase (Waterham,H.R. et al. (2001): Mutations in the 3beta-hydroxysterol Delta24-reductase gene cause desmosterolosis,an autosomal recessive disorder of cholesterol biosynthesis. Am.J.Hum.Genet.69(4),685-694 und

Nukleinsäuren kodierend eine humane Sterol-Acyltransferase (Chang, C.C. et al.,Molecular cloning and functional expression of human acyl-coenzyme A:cholesterol acyltransferase cDNA in mutant Chinese hamster ovary cells, J. Biol. Chem. 1993, Oct 5;268(28):20747-55) und eine murine Sterol-Acyltransferase (Uelmen, P.J.: Tissue-specific expression and cholesterol regulation of acylcoenzyme A:cholesterol acyltransferase (ACAT) in mice. Molecular cloning of mouse ACAT cDNA, chromosomal localization, and regulation of ACAT in vivo and in vitro, J. Biol. Chem. 1995 Nov 3;270(44):26192-201.

Die Gene des Ergosterol-Stoffwechsels in Hefe sind weitgehend bekannt und kloniert, wie beispielsweise

Nukleinsäuren kodierend eine Δ8-Δ7-Isomerase (ERG2) (Ashman, W.H. et al. (1991):Cloning and disruption of the yeast C-8 sterol isomerase gene.Lipids.Aug;26(8):628-32.),

Nukleinsäuren kodierend eine Δ5-Desaturase (ERG3) (Arthington, B.A. et al. (1991): Cloning, disruption and sequence of the gene encoding yeast C-5 sterol desaturase. Gene.Jun15;102(1):39-44.),

Nukleinsäuren kodierend eine Δ24-Reduktase (ERG 4) (Lai, M.H. et al., (1994): The identification of a gene family in the Saccharomyces cerevisiae ergosterol biosynthesis pathway. Gene.Mar11;140(1):41-9.),

Nukleinsäuren kodierend eine HMG-CoA-Reduktase *(HMG)* (Bason M.E. et al, (1988) Structural and functional conservation between yeast and human 3-hydroxy-3-methylglutaryl coenzyme A reductases, the rate-limiting enzyme of sterol biosynthesis. Mol Cell Biol 8:3797-3808,

Nukleinsäuren kodierend eine trunkierte HMG-CoA-Reduktase *(t-HMG)* (Polakowski T, Stahl U, Lang C.(1998) Overexpression of a cytosolic hydroxymethylglutaryl-CoA reductase leads to squalene accumulation in yeast. Appl Microbiol Biotechnol. Jan; 49(1):66-71,

Nukleinsäuren kodierend eine Lanosterol-C14-Demethylase *(ERG11)* (Kalb VF, Loper JC, Dey CR, Woods CW, Sutter TR (1986) Isolation of a cytochrome P-450 structural gene from Saccharomyces cerevisiae. Gene 45(3):237-45,

Nukleinsäuren kodierend eine Squalensynthetase *(ERG9)* (Jennings, S.M., (1991): Molecular cloning and characterization of the yeast gene for squalene synthetase. Proc Natl Acad Sci USA. Ju115;88(14):6038-42),

Nukleinsäuren kodierend eine Sterol-Acyltransferase *(SAT1)* und *(SAT2)* (Yang, H.:Sterol esterification in yeast: a two-gene process.Science. 1996 May 31;272(5266):1353-6.) sowie eine weitere Sterol-Acyltransferase (J. Biol. Chem. 1996, Sep 27;271(39):24157-63),

Nukleinsäuren kodierend eine Squalenepoxidase *(ERG1)* (Jandrositz, A., et al (1991) The gene encoding squalene epoxidase from Saccharomyces cerevisiae: cloning and characterization. Gene 107:155-160),

Nukleinsäuren kodierend eine C24-Methyltransferase *(ERG6)* (Hardwick, K.G. et al.,:SED6 is identical to ERG6, and encodes a putative methyltransferasere quired for ergosterol synthesis.Yeast.Feb;10(2):265-9) und

Nukleinsäuren kodierend eine Delta22-Desaturase *(ERG5)* (Skaggs, B.A. et al,: Cloning and characterization of the Saccharomyces cerevisiae C-22 sterol desaturase gene,encoding a second cytochrome P-450 involved in ergosterol biosynthesis, Gene.1996 Feb22;169(1):105-9.).

Weiterhin sind Verfahren bekannt, die eine Erhöhung des Gehalts an spezifischen Intermediaten und Endprodukten des Sterolstoffwechsels in Hefen und Pilzen zum Ziel haben.

Es ist aus EP 486 290 bekannt, daß der Gehalt an Squalen und weiteren spezifischen Sterolen, wie beispielsweise Zymosterol in Hefen erhöht werden kann, indem man die Expressionsrate der HMG-CoA-Reduktase erhöht und gleichzeitig den Stoffwechselweg der Zymosterol-C24-Methyltransferase (ERG6) und der Ergosta-5,7,24(28)-trienol-22-dehydrogenase (ERG5) unterbricht.

Aus T. Polakowski, Molekularbiologische Beeinflussung des Ergosterolstoffwechsels der Hefe Saccharomyces cerevisiae, Shaker Verlag Aachen, 1999, Seite 59 bis 66 ist bekannt, daß die Erhöhung der Expressionsrate der HMG-CoA-Reduktase alleine ohne Unterbrechung des abfließenden Stoffwechselflußes wie in EP 486 290 lediglich zu einer leichten Erhöhung des Gehalts an frühen Sterolen, sowie Squalen führt, während sich der Gehalt an späteren Sterolen, wie Ergosterol nicht signifikant ändert, bzw. bei Ergosterol sogar tendenziell abnimmt.

WO 99/16886 beschreibt ein Verfahren zur Herstellung von Ergosterol in Hefen, die eine Kombination der Gene tHMG, ERG9, SAT1 und ERG1 überexprimieren.

Tainaka et al., J, Ferment. Bioeng. 1995, 79, 64-66, beschreiben ferner, daß die Überexpression von ERG11 (Lanosterol-C14-Demethylase) zu einer Anreicherung von 4,4-Dimethylzymosterol jedoch nicht von Ergosterol führt. Die Transformante zeigte gegenüber dem Wildtyp einen, je nach Fermentationsbedingungen, um den Faktor 1,1 bis 1,47 gesteigerten Zymosterolgehalt.

Avruch et al, Can.J.Biochem 1976, 54(7), 657-665 sowie Xu et al, Biochem. Biophys. Res. Commun. 1988, 155(1), 509-517 beschreiben, daß durch spezifische Hemmung der C24-Methyltransferase und auch durch eine Mutation am Genlocus erg6 in *S. cerevisiae* neben Zymosterol, auch Spuren von Cholesterol nachzuweisen sind.

Aus der Literaturstelle WO 98/45457 sind Pflanzen mit DNA Konstrukten bekannt, welche die Expression einer delta-24-25- Reduktase zur Folge haben

Aufgabe der vorliegenden Erfindung ist es, ein Verfahren zu Herstellung von 7-Dehydrocholesterol mit vorteilhaften Eigenschaften, wie einer höheren Produktausbeute, zur Verfügung zu stellen.

Demgemäß wurde ein Verfahren gemaβ Anspruch 1 gefunden.

Eine erhöhte Aktivität gegenüber dem Wildtyp bedeutet für den Fall, daß der Ausgangsorganismus diese Aktivität nicht aufweist, daß die Aktivität verursacht wird. Für den Fall, daß der Ausgangsorganismus die Aktivität bereits aufweist, bedeutet eine gegenüber dem Wildtyp erhöhte Aktivität eine um einen Prozentsatz erhöhte Aktivität.

Unter Δ8-Δ7-Isomerase-Aktivität wird die Enzymaktivität einer Δ8-Δ7-Isomerase, auch Δ8-Δ7-Sterolisomerase genannt, verstanden.

Unter einer Δ8-Δ7-Isomerase wird ein Protein verstanden, das die enzymatische Aktivität aufweist, Zymosterol in Cholesta-7,24-dienol umzuwandeln.

Dementsprechend wird unter Δ8-Δ7-Isomerase-Aktivität die in einer bestimmten Zeit durch das Protein Δ8-Δ7-Isomerase umgesetzte Menge Zymosterol bzw. gebildete Menge Cholesta-7,24-dienol verstanden.

Bei einer erhöhten Δ8-Δ7-Isomerase-Aktivität gegenüber dem Wildtyp wird somit im Vergleich zum Wildtyp in einer bestimmten Zeit durch das Protein Δ8-Δ7-Isomerase die umgesetzte Menge Zymosterol bzw. die gebildete Menge Cholesta-7,24-dienol erhöht. Vorzugsweise beträgt diese Erhöhung der Δ8-Δ7-Isomerase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der Δ8-Δ7-Isomerase-Aktivität des Wildtyps.

Unter Δ5-Desaturase-Aktivität wird die Enzymaktivität einer Δ5-Desaturase, auch Lathosterol-5-desaturase oder Sterol-C5-Desaturase genannt, verstanden.

Unter einer Δ5-Desaturase wird ein Protein verstanden, das die enzymatische Aktivität aufweist, Cholesta-7,24-dienol in Cholesta-5,7,24-trienol umzuwandeln.

Dementsprechend wird unter Δ5-Desaturase-Aktivität die in einer bestimmten Zeit durch das Protein Δ5-Desaturase umgesetzte Menge Cholesta-7,24-dienol bzw. gebildete Menge Cholesta-5,7,24-trienol verstanden.

Bei einer erhöhten Δ5-Desaturase-Aktivität gegenüber dem Wildtyp wird somit im Vergleich zum Wildtyp in einer bestimmten Zeit durch das Protein Δ5-Desaturase die umgesetzte Menge Cholesta-7,24-dienol bzw. die gebildete Menge Cholesta-5,7,24-trienol erhöht.

Vorzugsweise beträgt diese Erhöhung der Δ5-Desaturase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der Δ5-Desaturase-Aktivität des Wildtyps.

Unter Δ24-Reduktase-Aktivität wird die Enzymaktivität einer Δ24-Reduktase, auch 24-Dehydrocholesterol-Reduktase genannt, verstanden.

Unter einer Δ24-Reduktase wird ein Protein verstanden, das die enzymatische Aktivität aufweist, die Doppelbindung zwischen C24 und C25 von Cholesterol-Verbindungen in eine Einfachbindung zu überführen, wie beispielsweise Cholesta-5,7,24-trienol in 7-Dehydrocholesterol oder Zymosterol in Lathosterol oder Cholsta-7,24-dienol in Cholesta-7-enol umzuwandeln.

Dementsprechend wird unter Δ24-Reduktase-Aktivität vorzugsweise die in einer bestimmten Zeit durch das Protein Δ24-Reduktase umgesetzte Menge Cholesta-5,7,24-trienol bzw. gebildete Menge 7-Dehydrocholesterol verstanden.

Bei einer erhöhten Δ24-Reduktase-Aktivität gegenüber dem Wildtyp wird somit im Vergleich zum Wildtyp in einer bestimmten Zeit durch das Protein Δ24-Reduktase die umgesetzte Menge Cholesta-5,7,24-trienol bzw. die gebildete Menge 7-Dehydrocholesterol erhöht.

Vorzugsweise beträgt diese Erhöhung der Δ24-Reduktase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der Δ24-Reduktase-Aktivität des Wildtyps.

Unter einem Wildtyp wird der entsprechende nicht genetisch veränderte Ausgangsorganismus verstanden. Vorzugsweise und insbesondere in Fällen in denen der Organismus oder der Wildtyp nicht eindeutig zuordenbar ist, wird unter Wildtyp für die Erhöhung der Δ8-Δ7-Isomerase-Aktivität, die Erhöhung der Δ5-Desaturase-Aktivität, die Erhöhung der Δ24-Reduktase-Aktivität, die Reduzierung der nachstehend beschriebenen C24-Methyltransferase-Aktivität, die Reduzierung der nachstehend beschriebenen A22-Desaturase-Aktivität, die Erhöhung der nachstehend beschriebenen HMG-CoA-Reduktase-Aktivität, die Erhöhung der nachstehend beschriebenen Lanosterol-C14-Demethylase-Aktivität, die Erhöhung der nachstehend beschriebenen Squalenepoxidase-Aktivität, die Erhöhung der nachstehend beschriebenen Squalensynthetase-Aktivität und die Erhöhung der nachstehend beschriebenen Sterol-Acyltransferase-Aktivität sowie für die Erhöhung des Gehalts an 7-Dehydrocholesterol und/oder dessen biosynthetischen Zwischen- und/oder Folgeprodukten ein Referenzorganismus verstanden. Dieser Referenzorganismus ist vorzugsweise der Hefestamm *Saccharomyces cerevisiae AH22.*

Die Erhöhung der Δ8-Δ7-Isomerase-Aktivität, Δ5-Desaturase-Aktivität und Δ24-Reduktase-Aktivität sowie der der nachstehend beschriebenen HMG-CoA-Reduktase-Aktivität, Lanosterol-C14-Demethylase-Aktivität, Squalenepoxidase-Aktivität, Squalensynthetase-Aktivität und Sterol-Acyltransferase-Aktivität kann unabhängig voneinander durch verschiedene Wege erfolgen, beispielsweise durch Ausschalten von hemmenden Regulationsmechanismen auf Expressionsund Proteinebene oder durch Erhöhung der Genexpression der entsprechenden Nukleinsäuren, also Nukleinsäuren codierend eine Δ8-Δ7-Isomerase, Δ5-Desaturase, Δ24-Reduktase, HMG-CoA-Reduktase, Lanosterol-C14-Demethylase, Squalenepoxidase, Squalensynthetase oder Sterol-Acyltransferase gegenüber dem Wildtyp.

Die Erhöhung der Genexpression der entsprechenden Nukleinsäure gegenüber dem Wildtyp kann ebenfalls durch verschiedene Wege erfolgen, beispielsweise durch Induzierung der entsprechenden Gene durch Aktivatoren, also durch Induzierung des Δ8-Δ7-Isomerase-Gens, des Δ5-Desaturase-Gens, des Δ24-Reduktase-Gens, des HMG-CoA-Reduktase-Gens, des Lanosterol-C14-Demethylase-Gens, des Squalenepoxidase-Gens, des Squalensynthetase-Gens oder des Sterol-Acyltransferase-Gens durch Aktivatoren oder durch Einbringen von einer oder mehrerer Genkopien der entsprechenden Nukleinsäuren, also durch Einbringen einer oder mehrerer Nukleinsäuren codierend eine Δ8-Δ7-Isomerase, Δ5-Desaturase, Δ24-Reduktase, HMG-CoA-Reduktase, Lanosterol-C14-Demethylase, Squalenepoxidase, Squalensynthetase oder Sterol-Acyltransferase in den Organismus.

Unter Erhöhung der Genexpression einer Nukleinsäure codierend eine Δ8-Δ7-Isomerase, Δ5-Desaturase, Δ24-Reduktase, HMG-CoA-Reduktase, Lanosterol-C14-Demethylase, Squalenepoxidase, Squalensynthetase oder Sterol-Acyltransferase wird erfindungsgemäß auch die Manipulation der Expression der Organismus eigenen, insbesondere der Hefen eigenen, endogenen Δ8-Δ7-Isomerasen, Δ5-Desaturasen, Δ24-Reduktasen, HMG-CoA-Reduktasen, Lanosterol-C14-Demethylasen, Squalenepoxidasen, Squalensynthetasen oder Sterol-Acyltransferasen verstanden.

Dies kann beispielsweise durch Veränderung der Promotor DNA-Sequenz für Δ8-Δ7-Isomerase, Δ5-Desaturase, Δ24-Reduktase, HMG-CoA-Reduktase, Lanosterol-C14-Demethylase, Squalenepoxidase, Squalensynthetase oder Sterol-Acyltransferase kodierende Gene erreicht werden. Eine solche Veränderung, die eine erhöhte Expressionsrate des entsprechenden Gens zur Folge hat, kann beispielsweise durch Deletion oder Insertion von DNA Sequenzen erfolgen.

Es ist, wie vorstehend beschrieben, möglich, die Expression der endogenen Δ8-Δ7-Isomerase, Δ5-Desaturase, Δ24-Reduktase, HMG-CoA-Reduktase, Lanosterol-C14-Demethylase, Squalenepoxidase, Squalensynthetase oder Sterol-Acyltransferase durch die Applikation exogener Stimuli zu verändern. Dies kann durch besondere physiologische Bedingungen, also durch die Applikation von Fremdsubstanzen erfolgen.

Desweiteren kann eine veränderte bzw. erhöhte Expression endogener Δ8-Δ7-Isomerase-, Δ5-Desaturase-, Δ24-Reduktase-, HMG-CoA-Reduktase-, Lanosterol-C14-Demethylase-, Squalenepoxidase-, Squalensynthetase- oder Sterol-Acyltransferase-Gens dadurch erzielt werden, dass ein im nicht transformierten Organismus nicht vorkommendes Regulator-Protein mit dem Promotor dieser Gene in Wechselwirkung tritt.

Solch ein Regulator kann ein chimäres Protein darstellen, welches aus einer DNA-Bindedomäne und einer Transkriptionsaktivator-Domäne besteht, wie beispielsweise in WO 96/06166 beschrieben.

In einer bevorzugten Ausführungsform erfolgt die Erhöhung der Δ8-Δ7-Isomerase-Aktivität gegenüber dem Wildtyp durch eine Erhöhung der Genexpression einer Nukleinsäure codierend eine Δ8-Δ7-Isomerase.

In einer weiter bevorzugten Ausführungsform erfolgt die Erhöhung der Genexpression einer Nukleinsäure codierend eine Δ8-Δ7-Isomerase durch Einbringen von einer oder mehrer Nukleinsäuren codierend eine Δ8-Δ7-Isomerase in den Organismus.

Dazu kann prinzipiell jedes Δ8-Δ7-Isomerase-Gen, also jede Nukleinsäure, die eine Δ8-Δ7-Isomerase codiert, verwendet werden.

Bei genomischen Δ8-Δ7-Isomerase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Δ8-Δ7-Isomerase zu exprimieren, bevorzugt bereits prozessierte Nukleinsäuresequenzen, wie die entsprechenden cDNAs zu verwenden.

Beispiele für Δ8-Δ7-Isomerase-Gene sind Nukleinsäuren, codierend eine murine Δ8-Δ7-Isomerase (Nukleinsäure: Seq. ID. No. 1, Protein: Seq. ID. No. 2) oder eine humane Δ8-Δ7-Isomerase (Nukleinsäure: Seq. ID. No. 3, Protein: Seq. ID. No. 4)(Braverman,N. et al.,(1999): Mutations in the gene encoding 3beta-hydroxysteroid-delta8,delta7-isomerase cause X-linked dominant Conradi-Hunermann syndrome, Nat. Genet. 22(3), 291-294), aber auch Nukleinsäuren die Proteine codieren, die beispielsweise aufgrund einer breiten Substratspezifität die Aktivität einer Δ8-Δ7-Isomerase aufweisen, wie beispielsweise Nukleinsäuren, codierend eine C8-Isomerase aus *Saccharomyces cerevisiae (ERG2),* Nukleinsäure: Seq. ID. No. 5, Protein: Seq. ID. No. 6), (Ashman, W.H. et al. (1991):Cloning and disruption of the yeast C-8 sterol isomerase gene.Lipids.Aug;26(8):628-32.).

In den erfindungsgemäßen transgenen Organismen liegt also in dieser bevorzugten Ausführungsform gegenüber dem Wildtyp mindestens ein weiteres Δ8-Δ7-Isomerase-Gen vor.

Die Anzahl der Δ8-Δ7-Isomerase-Gene in den erfindungsgemäßen transgenen Organismen beträgt mindestens zwei, vorzugsweise mehr als zwei, besonders bevorzugt mehr als drei, ganz besonders bevorzugt mehr als fünf.

Alle in der Beschreibung erwähnten Nukleinsäuren können beispielsweise eine RNA-, DNA- oder cDNA-Sequenz sein.

Bevorzugte Δ8-Δ7-Isomerase-Gene sind Nukleinsäuren, die Proteine kodieren, die eine hohe Substratspezifität für Zymosterol aufweisen. Demnach sind insbesondere Δ8-Δ7-Isomerase-Gene und die entsprechenden Δ8-Δ7-Isomerasen aus Säugetieren und deren funktionelle Äquivalente bevorzugt.

Dementsprechend bevorzugt verwendet man im vorstehend beschriebenen Verfahren Nukleinsäuren, die Proteine kodieren, enthaltend die Aminosäuresequenz SEQ. ID. NO. 2 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 2, und die die enzymatische Eigenschaft einer Δ8-Δ7-Isomerase aufweisen.

Die Sequenz SEQ. ID. NO. 2 stellt die Aminosäuresequenz der Δ8-Δ7-Isomerase aus *Mus musculus* dar.

Weitere Beispiele für Δ8-Δ7-Isomerasen und Δ8-Δ7-Isomerase-Gene lassen sich beispielsweise aus verschiedenen Organismen, deren genomische Sequenz bekannt ist, durch Homologievergleiche der Aminosäuresequenzen oder der entsprechenden rückübersetzten Nukleinsäuresequenzen aus Datenbanken mit der SeQ ID. NO. 2 leicht auffinden.

Die Δ8-Δ7-Isomerase aus *Homo sapiens* (Seq. ID. No. 4) weist beispielsweise mit der Δ8-Δ7-Isomerase aus *Mus musculus* (Seq. ID. No. 2) eine Identität von 74 % auf.

Weitere Beispiele für Δ8-Δ7-Isomerasen und Δ8-Δ7-Isomerase-Gene lassen sich weiterhin beispielsweise ausgehend von der Sequenz SEQ. ID. No. 1 aus verschiedenen Organismen deren genomische Sequenz nicht bekannt ist, durch Hybridisierungs- und PCR-Techniken in an sich bekannter Weise leicht auffinden.

Unter dem Begriff "Substitution" ist in der Beschreibung der Austausch einer oder mehrerer Aminosäuren durch eine oder mehrere Aminosäuren zu verstehen. Bevorzugt werden sog. konservative Austausche durchgeführt, bei denen die ersetzte Aminosäure eine ähnliche Eigenschaft hat wie die ursprüngliche Aminosäure, beispielsweise Austausch von Glu durch Asp, Gln durch Asn, Val durch Ile, Leu durch Ile, Ser durch Thr.

Deletion ist das Ersetzen einer Aminosäure durch eine direkte Bindung. Bevorzugte Positionen für Deletionen sind die Termini des Polypeptides und die Verknüpfungen zwischen den einzelnen Proteindomänen.

Insertionen sind Einfügungen von Aminosäuren in die Polypeptidkette, wobei formal eine direkte Bindung durch ein oder mehrere Aminosäuren ersetzt wird.

Unter Identität zwischen zwei Proteinen wird die Identität der Aminosäuren über die jeweils gesamte Proteinlänge verstanden, insbesondere die Identität die durch Vergleich mit Hilfe der Lasergene Software der Firma DNASTAR, inc.Madison, Wisconsin (USA) unter Anwendung der Clustal Methode (Higgins DG, Sharp PM. Fast and sensitive multiple sequence alignments on a microcomputer. Comput Appl. Biosci. 1989 Ap;5(2):151-1) unter Einstellung folgender Parameter berechnet wird:

| Multiple alignment parameter: | |
|---|---|
| Gap penalty | 10 |
| Gap length penalty | 10 |

| Pairwise alignment parameter: | |
|---|---|
| K-tuple | 1 |
| Gap penalty | 3 |
| Window | 5 |
| Diagonals saved | 5 |

Unter einem Protein, das eine Identität von mindestens 30 % auf Aminosäureebene mit der Sequenz SEQ. ID. NO. 2 aufweist, wird dementsprechend ein Protein verstanden, das bei einem Vergleich seiner Sequenz mit der Sequenz SEQ. ID. NO. 2, insbesondere nach obigen Programmalgorithmus mit obigem Parametersatz eine Identität von mindestens 30 % aufweist.

In einer weiter besonders bevorzugten Ausführungsform werden zur Erhöhung der Δ8-Δ7-Isomerase-Aktivität Nukleinsäuren in Organismen eingebracht, die Proteine kodieren, enthaltend die Aminosäuresequenz der Δ8-Δ7-Isomerase aus *Mus musculus* (SEQ. ID. NO. 2).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Hefe exprimiert werden, so ist es häufig vorteilhaft, die codon usage der Hefe bei der Rückübersetzung zu verwenden.

In einer besonders bevorzugten Ausführungsform bringt man eine Nukleinsäure, enthaltend die Sequenz SEQ. ID. NO. 1 in den Organismus ein.

Die Sequenz SEQ. ID. NO. 1 stellt die cDNA aus *Mus musculus* dar, die die Δ8-Δ7-Isomerase der Sequenz SEQ ID NO. 2 codiert.

Alle vorstehend erwähnten Δ8-Δ7-Isomerase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Δ5-Desaturase-Aktivität gegenüber dem Wildtyp durch eine Erhöhung der Genexpression einer Nukleinsäure codierend eine Δ5-Desaturase.

In einer weiter bevorzugten Ausführungsform erfolgt die Erhöhung der Genexpression einer Nukleinsäure codierend eine Δ5-Desaturase durch Einbringen von einer oder mehrer Nukleinsäuren codierend eine Δ5-Desaturase in den Organismus.

Dazu kann prinzipiell jedes Δ5-Desaturase-Gen, also jede Nukleinsäure die eine Δ5-Desaturase codiert, verwendet werden.

Bei genomischen Δ5-Desaturase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Δ5-Desaturase zu exprimieren, bevorzugt bereits prozessierte Nukleinsäuresequenzen, wie die entsprechenden cDNAs zu verwenden.

Beispiele für Δ5-Desaturase-Gene sind Nukleinsäuren, codierend eine murine Δ5-Desaturase (Nukleinsäure: Seq. ID. No. 7, Protein: Seq. ID. No. 8 oder eine humane Δ5-Desaturase (Nukleinsäure: Seq. ID. No. 9, Protein: Seq. ID. No. 10) (Nishi,S. et al., (2000): cDNA cloning of the mammalian sterol C5-desaturase and the expression in yeast mutant. Biochim. Biophys. Acta, 1490, (1-2), 106-108), aber auch Nukleinsäuren die Proteine codieren, die beispielsweise aufgrund einer breiten Substratspezifität die Aktivität einer Δ5-Desaturase aufweisen, wie beispielsweise Nukleinsäuren, codierend eine C5-Desaturase aus *Saccharomyces cerevisiae* (ERG3) , (Nukleinsäure: Seq. ID. No. 11, Protein: Seq. ID. No. 12), (Arthington, B.A. et al. (1991): Cloning, disruption and sequence of the gene encoding yeast C-5 sterol desaturase. Gene.Jun15;102(1):39-44.).

In den erfindungsgemäßen transgenen Organismen liegt also in dieser bevorzugten Ausführungsform gegenüber dem Wildtyp mindestens ein weiteres Δ5-Desaturase-Gen vor.

Die Anzahl der Δ5-Desaturase-Gene in den erfindungsgemäßen transgenen Organismen beträgt mindestens zwei, vorzugsweise mehr als zwei, besonders bevorzugt mehr als drei, ganz besonders bevorzugt mehr als fünf.

Bevorzugte Δ5-Desaturase-Gene sind Nukleinsäuren, die Proteine kodieren, die eine hohe Substratspezifität für Cholesta-7,24-dienol aufweisen. Demnach sind insbesondere Δ5-Desaturase-Gene und die entsprechenden Δ5-Desaturasen aus Säugetieren und deren funktionellen Äquivalente bevorzugt.

Dementsprechend bevorzugt verwendet man im vorstehend beschriebenen Verfahren Nukleinsäuren, die Proteine kodieren, enthaltend die Aminosäuresequenz SEQ. ID. NO. 8 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 8, und die die enzymatische Eigenschaft einer Δ5-Desaturase aufweisen.

Die Sequenz SEQ. ID. NO. 8 stellt die Aminosäuresequenz der Δ5-Desaturase aus *Mus musculus* dar.

Weitere Beispiele für Δ5 Desaturasen und Δ5-Desaturase-Gene lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SeQ ID. NO. 2 leicht auffinden. Die Δ5-desaturase aus *Homo sapiens* (Seq. ID. No. 10) weist beispielsweise mit der Δ5-Desaturase aus *Mus musculus* (Seq. ID. No. 8) eine Identität von 84% auf.

Weitere Beispiele für Δ5-Desaturasen und Δ5-Desaturase-Gene lassen sich weiterhin beispielsweise ausgehend von der Sequenz SEQ. ID. No. 7 aus verschiedenen Organismen deren genomische Sequenz nicht bekannt ist, durch Hybridisierungs- und PCR-Techniken in an sich bekannter Weise leicht auffinden.

Unter einem Protein, das eine Identität von mindestens 30 % auf Aminosäureebene mit der Sequenz SEQ. ID. NO. 8 aufweist, wird dementsprechend ein Protein verstanden, das bei einem Vergleich seiner Sequenz mit der Sequenz SEQ. ID. NO. 8, insbesondere nach obigen Programmalgorithmus mit obigem Parametersatz eine Identität von mindestens 30 % aufweist.

In einer weiter besonders bevorzugten Ausführungsform werden zur Erhöhung der A5-Desaturase-Aktivität Nukleinsäuren in Organismen eingebracht, die Proteine kodieren, enthaltend die Aminosäuresequenz der Δ5-Desaturase aus *Mus musculus* (SEQ. ID. NO. 8).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Hefe exprimiert werden, so ist es häufig vorteilhaft, die codon usage der Hefe bei der Rückübersetzung zu verwenden.

In einer besonders bevorzugten Ausführungsform bringt man eine Nukleinsäure, enthaltend die Sequenz SEQ. ID. NO. 7 in den Organismus ein.

Die Sequenz SEQ. ID. NO. 7 stellt die cDNA aus *Mus musculus* dar, die die Δ5-Desaturase der Sequenz SEQ ID NO. 8 codiert.

Alle vorstehend erwähnten Δ5-Desaturase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Δ24-Reduktase-Aktivität gegenüber dem Wildtyp durch eine Erhöhung der Genexpression einer Nukleinsäure codierend eine Δ24-Reduktase.

In einer weiter bevorzugten Ausführungsform erfolgt die Erhöhung der Genexpression einer Nukleinsäure codierend eine Δ24-Reduktase durch Einbringen von einer oder mehrer Nukleinsäuren codierend eine Δ24-Reduktase, in den Organismus.

Dazu kann prinzipiell jedes Δ24-Reduktase-Gen, also jede Nukleinsäure die eine Δ24-Reduktase codiert, verwendet werden.

Bei genomischen A24-Reduktase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Δ24-Reduktase zu exprimieren, bevorzugt bereits prozessierte Nukleinsäuresequenzen, wie die entsprechenden cDNAs zu verwenden.

Beispiele für Δ24-Reduktase-Gene sind Nukleinsäuren, codierend eine murine Δ24-Reduktase (Nukleinsäure: Seq. ID. No. 13, Protein: Seq. ID. No. 14 oder eine humane Δ24-Reduktase (Nukleinsäure: Seq. ID. No. 15, Protein: Seq. ID. No. 16), (Waterham,H.R. et al.: Mutations in the 3beta-Hydroxysterol Delta24-Reductase Gene Cause Desmosterolosis, an Autosomal Recessive Disorder of Cholesterol Biosynthesis, Am. J. Hum. Genet. 69 (4), 685-694 (2001)), aber auch Nukleinsäuren die Proteine codieren, die beispielsweise aufgrund einer breiten Substratspezifität die Aktivität einer Δ24-Reduktase aufweisen, wie beispielsweise Nukleinsäuren, codierend eine Δ24-Reduktase, aus *Saccharomyces cerevisiae* (ERG4), (Nukleinsäure: Seq. ID. No. 17, Protein: Seq. ID. No. 18), (Lai, M.H. et al.,(1994): The identification of a gene family in the Saccharomyces cerevisiae ergosterol biosynthesis pathway. Gene.Mar11;140(1):41-9.).

In den erfindungsgemäßen transgenen Organismen liegt also in dieser bevorzugten Ausführungsform gegenüber dem Wildtyp mindestens ein weiteres Δ24-Reduktase-Gen vor.

Die Anzahl der Δ24-Reduktase-Gene in den erfindungsgemäßen transgenen Organismen beträgt mindestens zwei, vorzugsweise mehr als zwei, besonders bevorzugt mehr als drei, ganz besonders bevorzugt mehr als fünf.

Bevorzugte Δ24-Reduktase-Gene sind Nukleinsäuren, die Proteine kodieren, die eine hohe Substratspezifität für Cholessta-5,7,24-trienol aufweisen. Demnach sind insbesondere Δ24-Reduktase-Gene und die entsprechenden Δ24-Reduktase aus Säugetieren und deren funktionellen Äquivalente bevorzugt.

Dementsprechend bevorzugt verwendet man im vorstehend beschriebenen Verfahren Nukleinsäuren, die Proteine kodieren, enthaltend die Aminosäuresequenz SEQ. ID. NO. 14 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 14, und die die enzymatische Eigenschaft einer A24-Reduktase aufweisen.

Die Sequenz SEQ. ID. NO. 14 stellt die Aminosäuresequenz der Δ24-Reduktase aus *Mus musculus* dar.

Weitere Beispiele für Δ24-Reduktasen und Δ24-Reduktase-Gene lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SeQ ID. NO. 14 leicht auffinden.

Die Δ24-Reduktase aus *Homo sapiens* (Seq. ID. No. 16) weist beispielsweise mit der Δ24-Reduktase aus *Mus musculus* (Seq. ID. No. 14) eine Identität von 96% auf.

Weitere Beispiele für Δ24₋Reduktasen und Δ24-Reduktase-Gene lassen sich weiterhin beispielsweise ausgehend von der Sequenz SEQ. ID. No. 13 aus verschiedenen Organismen deren genomische Sequenz nicht bekannt ist, durch Hybridisierungs- und PCR-Techniken in an sich bekannter Weise leicht auffinden.

Unter einem Protein, das eine Identität von mindestens 30 % auf Aminosäureebene mit der Sequenz SEQ. ID. NO. 14 aufweist, wird dementsprechend ein Protein verstanden, das bei einem Vergleich seiner Sequenz mit der Sequenz SEQ. ID. NO. 14, insbesondere nach obigen Programmalgorithmus mit obigem Parametersatz eine Identität von mindestens 30 % aufweist.

In einer weiter besonders bevorzugten Ausführungsform werden zur Erhöhung der Δ24-Reduktase-Aktivität Nukleinsäuren in Organismen eingebracht, die Proteine kodieren, enthaltend die Aminosäuresequenz der Δ24-Reduktase aus *Mus musculus* (SEQ. ID. NO. 14). Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Hefe exprimiert werden, so ist es häufig vorteilhaft, die codon usage der Hefe bei der Rückübersetzung zu verwenden.

In einer besonders bevorzugten Ausführungsform bringt man eine Nukleinsäure, enthaltend die Sequenz SEQ. ID. NO. 13 in den *Orga*nismus ein.

Die Sequenz SEQ. ID. NO. 13 stellt die genomische DNA aus *Mus musculus* dar, die die Δ24-Reduktase der Sequenz SEQ ID NO. 14 codiert.

Alle vorstehend erwähnten Δ24-Reduktase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer weiter bevorzugten Ausführungsform des erfindungsgemäßen Verfahren werden Organismen kultiviert, die gegenüber dem Wildtyp zusätzlich zu den vorstehend beschriebenen genetischen Veränderungen eine reduzierte Aktivität mindestens einer der Aktivitäten, ausgewählt aus der Gruppe C24-Methyltransferase-Aktivität und Δ22-Desaturase-Aktivität aufweisen.

In einer weiter besonders bevorzugten Ausführungsform werden Organismen kultiviert, die gegenüber dem Wildtyp zusätzlich zu den vorstehend beschriebenen genetischen Veränderungen eine reduzierte C24-Methyltransferase-Aktivität und eine reduzierte Δ22-Desaturase-Aktivität aufweisen.

Unter einer reduzierten Aktivität wird sowohl die reduzierte als auch das komplette Ausschalten der Aktivität verstanden. Eine Reduzierung einer Aktivität umfasst demnach auch eine mengenmässige Verringerung des entsprechenden Proteins in dem Organismus bis hin zu einem vollständigen Fehlen des entsprechenden Proteins, beispielsweise zu testen durch eine fehlende Nachweisbarkeit der entsprechenden Enzymaktivität oder eine fehlende immunologische Nachweisbarkeit der entsprechenden Proteine.

Unter C24-Methyltransferase-Aktivität wird die Enzymaktivität einer C24-Methyltransferase verstanden.

Unter einer C24-Methyltranferase wird ein Protein verstanden, das die enzymatische Aktivität aufweist, Zymosterol in Fecosterol (Ergosta-8,24(28) dienol umzuwandeln.

Dementsprechend wird unter C24-Methyltransferase-Aktivität die in einer bestimmten Zeit durch das Protein C24-Methyltransferase umgesetzte Menge Zymosterol bzw. gebildete Menge Fecosterol verstanden.

Bei einer reduzierten C24-Methyltransferase-Aktivität gegenüber dem Wildtyp wird somit im Vergleich zum Wildtyp in einer bestimmten Zeit durch das Protein C24-Methyltransferase die umgesetzte Menge Zymosterol bzw. die gebildete Menge Fecosterol reduziert.

Vorzugsweise erfolgt diese Reduzierung der C24-Methyltransferase-Aktivität auf mindestens 90%, weiter bevorzugt auf mindestens 70%, weiter bevorzugt auf mindestens 50%, weiter bevorzugt auf mindestens 30%, bevorzugter auf mindestens 10%, noch bevorzugter auf mindestens 5%, insbesondere auf 0% der C24-Methyltransferase-Aktivität des Wildtyps. Besonders bevorzugt ist demnach ein Ausschalten der C24-Methyltransferase-Aktivität im Organismus.

Unter Δ22-Desaturase-Aktivität wird die Enzymaktivität einer Δ22-desaturase verstanden.

Unter einer Δ22-Desaturase wird ein Protein verstanden, dasdie enzymatische Aktivität aufweist, Ergosta-5,7-dienol in Ergosta-5,7,22,24-tetraen-3β-ol umzuwandeln.

Dementsprechend wird unter Δ22-Desaturase-Aktivität die in einer bestimmten Zeit durch das Protein Δ22-Desaturase umgesetzte Menge Ergosta-5,7-dienol bzw. gebildete Menge Ergosta-5,7,22,24-tetraen-3β-ol verstanden.

Bei einer reduzierten A22-Desaturase-Aktivität gegenüber dem Wildtyp wird somit im Vergleich zum Wildtyp in einer bestimmten Zeit durch das Protein Δ22-Desaturase die umgesetzte Menge Ergosta-5,7-dienol bzw. die gebildete Menge Ergosta-5,7,22,24-tetraen-3β-ol reduziert.

Vorzugsweise erfolgt diese Reduzierung der A22-Desaturase-Aktivität auf mindestens 90%, weiter bevorzugt auf mindestens 70%, weiter bevorzugt auf mindestens 50%, weiter bevorzugt auf mindestens 30%, bevorzugter auf mindestens 10%, noch bevorzugter auf mindestens 5%, insbesondere auf 0% der Δ22-Desaturase-Aktivität des Wildtyps. Besonders bevorzugt ist demnach ein Ausschalten der D22-Desaturase-Aktivität im Organismus.

Die Reduzierung der C24-Methyltransferase-Aktivität und/oder A22-Desaturase-Aktivität kann unabhängig voneinander durch unterschiedliche zellbiologische Mechanismen erfolgen, beispielsweise durch Inhibition der entsprechenden Aktivität auf Proteinebene, beispielsweise durch Zugabe von Inhibitoren der entsprechenden Enzyme oder durch Reduzierung der Genexpression der entsprechenden Nukleinsäuren, codierend eine C24-Methyltransferase oder Δ22-Desaturase, gegenüber dem Wildtyp.

In einer bevorzugten Ausführungsform des erfindungsgemäßen Verfahrens erfolgt die Reduzierung der C24-Methyltransferase- und/ oder Δ22-Desaturase-Aktivität gegenüber dem Wildtyp durch eine Reduzierung der Genexpression der entsprechenden Nukleinsäuren, codierend eine C24-Methyltransferase oder Δ22-desaturase.

Die Reduzierung der Genexpression der Nukleinsäuren, codierend eine C24-Methyltransferase oder Δ22-Desaturase, gegenüber dem Wildtyp kann ebenfalls durch verschiedene Wege erfolgen, beispielsweise durch
a) Einbringen von Nukleinsäuresequenzen, welche zu einer antisense-Nukleinsäuresequenz transkribierbar sind, die zur Inhibition der C24-Methyltransferase-Aktivität und/oder Δ22-Desaturase-Aktivität befähigt ist, beispielsweise indem sie die Expression von endogener C24-Methyltransferase und/oder Δ22-Desaturase-Aktivität inhibiert,
b) die zu Kosuppression führende Überexpression homologer C24-Methyltransferase- und/oder Δ22-Desaturase-Nukleinsäuresequenzen,
c) die Einführung von Nonsense-Mutationen in das Endogen mittels Einführung von RNA/DNA-Oligonukleotiden in den Organismus,
d) durch das Einbringen von spezifischen DNA-bindenden Faktoren, beispielsweise Faktoren vom Typus der Zinkfingertranskriptionsfaktoren, die eine Reduzierung der Genexpression bewirken oder
e) die Generierung von Knockout-Mutanten, beispielsweise mit Hilfe von T-DNA-Mutagenese oder homologer Rekombination.

In einer bevorzugten Ausführungsform des erfindungsgemäßen Verfahrens erfolgt die Reduzierung der Genexpression der Nukleinsäuren, codierend eine C24-Methyltransferase oder Δ22-Desaturase durch Generierung von Knockout-Mutanten, besonders bevorzugt durch homologe Rekombination.

Demnach wird bevorzugt ein Organismus verwendet, der kein funktionelles C24-Methyltransferase- und/oder Δ22-Desaturase-Gen aufweist.

In einer bevorzugten Ausführungsform erfolgt die Generierung von Knockout-Mutanten, also die Deletion des Ziellocus C24-Methyltransferase- und/oder Δ22-Desaturase-Gen bei gleichzeitiger Integration einer Expressiönskasette, enthaltend mindestens eine der vorstehend oder nachstehend beschriebenen Nukleinsäuren, codierend ein Protein dessen Aktivität im Vergleich zum Wildtyp erhöht wird, durch homologe Rekombination.

Dazu können Nukleinsäurekonstrukte verwendet werden, die neben den nachstehend beschriebenen Expressionskasetten, enthaltend Promotor, kodierende Sequenz und gegebenenfalls Terminator und neben einem nachstehend beschriebenen Selektionsmarker am 3'- und 5'-Ende Nukleinsäuresequenzen enthalten, die mit Nukleinsäuresequenzen am Anfang und am Ende des zu deletierenden Gens identisch sind.

Vorzugsweise kann der Selektionsmarker nach der Selektion durch Rekombinase-Systeme wieder entfernt werden, beispielsweise durch loxP-Signale am 3'- und 5'-Ende des Selektionsmarkers unter Verwendung einer Cre-Rekombinase (Cre-LoxP-System).

Im bevorzugten Organismus *Saccharomyces cerevisiae* bedeutet das C24-Methyltransferase-Gen das Gen *ERG6* (SEQ. ID. NO. 19). SEQ. ID. NO. 20 stellt die entsprechende C24-Methyltransferase aus *Saccharomyces cerevisiae* dar (Hardwick, K.G. et al., :SED6 is identical to ERG6, and encodes a putative methyltransferasere quired for ergosterol synthesis. Yeast.Feb;10(2):265-9).

Im bevorzugten Organismus *Saccharomyces cerevisiae* bedeutet das Δ22-Desaturase-Gen das Gen *ERG5* (SEQ. ID. NO. 21). SEQ. ID. NO. 22 stellt die entsprechende Δ22-desaturase aus *Saccharomyces cerevisiae* dar (Skaggs, B.A. et al,: Cloning and characterization of the Saccharomyces cerevisiae C-22 sterol desaturase gene,encoding a second cytochrome P-450 involved in ergosterol biosynthesis, Gene,1996 Feb22;169(1):105-9.).

In einer weiter bevorzugten Ausführungsform des erfindungsgemäßen Verfahrens werden Organismen kultiviert, die zusätzlich zu den vorstehend beschriebenen Veränderungen gegenüber dem Wildtyp eine erhöhte Aktivität mindestens einer der Aktivitäten, ausgewählt aus der Gruppe HMG-CoA-Reduktase-Aktivität, Lanosterol-C14-Demethylase-Aktivität, Squalenepoxidase-Aktivität, Squalensynthetase-Aktivität und Sterol-Acyltransferase-Aktivität aufweisen.

Unter HMG-CoA-Reduktase-Aktivität wird die Enzymaktivität einer HMG-CoA-Reduktase (3-Hydroxy-3-Methyl-Glutaryl-Coenzym-A-Reduktase) verstanden.

Unter einer HMG-CoA-Reduktase wird ein Protein verstanden, das die enzymatische Aktivität aufweist, 3-Hydroxy-3-Methyl-Glutaryl-Coenzym-A in Mevalonat umzuwandeln.

Dementsprechend wird unter HMG-CoA-Reduktase-Aktivität die in einer bestimmten Zeit durch das Protein HMG-CoA-Reduktase umgesetzte Menge 3-Hydroxy-3-Methyl-Glutaryl-Coenzym-A bzw. gebildete Menge Mevalonat verstanden.

Bei einer erhöhten HMG-CoA-Reduktase-Aktivität gegenüber dem Wildtyp wird somit im Vergleich zum Wildtyp in einer bestimmten Zeit durch das Protein HMG-CoA-Reduktase die umgesetzte Menge 3-Hydroxy-3-Methyl-Glutaryl-Coenzym-A bzw. die gebildete Menge Mevalonat erhöht.

Vorzugsweise beträgt diese Erhöhung der HMG-CoA-Reduktase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der HMG-CoA-Reduktase-Aktivität des Wildtyps.

Unter Lanosterol-C14-Demethylase-Aktivität wird die Enzymaktivität einer Lanosterol-C14-Demethylase verstanden.

Unter einer Lanosterol-C14-Demethylase wird ein Protein verstanden, das die enzymatische Aktivität aufweist, Lanosterol in 4,4-Dimethylcholesta-8,14,24-trienol umzuwandeln.

Dementsprechend wird unter Lanosterol-C14-Demethylase-Aktivität die in einer bestimmten Zeit durch das Protein Lanosterol-C14-Demethylase umgesetzte Menge Lanosterol bzw. gebildete Menge 4,4-Dimethylcholesta-8,14,24-trienol verstanden.

Bei einer erhöhten Lanosterol-C14-Demethylase-Aktivität gegenüber dem Wildtyp wird somit im Vergleich zum Wildtyp in einer bestimmten Zeit durch das Protein Lanosterol-C14-Demethylase die umgesetzte Menge Lanosterol bzw. die gebildete Menge 4,4-Dimethylcholesta-8,14,24-trienol erhöht.

Vorzugsweise beträgt diese Erhöhung der Lanosterol-C14-Demethylase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der Lanosterol-C14-Demethylase-Aktivität des Wildtyps.

Unter Squalenepoxidase-Aktivität wird die Enzymaktivität einer Squalenepoxidase verstanden.

Unter einer Squalenepoxidase wird ein Protein verstanden, das die enzymatische Aktivität aufweist, Squalen in Squalenepoxid umzuwandeln.

Dementsprechend wird unter Squalenepoxidase-Aktivität die in einer bestimmten Zeit durch das Protein Squalenepoxidase umgesetzte Menge Squalen bzw. gebildete Menge Squalenepoxid verstanden.

Bei einer erhöhten Squalenepoxidase-Aktivität gegenüber dem Wildtyp wird somit im Vergleich zum Wildtyp in einer bestimmten Zeit durch das Protein Squalenepoxidase die umgesetzte Menge Squalen bzw. die gebildete Menge Squalenepoxid erhöht.

Vorzugsweise beträgt diese Erhöhung der Squalenepoxidase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der Squalenepoxidase-Aktivität des Wildtyps. Unter Squalensynthetase-Aktivität wird die Enzymaktivität einer Squalensynthetase verstanden.

Unter einer Squalensynthetase wird ein Protein verstanden, das die enzymatische Aktivität aufweist, Farnesylpyrophosphat in Squalen umzuwandeln.

Dementsprechend wird unter Squalensynthetase-Aktivität die in einer bestimmten Zeit durch das Protein Squalensynthetase umgesetzte Menge Farnesylpyrophosphat bzw. gebildete Menge Squalen verstanden.

Bei einer erhöhten Squalensynthetase-Aktivität gegenüber dem Wildtyp wird somit im Vergleich zum Wildtyp in einer bestimmten Zeit durch das Protein Squalensynthetase die umgesetzte Menge Faresylpyrophosphat bzw. die gebildete Menge Squalen erhöht.

Vorzugsweise beträgt diese Erhöhung der Squalensynthetase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der Squalensynthetase-Aktivität des Wildtyps.

Unter Sterol-Acyltransferase-Aktivität wird die Enzymaktivität einer Sterol-Acyltransferase verstanden.

Unter einer Sterol-Acyltransferase wird ein Protein verstanden, das die enzymatische Aktivität aufweist, 7-Dehydrocholesterol in in entsprechendes acetyliertes 7-Dehydrocholesterol umzuwandeln.

Dementsprechend wird unter Sterol-Acyltransferase-Aktivität die in einer bestimmten Zeit durch das Protein Sterol-Acyltransferase umgesetzte Menge 7-Dehydrocholesterol bzw. gebildete Menge acetyliertes 7-Dehydrocholesterol verstanden.

Bei einer erhöhten Sterol-Acyltransferase-Aktivität gegenüber dem Wildtyp wird somit im Vergleich zum Wildtyp in einer bestimmten Zeit durch das Protein Sterol-Acyltransferase die umgesetzte Menge 7-Dehydrocholesterol bzw. die gebildete Menge acetyliertes 7-Dehydrocholesterol erhöht.

Vorzugsweise beträgt diese Erhöhung der Sterol-Acyltransferase-Aktivität mindestens 5%, weiter bevorzugt mindestens 20%, weiter bevorzugt mindestens 50%, weiter bevorzugt mindestens 100%, bevorzugter mindestens 300%, noch bevorzugter mindestens 500%, insbesondere mindestens 600% der Sterol-Acyltransferase-Aktivität des Wildtyps.

In einer bevorzugten Ausführungsform erfolgt die Erhöhung der HMG-CoA-Reduktase-Aktivität gegenüber dem Wildtyp durch eine Erhöhung der Genexpression einer Nukleinsäure codierend eine HMG-CoA-Reduktase.

In einer besonders bevorzugten Ausführungsform des erfindungsgemäßen Verfahrens erfolgt die Erhöhung der Genexpression einer Nukleinsäure codierend eine HMG-CoA-Reduktase indem man ein Nukleinsäurekonstrukt, enthaltend eine Nukleinsäure codierend eine HMG-CoA-Reduktase in den Organismus einbringt, deren Expression in dem Organismus, verglichen mit dem Wildtyp, einer reduzierten Regulation unterliegt.

Unter einer reduzierten Regulation verglichen mit dem Wildtyp, wird eine im Vergleich zum vorstehend definierten Wildtyp verringerte, vorzugsweise keine Regulation auf Expressions- oder Proteinebene verstanden.

Die reduzierte Regulation kann vorzugsweise durch einen im Nukleinsäurekonstrukt mit der kodierenden Sequenz funktionell verknüpften Promotor erreicht werden, der in dem Organismus, verglichen mit dem Wildtyp-Promoter einer reduzierten Regulation unterliegt.

Beispielsweise unterliegt der mittlere ADH-Promotor in Hefe nur eine reduzierten Regulation und ist daher insbesondere als Promotor im vorstehend beschriebenen Nukleinsäurekonstrukt bevorzugt.

Dieses Promotorfragment des *ADH12s* Promotors, im folgenden auch ADH1 bezeichnet, zeigt eine annähernd konstitutive Expression (Ruohonen L, Penttila M, Keranen S. (1991) Optimization of Bacillus alpha-amylase production by Saccharomyces cerevisiae. Yeast. May-Jun;7(4):337-462; Lang C, Looman AC. (1995) Efficient expression and secretion of Aspergillus niger RH5344 polygalacturonase in Saccharomyces cerevisiae. Appl Microbiol Biotechnol. Dec; 44 (1-2): 147-56.), so daß die transkriptionelle Regulation nicht mehr über Intermediate der Ergosterolbiosynthese abläuft.

Weitere bevorzugte Promotoren mit reduzierter Regulation sind konstitutive Promotoren wie beispielsweise der TEF1-Promotor aus Hefe, der GPD-Promotor aus Hefe oder der PGK-Promotor aus Hefe (Mumberg D, Muller R, Funk M. (1995) Yeast vectors for the controlled expression of heterologous proteins in different genetic backgrounds. Gene. 1995 Apr 14;156(1):119-22; Chen CY, Oppermann H, Hitzeman RA. (1984) Homologous versus heterologous gene expression in the yeast, Saccharomyces cerevisiae. Nucleic Acids Res. Dec 11;12(23):8951-70.).

Die reduzierte Regulation kann in einer weiteren bevorzugten Ausführungsform dadurch erreicht werden, daß man als Nukleinsäure codierend eine HMG-CoA-Reduktase eine Nukleinsäure verwendet, deren Expression in dem Organismus, verglichen mit der Organismus eigenen, orthologen Nukleinsäure, einer reduzierten Regulation unterliegt.

Besonders bevorzugt ist die Verwendung einer Nukleinsäure, die nur den katalytischen Bereich der HMG-CoA-Reduktase kodiert (trunkierte (t-) HMG-CoA-Reduktase) als Nukleinsäure, codierend eine HMG-CoA-Reduktase. Diese in EP 486 290 und WO 99/16886 beschriebene Nukleinsäure (t-HMG) kodiert nur den katalytisch aktiven Teil der HMG-CoA-Reduktase, die für die Regulation auf Proteinebene verantwortliche Membran-Domäne fehlt. Diese Nukleinsäure unterliegt somit, insbesondere in Hefe, einer reduzierten Regulation und führt zu einer Erhöhung der Genexpression der HMG-CoA-Reduktase.

In einer besonders bevorzugten Ausführungsform bringt man Nukleinsäuren, vorzugsweise via vorstehend beschriebenes Nukleinsäurekonstrukt, ein, die Proteine kodieren, enthaltend die Aminosäuresequenz SEQ. ID. NO. 24 oder eine von dieser Sequenz durch Substitution, Insertion oder Deletion von Aminosäuren abgeleitete Sequenz, die eine Identität von mindestens 30% auf Aminosäureebene mit der Sequenz SEQ. ID. NO. 24, und die die enzymatische Eigenschaft einer HMG-CoA-Reduktase aufweisen.

Die Sequenz SEQ. ID. NO. 24 stellt die Aminosäuresequenz der trunkierten HMG-CoA-Reduktase (t-HMG) dar.

Weitere Beispiele für HMG-CoA-Reduktasen und damit auch für die auf den katalytischen Bereich reduzierten t-HMG-CoA-Reduktasen bzw. die kodierenden Gene lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SeQ ID. NO. 24 leicht auffinden.

Weitere Beispiele für HMG-CoA-Reduktasen und damit auch für die auf den katalytischen Bereich reduzierten t-HMG-CoA-Reduktasen bzw. die kodierenden Gene lassen sich weiterhin beispielsweise ausgehend von der Sequenz SEQ. ID. No. 23 aus verschiedenen Organismen deren genomische Sequenz nicht bekannt ist, durch Hybridisierungs- und PCR-Techniken in an sich bekannter Weise leicht auffinden.

Besonders bevorzugt verwendet man eine Nukleinsäure, enthaltend die Sequenz SEQ. ID. NO. 23 als Nukleinsäure, kodierend eine trunkierte HMG-CoA-Reduktase.

In einer besonders bevorzugten Ausführungsform wird die reduzierte Regulation dadurch erreicht, daß man als Nukleinsäure codierend eine HMG-CoA-Reduktase eine Nukleinsäure verwendet, deren Expression in dem Organismus, verglichen mit der Organismus eigenen, orthologen Nukleinsäure, einer reduzierten Regulation unterliegt und einen Promotor verwendet, der in dem Organismus, verglichen mit dem Wildtyp-Promoter einer reduzierten Regulation unterliegt.

In einer bevorzugten Ausführungsform erfolgt die Erhöhung der Lanosterol-C14-Demethylase-Aktivität gegenüber dem Wildtyp durch eine Erhöhung der Genexpression einer Nukleinsäure codierend eine Lanosterol-C14-Demethylase.

In einer weiter bevorzugten Ausführungsform erfolgt die Erhöhung der Genexpression einer Nukleinsäure codierend eine Lanosterol-C14-Demethylase durch Einbringen von einer oder mehrer Nukleinsäuren codierend eine Lanosterol-C14-Demethylase in den Organismus.

Dazu kann prinzipiell jedes Lanosterol-C14-Demethylase-Gen (ERG11), also jede Nukleinsäuren die eine Lanosterol-C14-Demethylase codiert, verwendet werden. Bei genomischen Lanosterol-C14-Demethylase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Lanosterol-C14-Demethylase zu exprimieren, bevorzugt bereits prozessierte Nukleinsäuresequenzen, wie die entsprechenden cDNAs zu verwenden.

Beispiele für Lanosterol-C14-Demethylase-Gene sind Nukleinsäuren, codierend eine Lanosterol-C14-Demethylase aus *Saccharomyces cerevisiae* (Kalb VF, Loper JC, Dey CR, Woods CW, Sutter TR (1986) Isolation of a cytochrome P-450 structural gene from Saccharomyces cerevisiae. Gene 45(3):237-45), *Candida albicans* (Lamb DC, Kelly DE, Baldwin BC, Gozzo F, Boscott P, Richards WG, Kelly SL (1997) Differential inhibition of Candida albicans CYP51 with azole antifungal stereoisomers. FEMS Microbiol Lett 149 (1):25-30), *Homo sapiens* (Stromstedt M, Rozman D, Waterman MR. (1996) The ubiquitously expressed human CYP51 encodes lanosterol 14 alpha-demethylase, a cytochrome P450 whose expression is regulated by oxysterols. Arch Biochem Biophys 1996 May 1;329(1):73-81c) oder *Rattus norvegicus,* Aoyama Y, Funae Y, Noshiro M, Horiuchi T, Yoshida Y. (1994) Occurrence of a P450 showing high homology to yeast lanosterol 14-demethylase (P450(14DM)) in the rat liver. Biochem Biophys Res Conmnun. Jun 30;201(3):1320-6)

In den erfindungsgemäßen transgenen Organismen liegt also in dieser bevorzugten Ausführungsform gegenüber dem Wildtyp mindestens ein weiteres Lanosterol-C14-Demethylase-Gen vor.

Die Anzahl der Lanosterol-C14-Demethylase-Gene in den erfindungsgefäßen transgenen Organismen beträgt mindestens zwei, vorzugsweise mehr als zwei, besonders bevorzugt mehr als drei, ganz besonders bevorzugt mehr als fünf.

Bevorzugt verwendet man im vorstehend beschriebenen Verfahren Nukleinsäuren, die Proteine kodieren, enthaltend die Aminosäuresequenz SEQ. ID. NO. 26 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 26, und die die enzymatische Eigenschaft einer Lanosterol-C14-Demethylase aufweisen.

Die Sequenz SEQ. ID. NO. 26 stellt die Aminosäuresequenz der Lanosterol-C14-Demethylase aus *Saccharomyces cerevisiae* dar.

Weitere Beispiele für Lanosterol-C14-Demethylasen und Lanosterol-C14-Demethylase-Gene lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SeQ ID. NO. 26 leicht auffinden.

Weitere Beispiele für Lanosterol-C14-Demethylasen und Lanosterol-C14-Demethylase-gene lassen sich weiterhin beispielsweise ausgehend von der Sequenz SEQ. ID. No. 25 aus verschiedenen Organismen deren genomische Sequenz nicht bekannt ist, durch Hybridisierungs- und PCR-Techniken in an sich bekannter Weise leicht auffinden.

Unter einem Protein, das eine Identität von mindestens 30 % auf Aminosäureebene mit der Sequenz SEQ. ID. NO. 26 aufweist, wird dementsprechend ein Protein verstanden, das bei einem Vergleich seiner Sequenz mit der Sequenz SEQ. ID. NO. 26, insbesondere nach obigen Programmalgorithmus mit obigem Parametersatz eine Identität von mindestens 30 % aufweist.

In einer weiter bevorzugten Ausführungsform werden Nukleinsäuren in Organismen eingebracht, die Proteine kodieren, enthaltend die Aminosäuresequenz der Lanosterol-C14-Demethylase aus *Saccharomyces cerevisiae* (SEQ. ID. NO. 26).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Hefe exprimiert werden, so ist es häufig vorteilhaft, die codon usage der Hefe bei der Rückübersetzung zu verwenden.

In einer besonders bevorzugten Ausführungsform bringt man eine Nukleinsäure, enthaltend die Sequenz SEQ. ID. NO. 25 in den Organismus ein.

Die Sequenz SEQ. ID. NO. 25 stellt die genomische DNA aus *Saccharomyces cerevisiae* (ORF S0001049) dar, die die Lanosterol-C14-Demethylase der Sequenz SEQ ID NO. 26 codiert.

Alle vorstehend erwähnten Lanosterol-C14-Demethylase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Squalenepoxidase-Aktivität gegenüber dem Wildtyp durch eine Erhöhung der Genexpression einer Nukleinsäure codierend eine Squalenepoxidase.

In einer weiter bevorzugten Ausführungsform erfolgt die Erhöhung der Genexpression einer Nukleinsäure codierend eine Squalenepoxidase durch Einbringen von einer oder mehrer Nukleinsäuren codierend eine Squalenepoxidase in den Organismus.

Dazu kann prinzipiell jedes Squalenepoxidase-Gen (ERG1), also jede Nukleinsäuren die eine Squalenepoxidase codiert, verwendet werden. Bei genomischen Squalenepoxidase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Squalenepoxidase zu exprimieren, bevorzugt bereits prozessierte Nukleinsäure-sequenzen, wie die entsprechenden cDNAs zu verwenden.

Beispiele für Nukleinsäuren kodierend eine Squalenepoxidase sind Nukleinsäuren, codierend eine Squalenepoxidase aus *Saccharomyces cerevisiae* (Jandrositz, A., et al (1991) The gene encoding squalene epoxidase from Saccharomyces cerevisiae: cloning and characterization. Gene 107:155-160, aus *Mus musculus* (Kosuga K, Hata S, Osumi T, Sakakibara J, Ono T. (1995) Nucleotide sequence of a cDNA for mouse squalene epoxidase, Biochim Biophys Acta, Feb 21;1260(3):345-8b), aus Rattus norvegicus (Sakakibara J, Watanabe R, Kanai Y, Ono T. (1995) Molecular cloning and expression of rat squalene epoxidase. J Biol Chem Jan 6;270(1):17-20c) oder aus *Homo sapiens* (Nakamura Y, Sakakibara J, Izumi T, Shibatä A, Ono T. (1996) Transcriptional regulation of squalene epoxidase by sterols and inhibitors in HeLa cells., J. Biol. Chem. 1996, Apr 5;271(14):8053-6).

In den erfindungsgemäßen transgenen Organismen liegt also in dieser bevorzugten Ausführungsform gegenüber dem Wildtyp mindestens ein weiteres Squalenepoxidase vor.

Die Anzahl der Squalenepoxidase-Gene in den erfindungsgemäßen transgenen Organismen beträgt mindestens zwei, vorzugsweise mehr als zwei, besonders bevorzugt mehr als drei, ganz besonders bevorzugt mehr als fünf.

Bevorzugt verwendet man im vorstehend beschriebenen Verfahren Nukleinsäuren, die Proteine kodieren, enthaltend die Aminosäuresequenz SEQ. ID. NO. 28 oder eine von dieser Sequenz durch Substitution, Insertion oder Deletion von Aminosäuren abgeleitete

Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 28, und die die enzymatische Eigenschaft einer Squalenepoxidase aufweisen.

Die Sequenz SEQ. ID. NO. 28 stellt die Aminosäuresequenz der Squalenepoxidase aus *Saccharomyces cerevisiae* dar.

Weitere Beispiele für Squalenepoxidasen und Squalenepoxidase-Gene lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SeQ ID. NO. 28 leicht auffinden.

Weitere Beispiele für Squalenepoxidase und Squalenepoxidase-Gene lassen sich weiterhin beispielsweise ausgehend von der Sequenz SEQ. ID. No. 27 aus verschiedenen Organismen deren genomische Sequenz nicht bekannt ist, durch Hybridisierungs- und PCR-Techniken in an sich bekannter Weise leicht auffinden.

In einer weiter bevorzugten Ausführungsform werden Nukleinsäuren in Organismen eingebracht, die Proteine kodieren, enthaltend die Aminosäuresequenz der Squalenepoxidase aus *Saccharomyces cerevisiae*) (SEQ. ID. NO. 28).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Hefe exprimiert werden, so ist es häufig vorteilhaft, die codon usage der Hefe bei der Rückübersetzung zu verwenden.

In einer besonders bevorzugten Ausführungsform bringt man eine Nukleinsäure, enthaltend die Sequenz SEQ. ID. NO. 27 in den Organismus ein.

Die Sequenz SEQ. ID. NO. 27 stellt die genomische DNA aus *Saccharomyces cerevisiae* (ORF YGR175C) dar, die die Squalenepoxidase der Sequenz SEQ. ID. NO. 28 codiert.

Alle vorstehend erwähnten Squalenepoxidase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Squalensynthetase-Aktivität gegenüber dem Wildtyp durch eine Erhöhung der Genexpression einer Nukleinsäure codierend eine Squalensynthetase.

In einer weiter bevorzugten Ausführungsform erfolgt die Erhöhung der Genexpression einer Nukleinsäure codierend eine Squalensynthetase durch Einbringen von einer oder mehrer Nukleinsäuren codierend eine Squalensynthetase in den Organismus.

Dazu kann prinzipiell jedes Squalensynthetase-Gen (ERG9), also jede Nukleinsäuren die eine Squalensynthetase codiert, verwendet werden. Bei genomischen Squalensynthetase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Squalensynthetase zu exprimieren, bevorzugt bereits prozessierte Nukleinsäure-sequenzen, wie die entsprechenden cDNAs zu verwenden.

Beispiele für Nukleinsäuren kodierend eine Squalensynthetase sind Nukleinsäuren, codierend eine Squalensynthetase aus *Saccharomyces cerevisiae (ERG9),* (Jennings, S.M., (1991): Molecular cloning and characterization of the yeast gene for squalene synthetase. Proc Natl Acad Sci USA. Jul15;88(14):6038-42), Nukleinsäuren, codierend eine Squalensynthetase aus *Botryococcus brauniiOkada* (Devarenne, T.P. et al.: Molecular characterization of squalene synthase from the green microalga Botryococcus braunii, raceB, Arch. Biochem. Biophys. 2000, Jan15, 373(2):307-17), Nukleinsäuren, codierend eine Squalensynthetase aus *Potato tuber* (Yoshioka H. et al.: cDNA cloning of sesquiter penecyclase and squalene synthase, and expression of the genes in potato tuber infected with Phytophthora infestans, Plant. Cell. Physiol.1999, Sep;40(9):993-8) oder Nukleinsäuren, codierend eine Squalensynthetase aus *Glycyrrhiza glabra* (Hayashi, H. et al.: Molecular cloning and characterization of two cDNAs for Glycyrrhiza glabra squalene synthase, Biol. Pharm. Bull. 1999, Sep;22(9):947-50.

In den erfindungsgemäßen transgenen Organismen liegt also in dieser bevorzugten Ausführungsform gegenüber dem Wildtyp mindestens ein weiteres Squalensynthetase-Gen vor.

Die Anzahl der Squalensynthetase-Gene in den erfindungsgemäßen transgenen Organismen beträgt mindestens zwei, vorzugsweise mehr als zwei, besonders bevorzugt mehr als drei, ganz besonders bevorzugt mehr als fünf.

Bevorzugt verwendet man im vorstehend beschriebenen Verfahren Nukleinsäuren, die Proteine kodieren, enthaltend die Aminosäuresequenz SEQ. ID. NO. 30 oder eine von dieser Sequenz durch Substitution, Insertion oder Deletion von Aminosäuren abgeleitete Sequenz, die eine Identität von mindestens 30 %, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 30, und die die enzymatische Eigenschaft einer Squalensynthetase aufweisen.

Die Sequenz SEQ. ID. NO. 30 stellt die Aminosäuresequenz der Squalensynthetase (ERG9) aus *Saccharomyces cerevisiae* dar.

Weitere Beispiele für Squalensynthetasen und Squalensynthetase-Gene lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SeQ ID. NO. 30 leicht auffinden.

Weitere Beispiele für Squalensynthetase und Squalensynthetase-Gene lassen sich weiterhin beispielsweise ausgehend von der Sequenz SEQ. ID. No. 29 aus verschiedenen Organismen deren genomische Sequenz nicht bekannt ist, durch Hybridisierungs- und PCR-Techniken in an sich bekannter Weise leicht auffinden.

In einer weiter bevorzugten Ausführungsform werden Nukleinsäuren in Organismen eingebracht, die Proteine kodieren, enthaltend die Aminosäuresequenz der Squalensynthetase aus *Saccharomyces cerevisiae*)(SEQ. ID. NO. 30).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Hefe exprimiert werden, so ist es häufig vorteilhaft, die codon usage der Hefe bei der Rückübersetzung zu verwenden.

In einer besonders bevorzugten Ausführungsform bringt man eine Nukleinsäure, enthaltend die Sequenz SEQ. ID. NO. 29 in den Organismus ein.

Die Sequenz SEQ. ID. NO. 29 stellt die genomische DNA aus *Saccharomyces cerevisiae* (ORF YHR190W) dar, die die Squalensynthetase der Sequenz SEQ. ID. NO. 30 codiert.

Alle vorstehend erwähnten Squalensynthetase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer bevorzugten Ausführungsform erfolgt die Erhöhung der Sterol-Acyltransferase-Aktivität gegenüber dem Wildtyp durch eine Erhöhung der Geneacpression einer Nukleinsäure codierend eine Sterol-Acyltransferase.

In einer weiter bevorzugten Ausführungsform erfolgt die Erhöhung der Genexpression einer Nukleinsäure codierend eine Sterol-Acyltransferase durch Einbringen von einer oder mehrer Nukleinsäuren codierend eine Sterol-Acyltransferase in den Organismus.

Dazu kann prinzipiell jedes Sterol-Acyltransferase-Gen (SAT1 oder SAT2), also jede Nukleinsäuren die eine Sterol-Acyltransferase codiert, verwendet werden. Bei genomischen Sterol-Acyltransferase-Nukleinsäure-Sequenzen aus eukaryontischen Quellen, die Introns enthalten, sind für den Fall daß der Wirtsorganismus nicht in der Lage ist oder nicht in die Lage versetzt werden kann, die entsprechenden Sterol-Acyltransferase zu exprimieren, bevorzugt bereits prozessierte Nukleinsäuresequenzen, wie die entsprechenden cDNAs zu verwenden.

Beispiele für Nukleinsäuren kodierend eine Sterol-Acyltransferase sind Nukleinsäuren, codierend eine Sterol-Acyltransferase aus *Saccharomyces cerevisiae* (*SAT1*) oder (*SAT2*), (Yang, H.:Sterol esterification in yeast: a two-gene process.Science. 1996 May 31;272(5266):1353-6.), eine weitere Nukleinsäure, codierend eine weitere Sterol-Acyltransferase aus *Saccharomyces cerevisiae* (J. Biol. Chem. 1996, Sep 27;271(39):24157-63), Nukleinsäuren kodierend eine humane Sterol-Acyltransferase (Chang, C.C. et al.,Molecular cloning and functional expression of human acyl-coenzyme A:cholesterol acyltransferase cDNA in mutant Chinese hamster ovary cells, J. Biol. Chem. 1993, Oct 5;268(28):20747-55) und Nukleinsäuren kodierend eine murine Sterol-Acyltransferase (Uelmen, P.J.: Tissue-specific expression and cholesterol regulation of acylcoenzyme A:cholesterol acyltransferase (ACAT) in mice. Molecular cloning of mouse ACAT cDNA, chromosomal localization, and regulation of ACAT in vivo and in vitro, J. Biol. Chem. 1995 Nov 3;270(44):26192-201).

In den erfindungsgemäßen transgenen Organismen liegt also in dieser bevorzugten Ausführungsform gegenüber dem Wildtyp mindestens ein weiteres Sterol-Acyltransferase vor.

Die Anzahl der Sterol-Acyltransferase-Gene in den erfindungsgemäßen transgenen Organismen beträgt mindestens zwei, vorzugsweise mehr als zwei, besonders bevorzugt mehr als drei, ganz besonders bevorzugt mehr als fünf.

Bevorzugt verwendet man im vorstehend beschriebenen Verfahren Nukleinsäuren, die Proteine kodieren, enthaltend die Aminosäuresequenz SEQ. ID. NO. 32 oder SEQ. ID. NO. 50 oder eine von diesen Sequenzen durch Substitution, Insertion oder Deletion von Aminosäuren abgeleitete Sequenz, die eine Identität von mindestens 30%, vorzugsweise mindestens 50%, bevorzugter mindestens 70%, noch bevorzugter mindestens 90%, am bevorzugtesten mindestens 95% auf Aminosäureebene mit der Sequenz SEQ. ID. NO. 32 oder SEQ. ID. NO. 50, und die die enzymatische Eigenschaft einer Sterol-Acyltransferase aufweisen.

Die Sequenz SEQ. ID. NO. 32 stellt die Aminosäuresequenz der Sterol-Acyltransferase SAT1 aus *Saccharomyces cerevisiae* dar.

Die Sequenz SEQ. ID. NO. 50 stellt die Aminosäuresequenz der Sterol-Acyltransferase SAT2 aus *Saccharomyces cerevisiae* dar.

SAT1 und SAT2 unterscheiden sich durch eine unterschiedliche Substratspezifität.

Weitere Beispiele für Sterol-Acyltransferasen und Sterol-Acyltransferase-Gene lassen sich beispielsweise aus verschiedenen Organismen deren genomische Sequenz bekannt ist durch Homologievergleiche der Aminosäuresequenzen oder der entsprechenden rückübersetzten Nukleinsäuresequenzen aus Datenbanken mit der SeQ ID. NO. 32 oder 50 leicht auffinden.

Weitere Beispiele für Sterol-Acyltransferase und Sterol-Acyltransferase-Gene lassen sich weiterhin beispielsweise ausgehend von der Sequenz SEQ. ID. No. 31 oder 49 aus verschiedenen Organismen deren genomische Sequenz nicht bekannt ist, durch Hybridisierungs- und PCR-Techniken in an sich bekannter.Weise leicht auffinden.

In einer weiter bevorzugten Ausführungsform werden Nukleinsäuren in Organismen eingebracht, die Proteine kodieren, enthaltend die Aminosäuresequenz der Sterol-Acyltransferase SAT1 aus *Saccharomyces cerevisiae*) (SEQ. ID. NO. 32) oder SAT2 aus *Saccharomyces cerevisiae*) (SEQ. ID. NO. 50).

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der organismusspezifischen codon usage häufig verwendet werden. Die codon usage läßt sich anhand von Computerauswertungen anderer, bekannter Gene der betreffenden Organismen leicht ermitteln.

Soll das Protein beispielsweise in Hefe exprimiert werden, so ist es häufig vorteilhaft, die codon usage der Hefe bei der Rückübersetzung zu verwenden.

In einer besonders bevorzugten Ausführungsform bringt man eine Nukleinsäure, enthaltend die Sequenz SEQ. ID. NO. 31 oder 49 in den Organismus ein.

Die Sequenz SEQ. ID. NO. 31 stellt die genomische DNA aus *Saccharomyces cerevisiae* (ORF YNR019W) dar, die die Sterol-Acyltransferase SAT1 der Sequenz SEQ ID NO. 32 codiert.

Die Sequenz SEQ. ID. NO. 49 stellt die genomische DNA aus *Saccharomyces cerevisiae* (ORF YCR048W) dar, die die Sterol-Acyltransferase SAT2 der Sequenz SEQ ID NO. 50 codiert.

Alle vorstehend erwähnten Sterol-Acyltransferase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Unter Organismen werden erfindungsgemäß beispielsweise Bakterien, insbesondere Bakterien der Gattung *Bacillus, Escherichia coli, Lactobacillus spec.* oder *Streptomyces spec.,*

beispielsweise Hefen, insbesondere Hefen der Gattung Saccharo*myces cerecisiae, Pichia pastoris* oder *Klyveromyces spec.*

beispielsweise Pilze, insbesondere Pilze der Gattung Aspergillus *spec., Penicillium spec.* oder *Dictyostelium spec.*

*sowie beispielsweise auch Insektenzellinien* verstanden, die in der Lage sind, als Wildtyp oder durch vorherige genetische Veränderung Zymosterol und/oder dessen biosynthetischen Zwischen- und/ oder Folgeprodukten herzustellen.

Besonders bevorzugte Organismen sind Hefen, insbesondere der Spezies Saccharomyces cerevisiae, insbesondere die Hefestämme *Saccharomyces cerevisiae AH22, Saccharomyces cerevisiae* GRF, *Saccharomyces cerevisiae* DBY747 und *Saccharomyces cerevisiae* BY4741.

Bei Hefen als Organismen oder genetisch veränderten Organismen können zur Erhöhung mindestens einer der Aktivitäten, ausgewählt aus der Gruppe Δ8-Δ7-Isomerase-Aktivität, Δ5-Desaturase-Aktivität und Δ24-Reduktase-Aktivität, wie vortsehend erwähnt, die entsprechenden Nukleinsäuren übexprimiert werden.

Die Überexpression kann sowohl homolog durch Einbringen von hefeeigenen Nukleinsäuren als auch heterolog durch Einbringen von Nukleinsäuren aus anderen Organismen, insbesondere Säugern, oder davon abgeleitete natürliche oder künstliche Varianten in die Hefe erfolgen. Vorzugsweise werden in Hefen Säugergene verwendet, da diese eine bessere Substratspezifität in Richtung 7-Dehydrocholesterol aufweisen.

Die Bestimmung der Δ8-Δ7-Isomerase-Aktivität, Δ5-Desaturase-Aktivität, Δ24-Reduktase-Aktivität, C24-Methyltransferase-Aktivität, Δ22-Desaturase-Aktivität, HMG-CoA-Reduktase-Aktivität, Lanosterol-C14-Demethylase-Aktivität, Squalenepoxidase-Aktivität, Squalensynthetase-Aktivität und-Sterol-Acyltransferase-Aktivität des erfindungsgemäßen genetisch veränderten Organismus sowie des Referenzorganismus erfolgt unter folgenden Bedingungen:

Die Bestimmung der Aktivität der HMG-CoA-Reduktase erfolgt wie in Th. Polakowski, Molekularbiologische Beeinflussung des Ergosterolstoffwechsels der Hefe Saccharomyces cerevisiae, Shaker-Verlag, Aachen 1999, ISBN 3-8265-6211-9, beschrieben.

Demgemäß werden 10⁹ Hefe-Zellen einer 48 h alten Kultur durch Zentrifugation (3500xg, 5 min) geerntet und in 2 ml Puffer I (100 mM Kaliumphosphat-Puffer, pH7,0) gewaschen. Das Zellpellet wird in 500 µl Puffer 1 (cytosolische Proteine) oder 2 (100 mM Kaliumphosphat-Puffer pH7,0; 1% Triton X-100) (Gesamtproteine) aufgenommen, und es wird 1 µl 500 mM PMSF in Isopropanol zugegegeben. Zu den Zellen kommen 500 µl Glasperlen (d= 0,5 mm), und die Zellen werden durch 5x eine Minute Vortexen aufgeschlossen. Die Flüssigkeit zwischen den Glasperlen wird in ein neues Eppi überführt. Zellreste bzw. Membranbestandteile werden durch 15 min Zentrifugieren (14000xg) abgetrennt. Der Überstand wird in ein neues Eppi überführt und stellt die Proteinfraktion dar.

Die Aktivität der HMG-CoA Aktivität wird durch Messung des Verbrauchs von NADPH+H⁺ bei der Reduktion von 3-Hydroxy-3-methylglutaryl-CoA, das als Substrat zugesetzt wird, bestimmt.

In einem Testansatz von 1000 µl werden 20 µl Hefeproteinisolat mit 910 µl Puffer I; 50 µl 0,1 M DTT und 10 µl 16 mM NADPH+H⁺ gegeben. Der Ansatz ist auf 30°C temperiert und wird für 7,5 min bei 340 nm im Photometer gemessen. Die Abnahme an NADPH, die in diesem Zeitraum gemessen wird, ist die Abbaurate ohne Substratzugabe und wird als Hintergrund berücksichtigt.

Danach erfolgt die Zugabe von Substrat (10 µl 30 mM HMG-CoA), und es werden weitere 7,5 min gemessen. Die Berechnung der HMG-CoA-Reduktase Aktivität erfolgt durch die Bestimmung der spezifischen NADPH-Abbaurate.

Die Bestimmung der Aktivität der Lanosterol-C14-Demethylase-Aktivität erfolgt wie in Omura, T and Sato, R. (1964) The carbon monoxide binding pigment in liver microsomes. J.Biol.Chem. 239, 2370-2378, beschrieben. Bei diesem Test ist die Menge an P450-Enzym als Holoenzym mit gebundenem Häm semi-quantifizierbar. Das (aktive) Holoenzym (mit Häm) kann durch CO reduziert werden und nur das CO-reduzierte Enzym weist ein Absorbtionsmaximum bei 450 nm auf. So ist das Absorbtionsmaximum bei 450 nm ein Maß für die Aktivität der Lanosterol-C14-Demethylase.

Zur Durchführung der Aktivitätsbestimmnung wird eine Microsomen-Fraktion (4-10 mg/ml Protein in 100 mM Kaliumphosphat Puffer) 1:4 verdünnt, so dass die für den Test eingesetzte Protein Konzentration 2 mg/ml beträgt. Der Test wird direkt in einer Küvette durchgeführt.

Zu den Microsomen wird eine Spatelspitze Dithionite (S₂O₄Na₂) zugeben. Mit einem Spektralphotometer wird die Baselinie aufgenommen im Bereich von 380-500 nm.

Anschliessend werden ca. 20-30 Blasen von CO durch die Probe gesprudelt. Die Absorbtion wird nun im selben Bereich gemessen. Die Höhe der Absorbtion bei 450 nm entspricht dem Abteil an P450 Enzym im Testansatz.

Die Bestimmung der Aktivität der Squalen Epoxidase erfolgt wie in Leber R, Landl K, Zinser E, Ahorn H, Spok A, Kohlwein SD, Turnowsky F, Daum G. (1998) Dual localization of squalene epoxidase, Erg1p, in yeast reflects a relationship between the endoplasmic reticulum and lipid particles, Mol. Biol. Cell. 1998, Feb;9(2):375-86, beschrieben.

Diese Methode enthält 0,35 bis 0,7 mg microsomales Protein oder 3,5 bis 75 µg Lipidpartikel Protein in 100mM Tris-HCl, pH 7,5, 1 mM EDTA, 0,1 mM FAD, 3 mM NADPH, 0,1 mM squalene 2,3-epoxidase cyclase inhibitor U18666A, 32 µM [³H] Squalen dispergiert in 0,005% Tween 80 in einem Gesamtvolumen von 500 µl.

Der Test wird bei 30°C durchgeführt. Nach einer Vorbehandlung für 10 min, wird die Reaktion durch Zugabe von Squalen gestartet und nach 15, 30 oder 45 min durch Lipid Extraktion mit 3 ml Chloroform/Methanol (2:1 vol/vol) und 750 µl 0,035 % MgCl₂ beendet.

Die Lipide werden unter Stickstoff getrocknet und in 0,5 ml Chloroform/Methanol (2:1 vol/vol) rückgelöst. Für eine Dünnschicht Chromatographie werden Teile auf eine Silica Gel 60 Platte (0,2 mm) gegeben und mit Chloroform als Laufmittel aufgetrennt. Die Positionen, die [³H] 2,3-oxidosqualen und [³H] Squalene enthalten wurden ausgekratzt und mit einem Szintilationzähler quantifiziert.

Die Bestimmung der Aktivität der Δ8-Δ7-Isomerase erfolgt in leichter Abwandlung wie in Silve S. et al.: Emopamil-binding Protein, a Mammalian Protein That Binds a Series of Structurally Diverse Neuroprotective Agents, Exhibits 8-7 Sterol Isomerase Activity in Yeast. J Biol Chem 1996 Sep 13;271(37):22434-40 beschrieben:

Aus 10 ml Kulturvolumen aufgearbeitete Microsomen werden für 3 h in der Anwesenheit von 75 µM Cholesta-8-en-3-ol bei 30 °C inkubiert. Die Sterole werden anschliessend mit 4 mal 5 ml Hexan extrahiert und aufgereinigt. Aliquots werden mittels GC/MS analysiert

Die Bestimmung der Aktivität der Δ5-Desaturase erfolgt in leichter Abwandlung wie in Nishi, S. et al. (2000):cDNA cloning of the mammalian sterol C5-desaturase and the expression in yeast mutant. Biochim.Biophys.Acta1490(1-2),106-108, beschrieben:

Aus 10 ml Kulturvolumen aufgearbeitete Microsomen werden für 3 h in der Anwesenheit von 75 µM Lathosterol und 2 mM NADH bei 30 °C inkubiert. Die Sterole werden anschliessend mit 4 mal 5 ml Hexan extrahiert und aufgereinigt. Aliquots werden mittels GC/MS analysiert

Die Bestimmung der Aktivität der Δ24-Reduktase kann wie im folgenden beschrieben durchgeführt werden:

Aus 10 ml Kulturvolumen aufgearbeitete Microsomen werden für 3 h in der Anwesenheit von 75 µM Cholesta-5,7,24-trienol bei 30 °C inkubiert. Sterole werden anschliessend mit 4 mal 5 ml Hexan extrahiert und aufgereinigt. Aliquots werden mittels GC/MS analysiert.

Die Bestimmung der Aktivität der C24-Methyltransferase kann wie im folgenden beschrieben durchgeführt werden:

Das Protein Erg6p (C24-Methyltransferase) ist zu 80% in Lipidpartikel in der Hefe nachweisbar (Athenstaedt K, Zweytick D, Jandrositz A, Kohlwein SD, Daum G: Identification and characterization of major lipid particle proteins of the yeast Saccharomyces cerevisiae.J Bacteriol. 1999 Oct;181(20):6441-8). Zur Bestimmung der Enzymaktivität werden Lipidpartikel aus einem Kulturvolumen (48h) von 100 ml aufgearbeitet (nach einer Methode beschrieben bei Athenstaedt K, Zweytick D, Jandrositz A, Kohlwein SD, Daum G: Identification and characterization of major lipid particle proteins of the yeast Saccharomyces cerevisiae. J Bacteriol. 1999 Oct;181(20):6441-8).

Der Proteingehalt wird durch einen Biorad Enzymtest)bestimmt und für jeden Testansatz werden 3 mg Protein eingesetzt in einem Volumen von 500 µl. Dem Testansatz werden 50 µM [methyl-³H₃]-S-Adenosyl-Methionin und 50 µM Zymosterol zugesetzt und der Ansatz wird 10 min bei 35°C inkubiert. Anschliessend wird das gleiche Volumen (500 µl) Chloroform/Methanol (4:1) zugesetzt und anschließend die Sterole extrahiert.

Mittels Szintillationsmessung kann der Anteil an Zymosterol mit eingebautem [methyl-³H₃]-S-Adenosyl-Methionin bestimmt werden, da durch die Chloroform/Methanol Extraktion nur lipidlösliche Substanzen extrahiert werden. Zur Quantifizierung werden die radioaktiven Zerfälle ebenfalls für 50 µM [methyl-³H₃]-S-AdenosylMethionin mittels Szintillationsmessung bestimmt.

Dieses Verfahren ist eine Abwandlung des in Nes WD, Guo D, Zhou W.:Substrate-based inhibitors of the (S)-adenosyl-L-methionine:delta24(25)- to delta24(28)-sterol methyl transferase from Saccharomyces cerevisiae, Arch. Biochem. Biophys. 1997 Jun 1;342(1):68-81. beschriebenen Verfahrens.

Die Bestimmung der Aktivität der Δ22-Desaturase (ERG5p) kann wie im folgenden beschrieben durchgeführt werden:

Verschiedene Konzentrationen von Ergosta-5,7-dienol, aufgereinigt aus *erg5* Mutantnen von *S. cerevisiae* (Parks et al, 1985. Yeast sterols.yeast mutants as tools for the study of sterol metabolism. Methods Enzymol. 111:333-346) und 50 µg dilauroylphosphatidylcholin werden gemischt und mit Ultraschall behandelt, bis eine weisse Suspension entsteht. Aufgearbeitete Mikrosomen werden hinzugegeben (1 ml) (3 mg/ml Protein). NADPH (Endkonzentration, 1 mM) wird dem Testansatz zum Start der Enzymreaktion hinzugegeben. Der Ansatz wird 20 min bei 37°C inkubiert. Die Reaktion wird durch Zugabe von 3 ml Methanol gestoppt und Sterole werden verseift durch Zugabe von 2 ml 60% (wt/vol) KOH in Wasser. Der Ansatz wird bei 90°C for 2 h inkubiert. Der Ansatz wird nach dem Abkühlen dreimal mit 5 ml Hexan extrahiert und durch Rotationsverdampfung eingeengt. Anschliessend werden die Sterole 1h bei 60°C mit bis (Trimethylsilyl) trifluoroacetamid (50 µl in 50 µl Toluol) silyliert. Die Sterole werden durch Gas Chromatographie-Massen Spektroskopie (GC-MS) (beispielsweise Model VG 12-250 gas chromatograph-mass spectrometer; VG Biotech, Manchester, United Kingdom) analysiert. Das entstandene Δ22-desaturierte Intermediat kann abhängig von der eingesetzten Menge an Substrat identifiziert werden. Als Referenz dienen Mikrosomen, die nicht mit Substrat inkubiert werden.

Dieses Verfahren ist eine Abwandlung des in Lamb et al: Purification, reconstitution, and inhibition of cytochrome P-450 sterol delta22-desaturase from the pathogenic fungus Candida glabrata. Antimicrob Agents Chemother. 1999 Jul;43(7):1725-8., beschriebenen Verfahrens.

Die Bestimmung der Aktivität der Squalensynthetase kann wie im folgenden beschrieben durchgeführt werden:

Die Tests enthalten 50 mM Mops, pH 7.2, 10 mM M₉Cl₂, 1% (v/v) Tween-80, 10% (v/v) 2-propanol, 1 mM DTT, 1 mg/mL BSA, NADPH, FPP (or PSPP) und Mikrosomen (3mg Proteingehalt) in einem Gesamtvolumen von 200 µl in Glassröhrchen. Reaktionen mit radioaktivem Substrat [1-³H] FPP (15-30 mCi/)µmol) werden bei 30 °C für 30 min inkubiert und der Suspensionsansatz mit einem Volumen von 1:1 (v/v) 40% wässriges KOH:Methanol aufgefüllt. Flüssiges NaCl wird zur Sättigung der Lösung hinzugegeben und 2 ml Ligroin enthaltend 0.5% (v/v) Squalen werden ebenfalls zugefügt.

Die Suspension wirde für 30 s gevortext. Je 1 ml der Ligroin Schicht wird in einer Pasteur Pipette auf eine gepackte 0.5 × 6 cm Aluminium Säule (80-200 mesh, Fisher) gegeben. Die Säule ist mit 2 ml Ligroin mit 0.5% (v/v) Squalen präequlibriert. Anschliesend wird die Säule mit 5 × 1 ml Toluol enthaltend 0.5% (v/v) Squalen eluiert. Die Radioaktivität von Squalen wird in Cytoscint (ICN) Szintillations Cocktail mit einem Szintilationszähler (Beckman) gemessen.

Dieses Verfahren ist eine Abwandlung der in Radisky et al., Biochemistry. 2000 Feb 22;39(7):1748-60, Zhang et al. (1993) Arch. Biochem. Biophys. 304, 133-143 und Poulter, C. D. et al. (1989) J. Am. chem. Soc. 111, 3734-3739, beschriebenen Verfahren.

Die Bestimmung der Aktivität der Sterol-Acyltransferase kann wie im folgenden beschrieben durchgeführt werden:

Aus einer 20 ml Vorkultur, die zwei Tage inkubiert wird, wird eine 200 ml Hauptkultur 1%ig angeimpft und über Nacht in Vollmedium bebrütet. Die Zellen werden geerntet und anschliessend in doppeltem Volumen HP-Puffer (100 mM Kaliumphosphatpuffer, pH 7,4; 0,5 mM EDTA; 1 mM Glutathion; 20 µM Leupeptin; 64 µM Benzamidin; 2 mM PMSF) gewaschen und in HP Puffer resuspendiert.

Nach Zugabe von 1 g Glasperlen werden die Zellen 8 mal eine Minute durch vortexen aufgeschlossen. Der Überstand wird bei 105000 xg ultrazentrifugiert. Das Pellet wird in 1 ml ACAT-Puffer (100 mM Kalium-Phophatpuffer pH7,4; 1 mM Glutathion) aufgenommen.

Der Enzymtest erfolgt in einem Volumen von 500 µl. Das Substrat Ergosterol wird in 62,5 ml 0,5 x ACAT-Puffer aufgenommen unter heftigem Vortexen. 250 µl dieser Lösung werden als Substrat für den Test eingesetzt. Dazu kommen 20 µl Proteinextrakt, 50 µl Wasser und 130 µl 0, 5 x ACAT-Puffer.

Der Ansatz wird für 15 min bei 37°C inkubiert. Danach werden 50 µl 14C-Oleoyl-CoA (600000 dpm) zugegeben und die Reaktion wird nach einer Minute durch Zugabe von 4 ml Chloroform/Methanol (2:1) gestoppt. Dazu kommen 500 µl H₂O. Zur Phasentrennung wird die Suspension kurz bei 2000 xg zentrifugiert. Die untere Phase wird in einem Spitzkolben bis zur Trockne eingeengt und in 100 µl Chloroform/Methanol (4:1) rückgelöst und auf eine DC-Platte (Kieselgel 60 F254) aufgetragen. Die DC wird mit Petroether/Diethylether/Essigsäure 90:10:1 als Laufmittel durchgeführt. Die Flecken der Sterylesterfraktion werden ausgeschnitten und in einem Szintilationszähler die Anzahl der radioaktiven Zerfälle bestimmt. Über die Menge der in Sterylester gebundene 14C-Oleoyl-CoA Moleküle ist die Enzymaktivität bestimmbar.

In einer bevorzugten Ausführungsform des erfindungsgemäßen Verfahrens erfolgt die Herstellung von 7-Dehydrocholesterol durch Kultivierung von Organismen, insbeondere von Hefen, nach Anspruch 1, welche zusätzlich eine reduzierte Aktivität mindestens einer der Aktivitäten, ausgewählt aus der Gruppe C24-Methyltransferase-Aktivität und Δ22-Desaturase-Aktivität aufweisen und zusätzlich eine eine erhöhte HMG-CoA-Reduktase-Aktivität, eine erhöhte Lanosterol-C14-Demethylase-Aktivität und eine erhöhte Squalenepoxidase-Aktivität aufweisen.

Weitere bevorzugte Ausführungsformen des erfindungsgemäßen Verfahrens sind in den Unteranspruchen angegeben

Im erfindungsgemäßen Verfahren zur Herstellung von 7-Dehydrocholesterol wird vorzugsweise dem Kultivierungsschritt der genetisch veränderten Organismen, im folgenden auch transgene Organismen bezeichnet, ein Ernten der Organismen und ein Isolieren von 7-Dehydrocholesterol angeschlossen.

Das Ernten der Organismen erfolgt in an sich bekannter Weise dem jeweiligen Organismus entsprechend. Mikroorganismen, wie Bakterien, Moose, Hefen und Pilze oder Pflanzenzellen, die durch Fermentation in flüssigen Nährmedien kultiviert werden, können beispielsweise durch Zentrifugieren, Dekantieren oder Filtrieren abgetrennt werden.

Die Isolierung von 7-Dehydrocholesterol aus der geernteten Biomasse erfolgt gemeinsam oder jede Verbindung für sich in an sich bekannter Weise, beispielsweise durch Extraktion und gegebenenfalls weiterer chemische oder physikalischer Reinigungsprozesse, wie beispielsweise Fällungsmethoden, Kristallographie, thermische Trennverfahren, wie Rektifizierverfahren oder physikalische Trennverfahren, wie beispielsweise Chromatographie.

Die Herstellung der transgenen Organismen, insbesondere Hefen kann vorzugsweise durch Transformation der Ausgangsorganismen insbesondere Hefen, mit einem Nukleinsäurekonstrukt nach Anspruch 40 erfolgen. Die Herstellung der transgenen Organismen erfolgt in dieser Ausführungsform mit einem Nukleinsäurekonstrukt.

In einer besonders bevorzugten Ausführungsform enthält das vorstehend beschriebene Nukleinsäurekonstrukt zusätzlich mindestens eine Nukleinsäure, ausgewählt aus der Gruppe Nukleinsäuren codierend eine HMG-CoA-Reduktase-Aktivität, Nukleinsäuren codierend eine Lanosterol-C14-Demethylase, Nukleinsäuren codierend eine Squalenepoxidase, Nukleinsäuren codierend eine Squalensynthetase und Nukleinsäuren codierend eine Sterol-Acyltransferase, die mit einem oder mehreren Regulationssignalen funktionell verknüpft sind, die die Transkription und Translation in Organismen gewährleisten.

Die Herstellung der transgenen Organismen kann aber auch vorzugsweise durch Transformation der Ausgangsorganismen, insbesondere Hefen, mit einer Kombination von Nukleinsaure konstrukten nach Anspruch 42 erfolgen.

In einer besonders bevorzugten Ausführungsform enthält die vorstehend beschriebene Kombination von Nukleinsäurekonstrukten zusätzlich mindestens ein Nukleinsäurekonstrukt, ausgewählt aus der Gruppe Nukleinsäurekonstrukt enthaltend Nukleinsäuren codierend eine HMG-CoA-Reduktase-Aktivität, Nukleinsäurekonstrukt enthaltend Nukleinsäuren codierend eine Lanosterol-C14-Demethylase, Nukleinsäurekonstrukt enthaltend Nukleinsäuren codierend eine Squalenepoxidase, Nukleinsäurekonstrukt enthaltend Nukleinsäuren codierend eine Squalensynthetase und Nukleinsäurekonstrukt enthaltend Nukleinsäuren codierend eine Sterol-Acyltransferase, die jeweils mit einem oder mehreren Regulationssignalen funktionell verknüpft sind, die die Transkription und Translation in Organismen gewährleisten.

Nukleinsäurekonstrukte, in denen die kodierende Nukleinsäuresequenz mit einem oder mehreren Regulationssignalen funktionell verknüpft sind, die die Transkription und Translation in Organismen, insbesondere in Hefen gewährleisten, werden im folgenden auch Expressionskasetten genannt.

Nukleinsäurekonstrukte enthaltend diese Expressionskasette sind beispielsweise Vektoren oder Plasmide.

Dementsprechend betrifft die Erfindung ferner vorstehende Nukleinsäurekonstrukte, insbesondere als Expressionskasette fungierende Nukleinsäurekonstrukte, die mit einem oder mehreren Regulationssignalen funktionell verknüpft sind, die die Transkription und Translation in Organismen gewährleisten.

In einer bevorzugten Ausführungsform umfasst dieses Nukleinsäurekonstrukt zusätzlich mindestens eine Nukleinsäure, ausgewählt aus der Gruppe Nukleinsäuren codierend eine HMG-CoA-Reduktase-Aktivität, Nukleinsäuren codierend eine Lanosterol-C14-Demethylase, Nukleinsäuren codierend eine Squalenepoxidase, Nukleinsäuren codierend eine Squalensynthetase und Nukleinsäuren codierend eine Sterol-Acyltransferase, die mit einem oder mehreren Regulationssignalen funktionell verknüpft sind, die die Transkription und Translation in Organismen gewährleisten.

Vorzugsweise enthalten die Regulationssignale einen oder mehrere Promotoren, die die Transkription und Translation in Organismen, insbesondere in Hefen gewährleisten.

Die Expressionskassetten beinhalten Regulationssignale, also regulative Nukleinsäuresequenzen, welche die Expression der kodierenden Sequenz in der Wirtszelle steuern. Gemäß einer bevorzugten Ausführungsform umfaßt eine Expressionskassette stromaufwärts, d.h. am 5'-Ende der kodierenden Sequenz, einen Promotor und stromabwärts, d.h. am 3'-Ende einen Terminator und gegebenenfalls weitere regulatorische Elemente, welche mit der dazwischenliegenden kodierenden Sequenz für mindestens eines der vorstehend beschriebenen Gene operativ verknüpft sind.

Unter.einer operativen Verknüpfung versteht man die sequenzielle Anordnung von Promotor, kodierender Sequenz, gegebenenfalls Terminator und gegebenenfalls weiterer regulativer Elemente derart, daß jedes der regulativen Elemente seine Funktion bei der Expression der kodierenden Sequenz bestimmungsgemäß erfüllen kann.

Im folgenden werden beispielhaft die bevorzugten Nukleinsäurekonstrukte, Expressionskassetten und Plasmide für Hefen und Pilze und Verfahren zur Herstellung von transgenen Hefen, sowie die transgenen Hefen selbst beschrieben.

Als Promotoren der Expressionskassette ist grundsätzlich jeder Promotor geeignet, der die Expression von Fremdgenen in Organismen, insbesondere in Hefen steuern kann.

Vorzugsweise verwendet man insbesondere einen Promotor, der in der Hefe einer reduzierten Regulation unterliegt, wie beispielsweise der mittlere ADH-Promotor.

Dieses Promotorfragment des *ADH12s* Promotors, im folgenden auch *ADH1+* bezeichnet, zeigt eine annähernd konstitutive Expression (Ruohonen L, Penttila M, Keranen S. (1991) Optimization of Bacillus alpha-amylase production by Saccbaromyces cerevisiae. Yeast. May-Jun;7(4):337-462; Lang C, Looman AC. (1995) Efficient expression and secretion of Aspergillus niger RH5344 polygalacturonase in Saccharomyces cerevisiae. Appl Microbiol Biotechnol. Dec;44(1-2):147-56.), so daß die transkriptionelle Regulation nicht mehr über Intermediate der Ergosterolbiosynthese abläuft.

Weitere bevorzugte Promotoren mit reduzierter Regulation sind konstitutive Promotoren wie beispielsweise der TEF1-Promotor aus Hefe, der GPD-Promotor aus Hefe oder der PGK-Promotor aus Hefe (Mumberg D, Muller R, Funk M.(1995) Yeast vectors for the controlled expression of heterologous proteins in different genetic backgrounds. Gene. 1995 Apr 14;156(1):119-22; Chen CY, Oppermann H, Hitzeman RA.(1984) Homologous versus heterologous gene expression in the yeast, Saccharomyces cerevisiae. Nucleic Acids Res. Dec 11;12(23):8951-70.).

Die Expressionskassette kann auch induzierbare Promotoren, insbesondere chemisch induzierbaren Promotor enthalten, durch den die Expression der Nukleinsäuren kodierend eine Δ8-Δ7-Isomerase, Δ5-Desaturase, Δ24-Reduktase, HMG-CoA-Reduktase, Lanosterol-C14-Demethylase, Squalenepoxidase, Squalensynthetase oder Sterol-Acyltransferase im Organismus zu einem bestimmten Zeitpunkt gesteuert werden kann.

Derartige Promotoren wie beispielsweise der CupI-Promotor aus Hefe (Etcheverry T. (1990) Induced expression using yeast copper metallothionein promoter. Methods Enzymol. 1990;185:319-29.), der Gal1-10-Promotor aus Hefe (Ronicke V, Graulich W, Mumberg D, Muller R, Funk M. (1997) Use of conditional promoters for expression of heterologous proteins in Saccharomyces cerevisiae, Methods Enzymol.283:313-22) oder der Pho5-Promotor aus Hefe (Bajwa W, Rudolph H, Hinnen A.(1987) PHO5 upstream sequences confer phosphate control on the constitutive PH03 gene. Yeast. 1987 Mar;3(1):33-42) können beispielsweise benutzt werden.

Als Terminator der Expressionskassette ist grundsätzlich jeder Terminator geeignet, der die Expression von Fremdgenen in Organismen, insbesondere in Hefen steuern kann.

Bevorzugt ist der Tryptophan-Terminator aus Hefe (TRP1-Terminator).

Die Herstellung einer Expressionskassette erfolgt vorzugsweise durch Fusion eines geeigneten Promotors mit den vorstehend beschriebenen Nukleinsäuren kodierend eine Δ8-Δ7-Isomerase, Δ5-Desaturase, Δ24-Reduktase, HMG-CoA-Reduktase, Lanosterol-C14-Demethylase, Squalenepoxidase, Squalensynthetase oder Sterol-Acyltransferase und gegebenenfalls einem Terminator nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor.Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben sind.

Die erfindungsgemäßen Nukleinsäuren können synthetisch hergestellt oder natürlich gewonnen sein oder eine Mischung aus synthetischen und natürlichen Nukleinsäure-Bestandteilen enthalten, sowie aus verschiedenen heterologen Genabschnitten verschiedener Organismen bestehen.

Bevorzugt sind, wie vorstehend beschrieben, synthetische Nukleotid-Sequenzen mit Kodons, die von Hefen bevorzugt werden. Diese von Hefen bevorzugten Kodons können aus Kodons mit der höchsten Proteinhäufigkeit bestimmt werden, die in den meisten interessanten Hefespezies exprimiert werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zum Wirtsorganismus sein. Die Expressionskassette beinhaltet vorzugsweise in der 5'-3'-Transkriptionsrichtung den Promotor, eine kodierende Nukleinsäuresequenz oder ein Nukleinsäurekonstrukt und eine Region für die transkriptionale Termination. Verschiedene Terminationsbereiche sind gegeneinander beliebig austauschbar.

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können *in vitro*-Mutagenese, "primerrepair", Restriktion oder Ligation verwendet werden.

Bei geeigneten Manipulationen, wie z.B. Restriktion, "chewingback" oder Auffüllen von Überhängen für "bluntends", können komplementäre Enden der Fragmente für die Ligation zur Verfügung gestellt werden.

Ferner betrifft die Erfindung die Verwendung der vorstehend beschriebenen Nukleinsäuren, der vorstehend beschriebenen Nukleinsäurekonstrukte oder der vorstehend besschriebenen Proteine zur Herstellung von transgenen Organismen, insbesondere Hefen.

Vorzugsweise weisen diese transgenen Organismen, insbesondere Hefen gegenüber dem Wildtyp einen erhöhten Gehalt an 7-Dehydrocholesterol auf.

Daher betrifft die Erfindung ferner die Verwendung der vorstehend beschriebenen Nukleinsäuren oder der erfindungsgemäßen Nukleinsäurekonstrukte zur Erhöhung des Gehalts an 7-Dehydrocholesterol und/oder dessen biosynthetischen Zwischen- und/oder Folgeprodukten in Organismen.

Die vorstehend beschriebenen Proteine und Nukleinsäuren können zur Herstellung von 7-Dehydrocholesterol in transgenen Organsimen verwendet werden.

Die Übertragung von Fremdgenen in das Genom eines Organismus, insbesondere von Hefe wird als Transformation bezeichnet.

Dazu können insbesondere bei Hefen an sich bekannte Methoden zur Transformation genutzt werden.

Geeignete Methoden zur Transformation von Hefen sind beispielsweise die LiAC-Methode, wie in Schiestl RH, Gietz RD. (1989) High efficiency transformation of intact yeast cells using single stranded nucleic acids as a carrier, Curr Genet. Dec;16 (5-6):339-46, beschrieben, die Elektroporation wie in Manivasakam P, Schiestl RH. (1993) High efficiency transformation of Saccharomyces cerevisiae by electroporation. Nucleic Acids Res. Sep 11;21(18):4414-5, beschrieben oder die Protoplasierung, wie in Morgan AJ. (1983) Yeast strain improvement by protoplast fusion and transformation, Experientia Suppl. 46:155-66 beschrieben.

Vorzugsweise wird das zu exprimierende Konstrukt in einen Vektor, insbesondere in Plasmide kloniert, die geeignet sind, Hefen zu transformieren, wie beispielsweise die Vektorsysteme Yep24 (Naumovski L, Friedberg EC (1982) Molecular cloning of eucaryotic genes required for excision repair of UV-irradiated DNA: isolation and partial characterization of the RAD3 gene of Saccharomyces cerevisiae.J Bacteriol Oct;152(1):323-31), Yep13 (Broach JR, Strathern JN, Hicks JB. (1979) Transformation in yeast: development of a hybrid cloning vector and isolation of the CAN1 gene. Gene. 1979 Dec;B(1):121-33), die pRS-Serie von Vektoren (Centromer und Episomal) (Sikorski RS, Hieter P. (1989) A system of shuttle vectors and yeast host strains designed for efficient manipulation of DNA in Saccharomyces cerevisiae. Genetics. May;122(1):19-27) sowie die Vektorsysteme YCp19 oder pYEXBX.

Dementsprechend betrifft die Erfindung weiterhin Vektoren, insbesondere Plasmide enthaltend die vorstehend beschriebenen Nukleinsäuren, Nukleinsäurekonstrukte oder Expressionskasetten.

Die Erfindung ist ferner erläuterbar mit einen Verfahren zur Herstellung von genetisch veränderten Organismen, indem man eine vorstehend beschriebene Nukleinsäure oder ein vorstehend beschriebenes Nukleinsäurekonstrukt in den Ausgangsorganismus funktionell einführt.

Die Erfindung betrifft ferner die genetisch veränderten Organismen nach Anspruch 40.

In einer bevorzugten Ausführungsform weist der genetisch veränderte Organismus gegenüber dem Wildtyp zusäzlich zu den vorstehend beschriebenen genetischen Veränderungen eine reduzierte Aktivität mindestens eine der Aktivitäten, ausgewählt aus der Gruppe C24-Methyltransferase-Aktivität und Delta22-Desaturase-Aktivität auf.

Die Reduzierung mindestens einer der Aktivitäten wird vorzugsweise durch eine Reduzierung der Genexpression mindestens einer Nukleinsäure, ausgewählt aus der Gruppe Nukleinsäuren codierend eine C24-Methyltransferase und Nukleinsäuren codierend eine Delta22-Desaturase, gegenüber dem Wildtyp bewirkt.

Ein besonders bevorzugter genetisch veränderter Organismus weist zusätzlich zu den vorstehend beschriebenen genetischen Veränderungen kein funktionelles C24-Methyltransferase-Gen und/oder Delta22-Desaturase-Gen auf.

Besonders bevorzugt sind vorstehend erwähnte, genetisch veränderte Organismen bei denen die genetische Veränderung zusätzlich mindestens eine der Aktivitäten, ausgewählt aus der Gruppe HMG-CoA-Reduktase-Aktivität, Lanosterol-C14-Demethylase-Aktivität, Squalenepoxidase-Aktivität, Squalensynthetase-Aktivität und Sterol-Acyltransferase-Aktivität, gegenüber einem Wildtyp erhöht.

Vorzugsweise erfolgt die Erhöhung mindestens einer dieser Aktivitäten, wie vorstehend erwähnt, durch eine Erhöhung der Genexpression mindestens einer Nukleinsäure, ausgewählt aus der Gruppe Nukleinsäuren codierend eine HMG-CoA-Reduktase-Aktivität, Nukleinsäuren codierend eine Lanosterol-C14-Demethylase, Nukleinsäuren codierend eine Squalenepoxidase, Nukleinsäuren codierend eine Squalensynthetase und Nukleinsäuren codierend eine Sterol-Acyltransferase, gegenüber dem Wildtyp.

Vorzugsweise erfolgt die Erhöhung der Genexpression mindestens einer Nukleinsäure, ausgewählt aus der Gruppe Nukleinsäuren codierend eine HMG-CoA-Reduktase-Aktivität, Nukleinsäuren codierend eine Lanosterol-C14-Demethylase, Nukleinsäuren codierend eine Squalenepoxidase, Nukleinsäuren codierend eine Squalensynthetase und Nukleinsäuren codierend eine Sterol-Acyltransferase gegenüber dem Wildtyp durch Erhöhung der Kopienzahl mindestens einer Nukleinsäure, ausgewählt aus der Gruppe Nukleinsäuren codierend eine HMG-CoA-Reduktase-Aktivität, Nukleinsäuren codierend eine Lanosterol-C14-Demethylase, Nukleinsäuren codierend eine Squalenepoxidase, Nukleinsäuren codierend eine Squalensynthetase und Nukleinsäuren codierend eine Sterol-Acyltransferase im Organismus.

Dementsprechend betrifft die Erfindung bevorzugt einen vorstehend beschriebenen genetisch verändertern Organismus der zwei oder mehr Nukleinsäuren mindestens einer Nukleinsäure, ausgewählt aus der Gruppe Nukleinsäuren codierend eine HMG-CoA-Reduktase-Aktivität, Nukleinsäuren codierend eine Lanosterol-C14-Demethylase, Nukleinsäuren codierend eine Squalenepoxidase, Nukleinsäuren codierend eine Squalensynthetase und Nukleinsäuren codierend eine Sterol-Acyltransferase enthält.

Insbesondere betrifft die Erfindung bevorzugt einen genetisch veränderten Organismus, der zusätzlich zu den vorstehend beschriebenen genetischen Veränderungen zwei oder mehr Nukleinsäuren codierend eine HMG-CoA-Reduktase und/oder zwei oder mehr Nukleinsäuren codierend eine Lanosterol-C14-Demethylase und/oder zwei oder mehr Nukleinsäuren codierend eine Squalenepoxidase und/ oder zwei oder mehr Nukleinsäuren codierend eine Squalensynthetase und/oder zwei oder mehr Nukleinsäuren codierend eine Sterol-Acyltransferase enthält.

Die vorstehend beschriebenen genetisch veränderten Organismen weisen gegenüber dem Wildtyp einen erhöhten Gehalt an 7-Dehydrocholesterol auf.

Bevorzugte, genetisch veränderte Organismen sind erfindungsgemäß genetisch veränderte Hefen oder Pilze, insbesondere erfindungsgemäß genetisch veränderte Hefen, insbesondere die erfindungsgemäß genetisch veränderte Hefespezies *Saccharomyces cerevisiae*, insbesondere die genetisch veränderten Hefestämme *Saccharomyces cerevisiae* AH22, *Saccharomyces cerevisiae* GRF, *Saccharomyces cerevisiae* DBY747 und *Saccharomyces cerevisiae* BY4741.

Erhöhung des Gehaltes an 7-Dehydrocholesterol bedeutet im Rahmen der vorliegenden Erfindung vorzugsweise die künstlich erworbene Fähigkeit einer erhöhten Biosyntheseleistung mindestens einer dieser, vorstehend erwähnten Verbindungen in dem genetisch veränderten Organsimus gegenüber dem nicht genetisch veränderten Organismus.

Unter Wildtyp wird dementsprechend, wie eingangs erwähnt, vorzugsweise der genetisch nicht veränderte Organismus, insbesondere aber der vorstehend erwähnte Referenzorganismus verstanden.

Unter einem erhöhten Gehalt an 7-Dehydrocholesterol im Vergleich zum Wildtyp wird insbesondere die Erhöhung des Gehaltes mindestens einer der vorstehend erwähnten Verbindungen im Organismus um mindestens 50%, vorzugsweise 100%, bevorzugter 200%, besonders bevorzugt 400% im Vergleich zum Wildtyp verstanden.

Die Bestimmung des Gehalts an mindestens einer der erwähnten Verbindungen erfolgt vorzugsweise nach an sich bekannten analytischen Methoden und bezieht sich vorzugsweise auf die Kompartimente des Organismus in denen Sterole produziert werden.

Die Erfindung wird durch die nun folgenden Beispiele erläutert, ist aber nicht auf diese beschränkt:

### I. Allgemeine experimentelle Bedingungen

### 1.Restriktion

Die Restriktion der Plasmide (1 bis 10 µg) wurde in 30 µI Ansätzen durchgeführt. Dazu wurde die DNA in 24 µI H₂0 aufgenommen, mit 3 µI des entsprechenden Puffers, 1 ml RSA (Rinderserumalbumin) und 2 µI Enzym versetzt. Die Enzymkonzentration betrug 1 Unit/µI oder 5 Units/µI je nach DNA Menge. In einigen Fällen wurde dem Ansatz noch 1 µI RNase zugegeben, um die tRNA abzubauen. Der Restriktionsansatz wurde für zwei Stunden bei 37°C inkubiert. Konrolliert wurde die Restriktion mit einem Minigel.

### 2.Gelelektrophoresen

Die Gelelektrophoresen wurden in Minigel- oder Wide-Minigelapparaturen durchgeführt. Die Minigele (ca. 20 ml, 8 Taschen) und die Wide-Minigele (50 ml, 15 oder 30 Taschen) bestanden aus 1%iger Agarose in TAE. Als Laufpuffer wurde 1 x TAE verwendet. Die Proben (10 µl) wurden mit 3 µl Stopperlosung versetzt und aufgetragen. Als Standard diente 1-DNA geschnitten mit *Hind*III (Banden bei: 23,1 kb; 9,4 kb; 6,6 kb; 4,4 kb; 2,3 kb; 2,0 kb; 0,6 kb). Zur Auftrennung wurde eine Spannung von 80 V für 45 bis 60 min angelegt. Danach wurde das Gel in Ethidiumbromidlosung angefärbt und unter UV-Licht mit dem Video-Dokumentationssystem INTAS festgehalten oder mit einem Orange-Filter fotografiert.

### 3.Gelelution

Mittels Gelelution wurden die gewünschten Fragmente isoliert. Der Restriktionsansatz wurde auf mehrere Taschen eines Minigels aufgetragen und aufgetrennt. Nur λ-*Hind*III und eine "Opferspur" wurden in Ethidiumbromidlösung angefärbt, unter UV-Licht betrachtet und das gewünschte Fragment markiert. Dadurch wurde verhindert, daß die DNA der restlichen Taschen durch das Ethidiumbromid und das UV-Licht geschädigt wird. Durch Aneinanderlegen des gefärbten und ungefärbten Gelstücks konnte anhand der Markierung das gewunschte Fragment aus dem ungefärbten Gelstück herausgeschnitten werden. Das Agarosestück mit dem zu isolierenden Fragment wurde in einen Dialyseschlauch gegeben, mit wenig TAE-Puffer luftblasenfrei verschlossen und in die BioRad-Minigelapparatur gelegt. Der Laufpuffer bestand aus 1 x TAE und die Spannung betrug 100 V für 40 min. Danach wurde für 2 min die Strompolaritat gewechselt, um am Dialyseschlauch klebende DNA wieder zu Iösen. Der die DNA-Fragmente enthaltende Puffer des Dialyseschlauches wurde in Reaktionsgefäße überführt und damit eine Ethanol Fallung durchgefuhrt. Dazu wurde der DNA-Losung 1/10 Volumen an 3 M Natriumacetat, tRNA (1 µl pro 50 µl Lösung) und dem 2,5 fachem Volumen an eiskaltem 96%igem Ethanol zugegeben. Der Ansatz wurde 30 min bei -20°C inkubiert und dann bei 12000 rpm, 30 min, 4°C abzentrifugiert. Das DNA-Pellet wurde getrocknet und in 10 bis 50 µl H₂0 (je nach DNA-Menge) aufgenommen.

### 4. Klenow-Behandlung

Durch die Klenow-Behandlung werden uberstehende Enden von DNA Fragmenten aufgefüllt, so daß "blunt-ends" entstehen. Pro 1 µg DNA wurde folgender Ansatz zusammenpipettiert:

| | | |
|---|---|---|
| DNA-Pellet | + 11 µl | H20 |
| | + 1,5 µl | 10 x Klenow Puffer |
| | + 1 µl | 0,1 M DTT |
| | + 1 µl | Nucleotide (dNTP 2 mM) |
| 25 | + 1 µl | Klenow-Polymerase (1 Unit/µl) |

Die DNA sollte dabei aus einer Ethanolfällung stammen, um zu verhindern, daß Verunreinigungen die Klenow-Polymerase hemmen. Die Inkubation erfolgte für 30 min bei 37 °C, durch weitere 5 min bei 70 °C wurde die Reaktion abgestoppt. Die DNA wurde aus dem Ansatz durch eine Ethanolfällung gewonnen und in 10 µl H₂0 aufgenommen.

### 5. Ligation

Die zu ligierenden DNA-Fragmente wurden vereinigt. Das Endvolumen von 13,1 µl enhielt ca. 0,5 µl DNA mit einem Vektor-Insert Verhaltnis von 1:5. Die Probe wurde 45 Sekunden bei 70 °C inkubiert, auf Raumtemperatur abgekühlt (ca. 3 min) und dann 10 min auf Eis inkubiert. Danach wurden die Ligationspuffer zugegeben: 2,6 µl 500 mM TrisHCl pH 7,5 und 1,3 µl 100 mM MgCl₂ und weitere 10 min auf Eis inkubiert. Nach der Zugabe von 1 µl 500 mM DTT und 1 µl 10 mM ATP und nochmaligen 10 min auf Eis wurde 1 µl Ligase (1 Unit/µl) zugegeben. Die ganze Behandlung sollte möglichst erschütterungsfrei erfolgen, um aneinanderliegende DNA-Enden nicht wieder zu trennen. Die Ligation erfolgte über Nacht bei 14 °C.

### 6.E. coli-Transformation

Kompetente *Escherichia coli* (*E*. coli) NM522 Zellen wurden mit der DNA des Ligationsansatzes transformiert. Als Positiv-Kontrolle lief ein Ansatz mit 50 µg des pScL3 Plasmids und als Null-Kontrolle ein Ansatz ohne DNA mit. Für jeden Transformationsansatz wurden 100 µl 8% PEG-Losung, 10 µl DNA und 200 µl kompetente Zellen (*E*. *coli* NM522) in ein Tischzentrifugenröhrchen pipettiert. Die Ansätze wurden für 30 min in Eis gestellt und gelegentlich geschüttelt.

Danach erfolgte der Hitzeschock: 1 min bei 42 °C. Fur die Regeneration wurde den Zellen 1 ml LB-Medium zugegeben und für 90 min bei 37 °C auf einem Schüttler inkubiert. Je 100 µl der unverdünnten Ansätze, einer 1:10 Verdünnung und einer 1:100 Verdünnung wurden auf LB + Ampicillin-Platten ausplattiert und über Nacht bei 37 °C bebrütet.

### 7. Plasmid-Isolation aus E. coli (Minipräp)

E. coli-Kolonien wurden über Nacht in 1,5 ml LB + Ampicillin-Medium in Tischzentrifugenröhrchen bei 37 °C und 120 rpm angezogen. Am nächsten Tag wurden die Zellen 5 min bei 5000 rpm und 4 °C abzentrifugiert und das Pellet in 50 µl TE-Puffer aufgenommen. Jeder Ansatz wurde mit 100 µl 0,2 N NaOH, 1 % SDS-Lösung versetzt, gemischt und für 5 min auf Eis gestellt (Lyse der Zellen). Danach wurden 400 µl Na-Acetat/NaCl-Losung (230 µl H₂0, 130 µl 3 M Natriumacetat, 40 µl 5M NaCl) zugegeben, der Ansatz gemischt und für weitere 15 min auf Eis gestellt (Proteinfällung). Nach 15 minütiger Zentrifugation bei 11000 rpm wurde der Überstand, der die Plasmid-DNA enthalt, in ein Eppendorfgefäß uberführt. War der Überstand nicht vollstandig klar, wurde nochmal zentrifugiert. Der Überstand wurde mit 360 µl eisgekühltem Isopropanol versetzt und für 30 min bei -20 °C inkubiert (DNA-Fällung). Die DNA wurde abzentrifugiert (15 min, 12000 rpm, 4 °C), der überstand verworfen, das Pellet in 100 µl eisgekuhltem 96%igem Ethanol gewaschen, 15 min bei -20 °C inkubiert und erneut abzentrifugiert (15 min, 12000 rpm, 4 °C). Das Pellet wurde im Speed Vac getrocknet und dann in 100 µl H₂0 aufgenommen. Die Plasmid DNA wurde durch Restriktionsanalyse charakterisiert. Dazu wurden 10 µl jedes Ansatzes restringiert und in einem Wide-Minigel gelelektrophoretisch aufgetrennt (siehe oben).

### 8. Plasmid-Aufarbeitung aus E. coli (Maxipräp)

Um größere Mengen an Plasmid-DNA zu isolieren, wurde die Maxipräp Methode durchgeführt. Zwei Kolben mit 100 ml LB + Ampicillin-Medium wurden mit einer Kolonie bzw. mit 100 µl einer Gefrierkultur, die das zu isolierende Plasmid trägt, angeimpft und über Nacht bei 37 °C und 120 rpm bebrütet. Die Anzucht (200 ml) wurde am nachsten Tag in einen GSA-Becher uberführt und bei 4000 rpm (2600 x g) 10 min zentrifugiert. Das Zellpellet wurde in 6 ml TE-Puffer aufgenommen. Zum Abdau der Zellwand wurden 1,2 ml Lysozymlosung (20 mg/ml TE-Puffer) zugegeben und 10 min bei Raumtemperatur inkubiert. Anschließend erfolgte die Lyse der Zellen mit 12 ml 0,2 N NaOH, 1 % SDS Lösung und weiteren 5 min Inkubation bei Raumtemperatur. Die Proteine wurden durch die Zugabe von 9 ml gekühlter 3 M Natriumacetat-Losung (pH 4,8) und einer 15 minutigen Inkubation auf Eis gefallt. Nach der Zentrifugation (GSA: 13000 rpm (27500 x g), 20 min, 4 °C) wurde der Überstand, der die DNA enthielt, in einen neuen GSA-Becher überführt und die DNA mit 15 ml eiskaltem Isopropanol und einer Inkubation von 30 min bei -20 °C gefällt. Das DNA-Pellet wurde in 5 ml eisgekühltem Ethanol gewaschen und an der Luft getrocknet (ca. 30 - 60 min). Danach wurde es in 1 ml H20 aufgenommen. Es fand eine Überprüfung des Plasmids durch Restriktionsanalyse statt. Die Konzentration wurde durch Auftragen von Verdünnungen auf einem Minigel bestimmt. Zur Verringerung des Salzgehaltes erfolgte eine 30 - 60 minutige Mikrodialyse (Porengröße 0,025 µm).

### 9. Hefe-Transformation

Für die Hefe-Transformation wurde eine Voranzucht des Stammes Saccharomyces cerevisiae AH22 angesetzt. Ein Kolben mit 20 ml YE-Medium wurde mit 100 µl der Gefrierkultur angeimpft und über Nacht bei 28 °C und 120 rpm bebrütet. Die Hauptanzucht erfolgte unter gleichen Bedingungen in Kolben mit 100 ml YE-Medium, die mit 10 µl, 20 µl oder 50 µl der Voranzucht angeimpft wurden.

### 9.1 Erstellen kompetenter Zellen

Am nächsten Tag wurden die Kolben mittels Thomakammer ausgezählt und es wurde mit dem Kolben, der eine Zellzahl von 3 - 5 x 10⁷ Zellen/ml besaß weitergearbeitet. Die Zellen wurden durch Zentrifugation (GSA: 5000 rpm (4000 x g) 10 min) geerntet. Das Zellpellet wurde in 10 ml TE-Puffer aufgenommen und auf zwei Tischzentrifugenröhrchen aufgeteilt (je 5 ml). Die Zellen wurden 3 min bei 6000 rpm abzentrifugiert und noch zweimal mit je 5 ml TE-Puffer gewaschen. Anschließend wurde das Zellpellet in 330 µl Lithiumacetat-Puffer pro 10⁹ Zellen aufgenommen, in einen sterilen 50 ml Erlenmeyerkolben überführt und eine Stunde bei 28 °C geschüttelt. Dadurch waren die Zellen kompetent für die Transformation.

### 9.2 Transformation

Für jeden Transformationsansatz wurden 15 µl Heringssperma DNA (10 mg/ml), 10 µl zu transformierende DNA (ca. 0,5 µg) und 330 µl kompetente Zellen in ein Tischzentrifugenrohrchen pipettiert und 30 min bei 28 °C (ohne Schütteln) inkubiert. Danach wurden 700 µl 50% PEG 6000 zugegeben und für eine weitere Stunde bei 28 °C, ohne Schütteln, inkubiert. Es folgte ein Hitzeschock von 5 min bei 42 °C.

100 µl der Suspension wurden auf Selektionsmedium (YNB, Difco) ausplattiert, m auf Leucinprototrophie zu selektionieren. Im Falle der Selektion auf G418 Resistenz wird nach dem Hitzeschock eine Regeneration der Zellen durchgeführt (s. unter 9.3 Regeneration sphase)

### 9.3 Regenerationsphase

Da der Selektionsmarker die Resistenz gegen G418 ist, brauchten die Zellen Zeit für die Expression des Resistenz-Gens. Die Transformationsansätze wurden mit 4 ml YE-Medium versetzt und über Nacht bei 28 °C auf dem Schüttler (120 rpm) bebrütet. Am nächsten Tag wurden die Zellen abzentrifugiert (6000 rpm, 3 min) in 1 ml YE-Medium aufgenommen und davon 100 µl bzw. 200 µl auf YE + G418-Platten ausplattiert. Die Platten wurden mehrere Tage bei 28 °C bebrütet.

### 10. Reaktionsbedingungen für die PCR

Die Reaktionsbedingungen für die Polymerase Chain Reaction müssen für den Einzelfall optimiert werden und sind nicht uneingeschränkt für jeden Ansatz gültig. So kann unter anderem die eingesetzte Menge an DNA, die Salzkonzentrationen und die Schmelztemperatur variiert werden. Fur unsere Problemstellung erwies es sich als günstig, in einem Eppendorfhütchen, das für den Einsatz im Thermocycler geeignet war, folgende Substanzen zu vereinigen: Zu 2 µl (= 0,1 U) Super Taq Polymerase wurden 5 µl Super Buffer, 8 µl dNTP's (je 0,625 µM), 5'-Primer, 3'-Primer und 0,2 µg Matritzen DNA, gelost in soviel Wasser, daß sich ein Gesamtvolumen von 50 µl für den PCR Ansatz ergibt, zugegeben. Der Ansatz wurde kurz abzentrifugiert und mit einem Tropfen Öl überschichtet. Es wurden zwischen 37 und 40 Zyklen zur Amplifizierung gewählt.

### II. Beispiele

Die Beispiele 1 bis 5 sind Vergleichsbeispiele, während Beispiel 6 erfindungsgemäβ ist.

### Beispiel 1

Expression und Überexpression einer trunkierten HMG-CoA-Reduktase, einer Squalenepoxidase (ERG1) und/oder einer Lanosterol-C14-Demethylase (ERG11) teilweise unter Deletion von ERG5 und *ERG6* in *S*.cerevisiae GRF18 bzw. GRFura3

### 1.1 Herstellung der Plasmide pFlat1 und pFlat3 und pFlat4

Zur Herstellung des Expressionsvektors pFlat3 wurde das Plasmid YEp24 (Naumovski L, Friedberg EC (1982) Molecular cloning of eucaryotic genes required for excision repair of UV-irradiated DNA: isolation and partial characterization of the RAD3 gene of Saccharomyces cerevisiae.J Bacteriol Oct;152(1):323-31) linearisiert durch Restriktion mit SphI und ein 900 bp SphI Fragment aus dem Vektor pPT2B (Lang C, Looman AC. (1995) Efficient expression and secretion of Aspergillus niger RH5344 polygalacturonase in Saccharomyces cerevisiae. Appl Microbiol Biotechnol. Dec; 44(1-2): 147-56), welches den *ADH*1 Promotor und den *TRP*1 Terminator der Hefe *Saccharomyces cerevisiae* und eine Multiple-cloning-site aus dem Vektor pUC19 (Yanisch-Perron C, Vieira J, Messing J. (1985) Improved M13 phage cloning vectors and host strains: nucleotide sequences of the M13mp18 and pUC19 vectors. Gene. 1985;33(1): 103-19.) enthält, integriert.

Die Multiple-cloning-site wurde durch einen Polylinker erweitert, der die Restriktionsstellen *Not*I und *Xho*I enthält. Der Polylinker wurde über die *Sal*I Schnittstelle des Vektors integriert. Das resultierende Plasmid heißt pFlat1. Zur Herstellung des Vektors pFlat3 wurde der Vektor pFlat1 durch das Enzym *Nco*I linearisiert und mittels Klenow-Behandlung blund end gemacht. Anschliessend wurde aus dem Plasmid YDpL (Berben, G., Dumont, J., Gilliquet, V., Bolle, P.A. and Hilger F. (1991) The YDp Plasmids: a Uniform Set of Vectors Bearing Versatile Disruption Cassettes for Saccharomyces cerevisiae. Yeast 7: 475-477.) ein mittels Klenow-Polymerase-Behandlung blund end gemachtes *Bam*HI-Fragment, welches das *LEU2* Gen der Hefe enthält, integriert.

Zur Herstellung des Vektors pFlat4 wurde der Vektor pFlat1 durch das Enzym *Nco*I linearisiert und mittels Klenow-Behandlung blund end gemacht. Anschliessend wurde aus dem Plasmid YDpH (Berben et al., 1991) ein mittels Klenow-Polymerase-Behandlung blund end gemachtes *Bam*HI-Fragment, welches das *HIS3* Gen der Hefe enthält, integriert.

1.2 Integration von *ERG1, ERG11, ERG4*, ERG2 oder ERG3 oder dem Gen der Δ24-Reduktase in die Vektoren pFLat1, pFlat3 und pFlat4

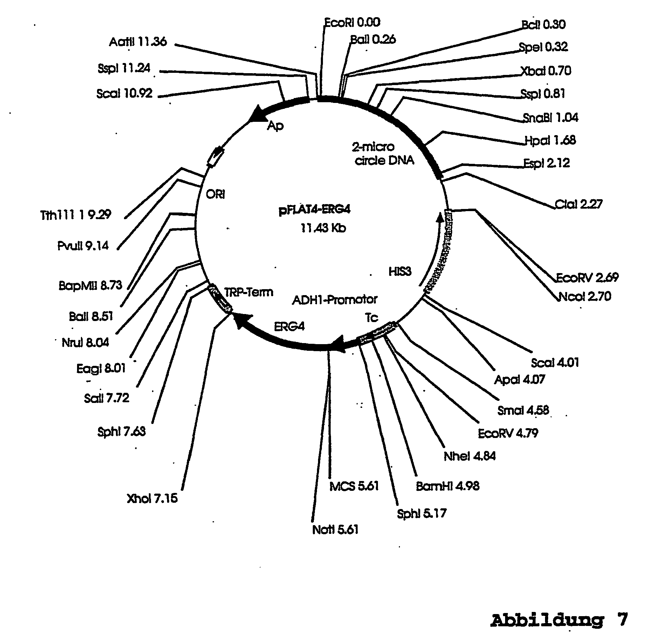

Zunächst wurde mittels PCR auf der 5' codierenden Seite der Gene *ERG1, ERG11, ERG4,* Delta24-Reduktase, *ERG2* oder *ERG3* eine *Not*I Restriktionsschnittstelle und auf der 3' codierenden Seite der Gene eine XhoI Restriktionsschnittstelle eingefügt und die entsprechenden codierenden Bereiche amplifiziert. Anschliessend wurden die Amplifikate mit den Restriktionsenzymen *Not*I und *Xho*I behandelt. Parallel wurden die Plasmide pFlat1, pFlat3 und pFlat4 mit den Enzymen *Not*I und *Xho*I behandelt. Durch Ligation mit der T4-Ligase wurden daraufhin die geschnittenen Amplifikate in die geschnittenen Plasmide integriert. Abbildung 7 zeigt beispielsweise das Plasmid pFLAT-3-*ERG4*.

Primer-Sequenzen für die Klonierung von *ERG1, ERG11, ERG2, ERG3, ERG4*, Delta24-Reduktase:
Primer ERG1-5' (SEQ. ID. No. 51):
   CTGCGGCCGC ATCATGTCTG CTGTTAACGT TGC
Primer ERG1-3' (SEQ. ID. No. 52):
   TTCTCGAGTT AACCAATCAA CTCACCAAAC
Primer ERG11-5' (SEQ. ID. No. 53):
   CTGCGGCCGCAGGATGTCTGCTACCAAGTCAATCG
Primer ERG11-3' (SEQ. ID. No. 54):
   ATCTCGAGCTTAGATCTTTTGTTCTGGATTTCTC
Primer ERG2-5'(SEQ. ID. No. 55):
   CTGCGGCCGCACCATGAAGTTTTTCCCACT CC
Primer ERG2-3'(SEQ. ID. No. 56):
   TTCTCGAGTTAGAACTTTTTGTTTTGCAACAAG
Primer ERG3-5'(SEQ. ID. No. 57):
   CTGCGGCCGCAATATGGATTTGGTCTTAGAAGTCG
Primer ERG3-3'(SEQ. ID. No. 58):
   AACTCGAGTCAGTTGTTCTTCTTGGTATTTG
Primer ERG4-5'(SEQ. ID. No. 59):
   CTGCGGCCGCACTATGGCAAAGGATAATAGTGAG
Primer ERG4-3'(SEQ. ID. No. 60):
   TTCTCGAGCTAGAAAACATAAGGAATAAAGAC
Primer Δ24R-5'(SEQ. ID. No. 47):
   CTGCGGCCGCAAGATGGAGCCCGCCGTGTCGC
Primer Δ24R-3'(SEQ. ID. No. 48):
   AACTCGAGTCAGTGCCTTGCCGCCTTGC

### 1.3 Herstellung der Integrationsvektoren pUG6-tHMG, pUG6-ERG1, pUG6-ERG11

### 1.3.1 pUG6-tHMG

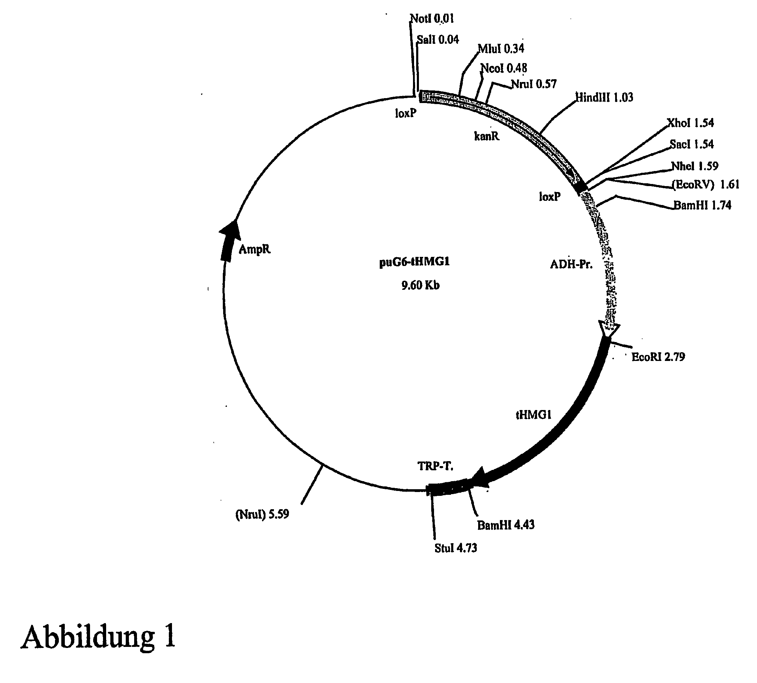

Die DNA-Sequenz für die Exprssionskassette aus *ADH1*-Promotor-*tHMG*-Trypophan-Terminator wurde aus dem Vektor YepH2 (Polakowski, T., Stahl, U., Lang, C. (1998): Overexpression of a cytosolic HMG-CoA reductase in yeast leads to squalene accumulation. Appl. Microbiol. Biotechnol.49: 66-71.) durch Restriktion mit den Enzymen *EcoR*V und *Bsp*68I (*Nru*I) unter Verwendung von Standardmethoden isoliert. Das erhaltene DNA-Fragment wurde in den Vekor pUG6 (Güldener, U et al. (1996): A new efficient gene disruption cassette for repeated use in budding yeast, Nucleic Acids Res. Jul 1;24(13):2519-24) in die *EcoR*V-Schnittstelle Blunt-end einkloniert und ergab den Vektor mit der Bezeichnung pUG6-*tHMG* (Abbildung1).

### 1.3.2 pUG6-ERG1

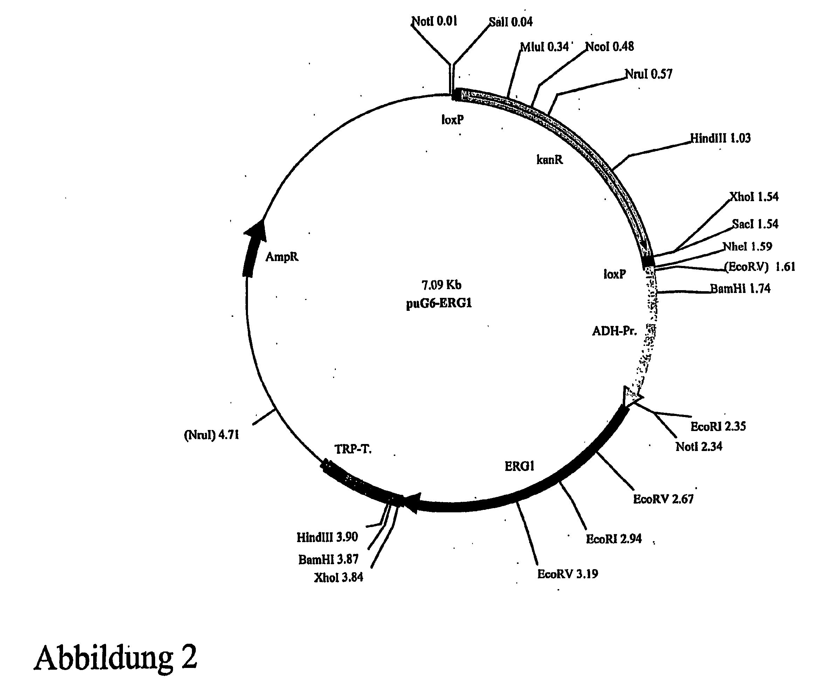

Die DNA-Sequenz für die Expressionskassette aus *ADH1*-Promotor-*ERG1*-Trypophan-Terminator wurde aus dem Vektor pFlat3-*ERG1* durch Restriktion mit den Enzymen *Nhe*I und *Bsp*68I (*Nru*I) unter Verwendung von Standardmethoden isoliert. Das erhaltene DNA-Fragment wurde nach einer Klenow-Behandlung in den Vekor pUG6 (Güldener, U et al. (1996): A new efficient gene disruption cassette for repeated use in budding yeast, Nucleic Acids Res. Jul 1;24(13):2519-24) in die *EcoR*V-Schnittstelle Blunt-end einkloniert und ergab den Vektor mit der Bezeichnung pUG6-*ERG1* (Abbildung2).

### 1.3.3 pUG6-ERG11

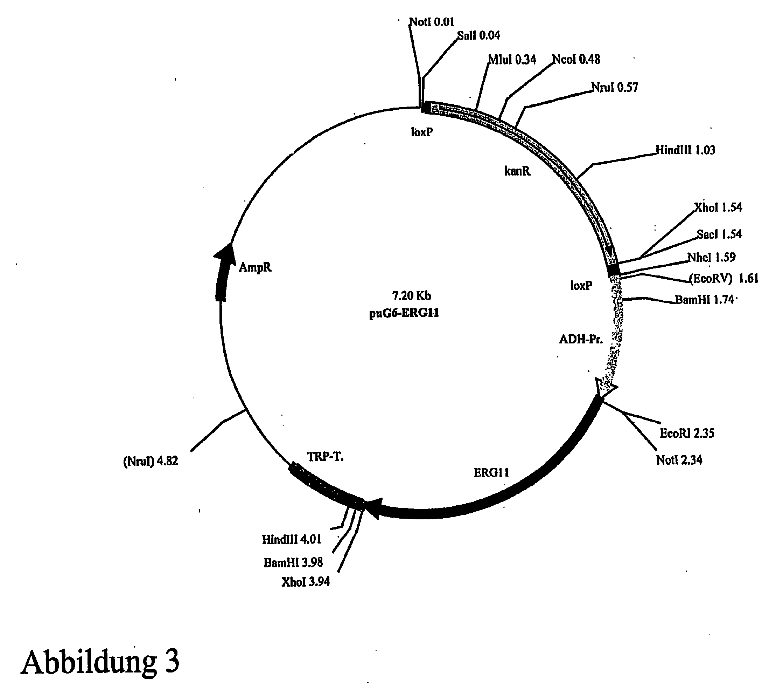

Die DNA-Sequenz für die Expressionskassette aus *ADH1*-Promotor-*ERG11*-Trypophan-Terminator wurde aus dem Vektor pFlat3-*ERG11* durch Restriktion mit den Enzymen *EcoRV* und *Bsp*68I (*Nru*I) unter Verwendung von Standardmethoden isoliert. Das erhaltene DNA-Fragment wurde in den Vekor pUG6 (Güldener, U et al. (1996): A new efficient gene disruption cassette for repeated use in budding yeast, Nucleic Acids Res. Jul 1;24(13):2519-24) in die *EcoR*V-Schnittstelle Blunt-end einkloniert und ergab den Vektor mit der Bezeichnung pUG6-*ERG11* (Abbildung 3).

### 1.4. Integrative Transformation der Expressionskassetten in die Hefestämme GRF oder GRFura3

Nach Plasmidisolation wurden Fragmente aus den Vektoren pUG6-*tHMG,* pUG6-*ERG1* und pUG6-*ERG11* mittels PCR so amplifiziert, dass die resultierenden Fragmente aus folgenden Komponenten bestehen: *loxP*-*kanMX*-*loxP*-*ADH1*-Promotor-*Zielgen*-*Tryptophan*-Terminator, wobei unter *Zielgen tHMG, ERG1* bzw. *ERG11* und unter *kanMX* ein Kanamycin-Resistenz-Gen *verstanden* wird.

Als Primer wurden Oligonukleotid-Sequenzen ausgewählt, die im annealenden Bereich die Sequenzen jenseits der zu amplifizierenden Kassetten des Vektors pUG6-*Zielgen* enthalten und an den 5' und 3' Überhängend je 40 Basenpaare der 5' oder der 3' Sequenz des Integrationslocus enthalten. So ist gewährleistet, dass einerseits das gesamte Fragment inklusive *KanMX* und Zielgen amplifiziert wird und anderseits dieses Fragment anschließend in Hefe transformiert werden kann und durch homologe Rekombination in den Ziel-Genlocus der Hefe integriert. Dabei wurden je nach Ziel-Genlocus der Hefe die folgenden Oligonucleotid-Sequenzen als Primer verwendet:

Zur Integration an den URA3 Genlocus:
URA3-Crelox-5' (SEQ. ID. No. 33):
URA3-Crelox-3' (SEQ. ID. No. 34):

Zur Integration an den LEU2 Genlocus:
LEU2-Crelox-5' (SEQ. ID. No. 35):
LEU2-Crelox-3' (SEQ. ID. No. 36):
Zur Integration an den HIS3 Genlocus:
HIS3-Crelox-5' (SEQ. ID. No. 37):
HIS3-Crelox-3' (SEQ. ID. No. 38):

Zur Integration an den ERG6 Genlocus:
ERG6-Crelox-5' (SEQ. ID. No. 39):
ERG6-Crelox-3' (SEQ. ID. No. 40):

Zur Integration an den ERG5 Genlocus:
ERG5-Crelox-5' (SEQ. ID. No. 41):
ERG5-Crelox-3' (SEQ. ID. No. 42):

Als Selektionsmarker diente die Resistenz gegen Geneticin (G418). Die resultierenden Stämme enthielten eine Kopie des jeweiligen Zielgens (*tHMG*, *ERG1* oder *ERG11*) unter der Kontrolle des *ADH*-Promotors und des Tryptophan-Terminators. Gleichzeitig war es möglich, durch die Integration der Expressionskassette das jeweilige Gen des Ziellocus zu deletieren. Um das Gen für die Resistenz gegen G418 anschliessend wieder zu entfernen wurde der entstandene Hefestamm mit dem cre Rekombinase enthaltenden Vektor pSH47 (Guldener U, Heck S, Fielder T, Beinhauer J, Hegemann JH. (1996) A new efficient gene disruption kassette for repeated use in budding yeast. Nucleic Acids Res. Jul 1;24(13):2519-24.) transformiert. Durch diesen Vektor wurde die cre Rekombinase in der Hefe exprimiert, was zur Folge hatte, das der Sequenz-Bereich innerhalb der beiden *loxP*-Sequenzen heraus rekombinierte, was wiederum zur Folge hatte, das lediglich eine der beiden *loxP*-Sequenzen und die *ADH1*-Promotor-*ZielGen*-*Tryptophan*-Terminator-Experssionskassetten in dem Ziel-Genlocus enthalten blieb.

Die Folge ist, dass der Hefestamm seine G418-Resistenz wieder verliert und damit geeignet ist, weitere Gene mittels dieses "cre-lox" Systems in den Hefestamm zu integrieren bzw. zu entfernen. Der Vektor pSH47 kann daraufhin durch Anzucht auf FOA-Medium selektiv entfernt werden.

Somit ist es möglich nacheinander mehrere Zielgene in den Hefestamm unter der Kontrolle des ADH1-Promotors und Tryptophan-Terminators an verschiedene Zielloci zu integrieren.

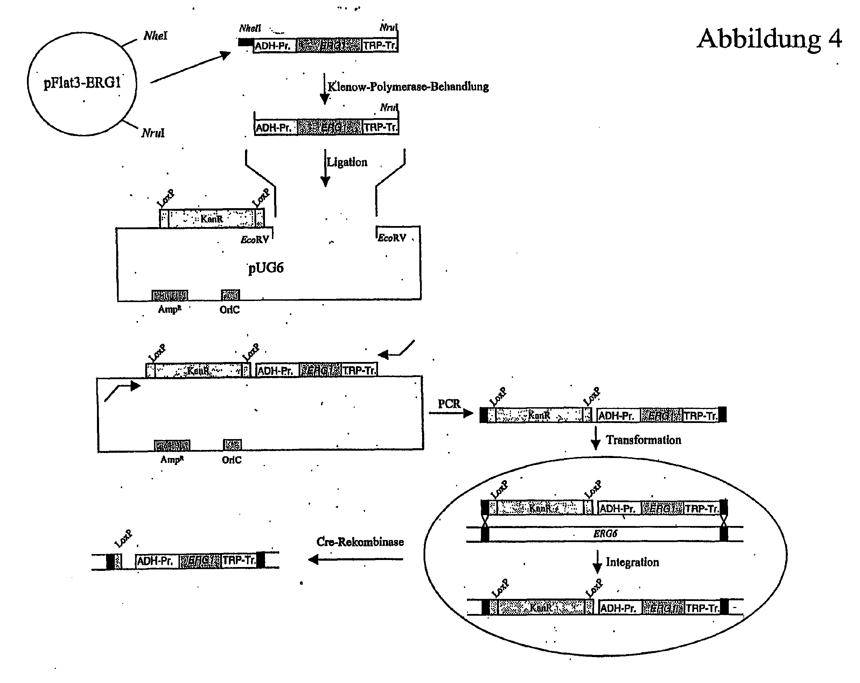

Zunächst wird ein Zielgen an den *URA3* Locus integriert bzw. wird ein *ura3*-Stamm verwendet, damit der Hefestamm Uracil auxotroph ist, da der Vektor pSH47 ein *URA3* Gen zur Selektion Uracil-prototropher Stämme enthält. Abbildung 4 zeigt ein methodisches Beispiel.

Durch diese Methode wurden die in Tabelle 1 aufgelisteten Hefe-Integrations- bzw. Deletionsstämme hergestellt, wobei, in an sich bekannter Weise, das Gen in Kleinbuchstaben für eine Deletion, das Gen in Großbuchstaben für eine Integration steht.

**Tabelle 1**

| Nr. | Stammbezeichnung | Veränderung gegenüber Hefestamm GRF |
|---|---|---|
| I | GRFtH1 | *ura3, tHMG*:*leu2* |
| II | GRFtH1E1 | *ERG1*: *ura3*, *tHMG*: *leu2* |
| III | GRFtH1E11 | *ura3, tHMG*:*leu2, ERG11*:*his3* |
| IV | GRFtH1E1E11 | *ERG1*:*ura3*, *tHMG*:*leu2, ERG11*:*his3* |
| V | GRFtH1E1E11*erg5e rg6* | *ura3*, *tHMG*:*leu2, ERG1*:*erg6, ERG11*:*erg5* |
| VI | *GRFtH1erg5erg6* | *ura3, tHMG*:*leu2, erg5, erg6* |

Die Hefestämme wurden 48 Stunden lang in WMVIII- Medium bei 28°C und 160 rpm in einem Kulturvolumen von 20 ml kultiviert. Anschliessend wurden 500 µl dieser Vorkultur in eine 50 ml Hauptkultur des gleichen Mediums überführt und für 3 Tage bei 28°C und 160 rpm in einem Schikanekolben kultiviert.

Die Sterole und Squalen wurden nach 3 Tagen extrahiert (Parks LW, Bottema CD, Rodriguez RJ, Lewis TA. (1985) Yeast sterols: yeast mutants as tools for the study of sterol metabolism. Methods Enzymol. 1985;111:333-46.) und mittels Gaschromatographie analysiert. Es ergaben sich folgende Werte (siehe Tabelle 2).

**Tabelle 2**

| | | Gehalt an Sterolen 1 bis 11 in [Peakfläche/gTS] | | | | | | | | | | |
|---|---|---|---|---|---|---|---|---|---|---|---|---|
| Nr | Stammbezeichnung | 1 | 2 | 3 | 4 | 5 | 6 | 7 | 8 | 9 | 10 | 11 |
| I | GRFtH1 | 9,9 | 0,8 | 0,3 | 1,2 | 1,1 | 1,0 | 0,0 | 0,0 | 0,0 | 0,0 | 4,7 |
| II | GRFtH1E1 | 6,8 | 1,9 | 0,4 | 1,5 | 2,2 | 2,1 | 0,0 | 0,0 | 0,0 | 0,0 | 6,9 |
| III | GRFtH1E11 | 9,9 | 0,4 | 0,7 | 2,3 | 1,9 | 1,9 | 0,0 | 0,0 | 0,0 | 0,0 | 5,0 |
| IV | GRFtH1E1E11 | 6,0 | 1,2 | 0,9 | 3,0 | 2,3 | 2,2 | 0,0 | 0,0 | 0,0 | 0,0 | 7,2 |
| V | GRFtH1E1E11 *erg5erg6* | 5,8 | 0,8 | 0,4 | 23,1 | 0,0 | 0,0 | 0,0 | 0,0 | 11,8 | 0,0 | 0,0 |
| VI | GRFtH1*erg5e rg6* | 9,9 | 0,8 | 0,3 | 12,6 | 0,0 | 0,0 | 0,0 | 0,0 | 7,1 | 0,0 | 0,0 |

| | | | | | | | | | | | | |
|---|---|---|---|---|---|---|---|---|---|---|---|---|
| - 1 =: Squalen - 2 =: Lanosterol - 3 =: Dimethyl-Zymosterol - 4 =: Zymosterol - 5 =: Fecosterol - 6 =: Episterol - 7 =: Cholesta-7,24-dienol - 8 =: Cholesta-8-enol - 9 =: Cholesta-5,7,24 trienol - 10 =: 7-Dehydrocholesterol - 11 =: Ergosterol | | | | | | | | | | | | |

### Beispiel 2

### Expression des heterologen Gens kodierend eine Δ8-Δ7-Isomerase (Ebp) aus der Maus (Mus musculus) in Hefe

Die cDNA-Sequenz der Δ8-Δ7-Isomerase aus *Mus musculus* (Moebius, F.F., Soellner, K.E.M., Fiechter, B., Huck, C.W., Bonn, G., Glossmann, H. (1999): Histidine77, Glutamic Acid123, Threonine126, Asparagine194, and Tryptophan197 of Human Emopamil Protein Are Required for in Vivo Sterol Δ8-Δ7 Ismerisation. Biochem. 38, 1119-1127.) wurde durch PCR aus dem cDNA-Klon IMAGp998A22757 (Host: *E.coli* DH10B) des Deutschen Resourcenzentrums für Genomforschung GmbH (Berlin) amplifiziert.

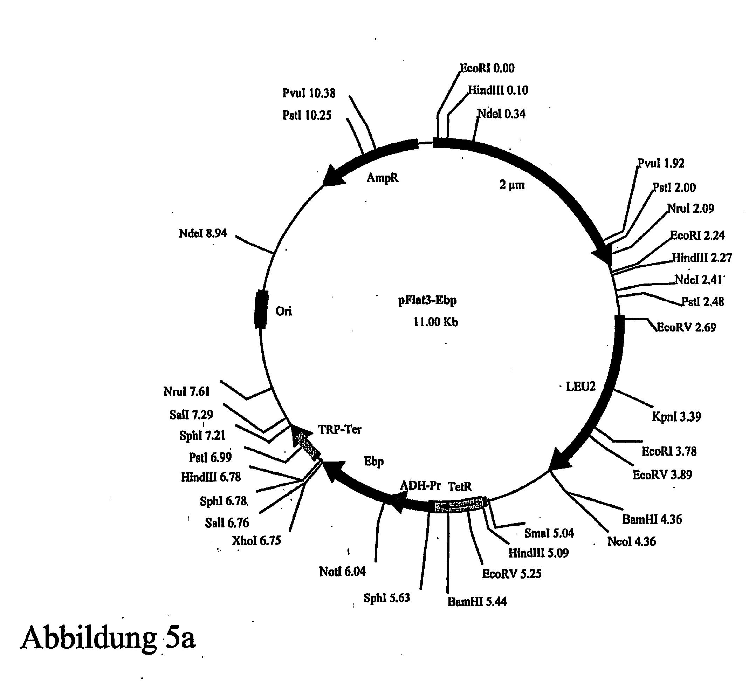

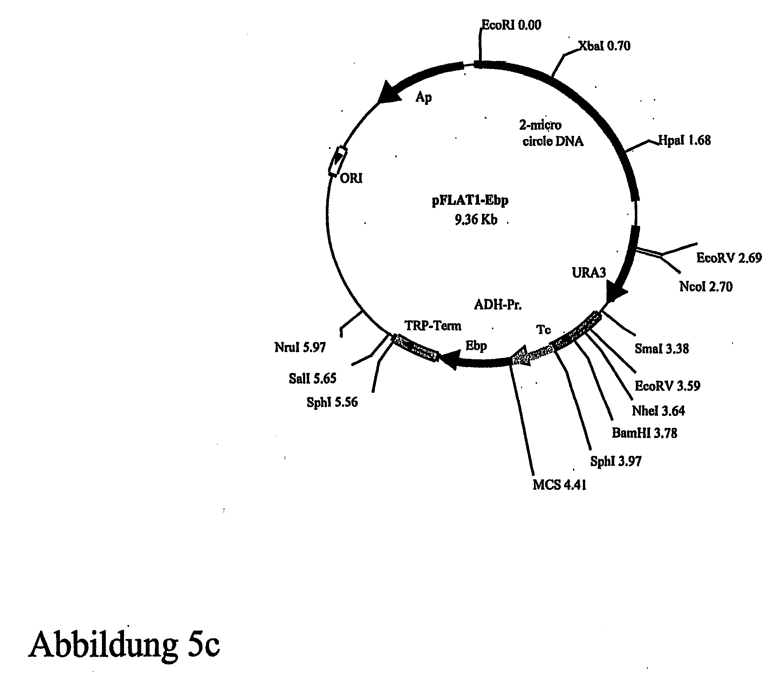

Die hierbei verwendeten Primer sind die DNA-Oligomere Ebp-5' (SEQ. ID. No. 43) und Ebp-3' (SEQ. ID. No. 44). Das erhaltene DNA-Fragment wurde mit den Restriktionsenzymen *Not*I und *XhoI* behandelt und anschliessend in den Vektor pFlat3 und pFlat1 (Abbildung 4), die zuvor ebenfalls mit den Enzymen *NotI* und *XhoI* behandelt wurden, mittels einer Ligase-Reaktion integriert. Die resultierenden Vektoren pFlat1-*EBP* und pFlat3-*EBP* (Abbildung 5a) enthalten das *EBP*-Gen unter der Kontrolle des *ADH*-Promotors und des Tryptophan-Terminators.

Der Expressionsvektor pFlat3-*EBP* wurde anschließend in die Hefestämme I bis VI der Tabelle 1 aus Beispiel 1 sowie in den Stamm GRFura3 transformiert. Die so gewonnenen Hefestämme wurden anschliessend 48 Stunden lang in WMVIII- Medium bei 28°C und 160 rpm in einem 20 ml Kulturvolumen kultiviert. Anschliessend wurden 500 µl dieser Vorkultur in eine 50 ml Hauptkultur des gleichen Mediums überführt und für 3 Tage bei 28°C und 160 rpm in einem Schikanekolben kultiviert.

Die Sterole wurden wie in Beispiel 1 beschrieben nach 3 Tagen extrahiert und mittels Gaschromatographie analysiert. Der Einfluss der Expression einer Δ8-Δ7-Isomerase aus *Mus musculus* in Kombination mit der Expression der transkriptionell deregulierten hefeeigenen Gene *tHMG* und/oder *ERG1* und/oder *ERG11* und/oder der Deletion der hefeeigenen Gene *ERG6* und ERG5 ist in Tabelle 3 aufgelistet. Die Abkürzungen bedeuten: - = Abnahme; 0 = keine Veränderung; / = nicht vorhanden; +, ++, +++, ++++ = angereichert bis stark angereichert.

**Tabelle 3**

| | | Einfluß der genetischen Veränderungen auf den Sterolgehalt gegenüber dem Hefe-Stamm GRF | | | | | | | | | | |
|---|---|---|---|---|---|---|---|---|---|---|---|---|
| Nr | Stammbezeichnung | 1 | 2 | 3 | 4 | 5 | 6 | 7 | 8 | 9 | 10 | 11 |
| VII | GRFtH1 pFlat3-Ebp | 0 | 0 | 0 | 0 | 0 | 0 | / | / | / | / | 0 |
| VIII | GRFtH1E1 pFlat3-Ebp | 0 | 0 | 0 | - | 0 | 0 | + | / | / | / | 0 |
| IX | GRFtH1E11 pFlat3-Ebp | 0 | 0 | 0 | - | 0 | 0 | + | / | / | / | 0 |
| X | GRFtH1E1E11 pFlat3-Ebp | 0 | 0 | 0 | - | 0 | 0 | + | / | / | / | 0 |
| XI | GRFtH1E1E11*erg5e rg6* pFlat3-Ebp | 0 | 0 | 0 | - | / | / | + | / | ++ | / | / |
| XII | GRFtH1erg5*erg6* pFlat3-Ebp | 0 | 0 | 0 | - | / | / | + | / | + | / | / |

| | | | | | | | | | | | | |
|---|---|---|---|---|---|---|---|---|---|---|---|---|
| - 1 =: Squalen - 2 =: Lanosterol - 3 =: Dimethyl-Zymosterol - 4 =: Zymosterol - 5 =: Fecosterol - 6 =: Episterol - 7 =: Cholesta-7,24-dienol - 8 =: Cholesta-8-enol - 9 =: Cholesta-5,7,24 trienol - 10 =: 7-Dehydrocholesterol - 11 =: Ergosterol | | | | | | | | | | | | |

### Beispiel 3

### Expression des heterologen Gens kodierend eine Δ5-Desaturase (Sc5d) aus der Maus (Mus musculus) in Hefe

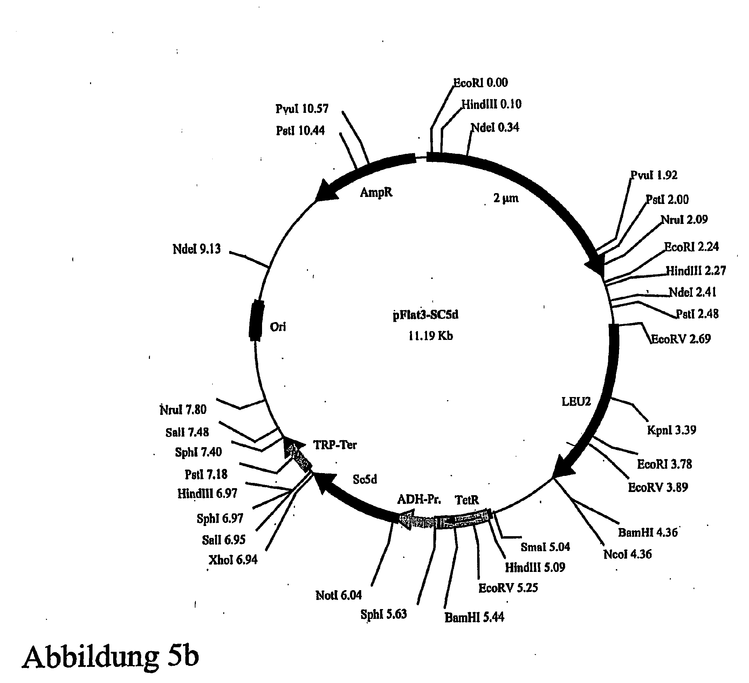

Die cDNA-Sequenz der Δ5-Desaturase aus *Mus musculus* (Nishi, S., Hideaki, N., Ishibashi, T. (2000): cDNA cloning of the mammalian sterol C5-desaturase and the expression in yeast mutant. Biochim. Biophys.A 1490, 106-108.) wurde durch PCR aus dem cDNA-Klon IMAGp998K144618 (Host: *E.coli* DH10B) des Deutschen Resourcenzentrums für Genomforschung GmbH (Berlin) amplifiziert. Die hierbei verwendeten Primer sind die DNA-Oligomere Sc5d-5' (SEQ. ID. No. 45) und Sc5d-3' (SEQ. ID. No. 46). Das erhaltene DNA-Fragment wurde mit den Restriktionsenzymen *NotI* und *XhoI* behandelt und anschliessend in den Vektor pFlat3 (Abbildung 4), der zuvor ebenfalls mit den Enzymen *NotI* und *XhoI* behandelt wurde, mittels einer Ligase-Reaktion integriert. Der resultierende Vektor pFlat3-*SC5D* (Abbildung 5b) enthält das *SC5D*-Gen unter der Kontrolle des *ADH*-Promotors und des Tryptophan-Terminators.

Der Expressionsvektor pFlat3-*SC5D* wurde anschliessend in die Hefestämme I bis VI der Tabelle 1 aus Beispiel 1 sowie in den Stamm GRF*ura3* transformiert. Die so gewonnenen Hefestämme wurden anschliessend 48 Stunden lang in WMVIII- Medium bei 28°C und 160 rpm in einem 20 ml Kulturvolumen kultiviert. Anschliessend wurden 500 µl dieser Vorkultur in eine 50 ml Hauptkultur des gleichen Mediums überführt und für 3 Tage bei 28°C und 160 rpm in einem Schikanekolben kultiviert.

Die Sterole wurden wie in Beispiel 1 beschrieben nach 3 Tagen extrahiert und mittels Gaschromatographie analysiert. Der Einfluss der Expression einer Δ5-Desaturase aus *Mus musculus* in Kombination mit der Expression der transkriptionell deregulierten hefeeigenen Gene *tHMG* und/oder *ERG1* und/oder *ERG11* und/oder der Deletion der hefeeigenen Gene *ERG6* und *ERG5* ist in Tabelle 4 aufgelistet. Die Abkürzungen bedeuten: - = Abnahme; 0 = keine Veränderung; / = nicht vorhanden; +, ++, +++, ++++ = angereichert bis stark angereichert.

**Tabelle 4**

| | | Einfluß der genetischen Veränderungen auf den Sterolgehalt gegenüber dem Hefe-Stamm GRF | | | | | | | | | | |
|---|---|---|---|---|---|---|---|---|---|---|---|---|
| Nr | Stammbezeichnung | 1 | 2 | 3 | 4 | 5 | 6 | 7 | 8 | 9 | 10 | 11 |
| XIII | GRFtH1 pFlat3-Sc5d | 0 | 0 | 0 | 0 | 0 | 0 | / | / | / | / | 0 |
| XIV | GRFtH1E1 pFlat3-Sc5d | 0 | 0 | 0 | - | 0 | 0 | / | / | + | / | 0 |
| XV | GRFtH1E11 pFlat3-Sc5d | 0 | 0 | 0 | - | 0 | 0 | / | / | + | / | 0 |
| XVI | GRFtH1E1E11 pFlat3-Sc5d | 0 | 0 | 0 | - | 0 | 0 | / | / | + | / | 0 |
| XVII | GRFtH1E1E11*erg5e rg6* pFlat3-Sc5d | 0 | - | 0 | - | / | / | / | / | ++ + | + | / |
| XVIII | GRFtH1*erg5erg6* pFlat3-Sc5d | 0 | 0 | 0 | - | / | / | / | / | ++ | / | / |

| | | | | | | | | | | | | |
|---|---|---|---|---|---|---|---|---|---|---|---|---|
| - 1 =: Squalen - 2 =: Lanosterol - 3 =: Dimethyl-Zymosterol - 4 =: Zymosterol - 5 =: Fecosterol - 6 =: Episterol - 7 =: Cholesta-7,24-dienol - 8 =: Cholesta-8-enol - 9 =: Cholesta-5,7,24 trienol - 10 =: 7-Dehydrocholesterol - 11 =: Ergosterol | | | | | | | | | | | | |

### Beispiel 4

### Expression des heterologen Gens kodierend eine A24-Reduktase (D24R) aus der Maus (Mus musculus) in Hefe

Die cDNA-Sequenz der Δ24-Reduktase aus *Mus musculus* (Waterham,H.R., Koster,J., Romeijn,G.J., Hennekam,R.C., Vreken,P.,Andersson,H.C., FitzPatrick,D.R., Kelley,R.I. and Wanders,R.J., Mutations in the 3beta-Hydroxysterol Delta24-Reductase Gene Cause Desmosterolosis, an Autosomal Recessive 'Disorder of Cholesterol Biosynthesis, Am. J. Hum. Genet. 69 (4), 685-694 (2001)) wurde durch PCR aus dem cDNA-Klon IMAGp998K179532 (Host: E.coli DH10B) des Deutschen Resourcenzentrums für Genomforschung GmbH (Berlin) amplifiziert.

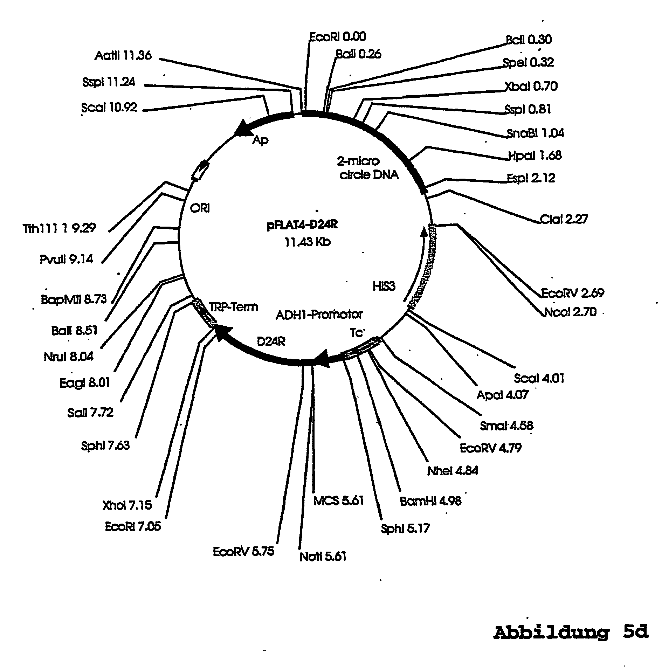

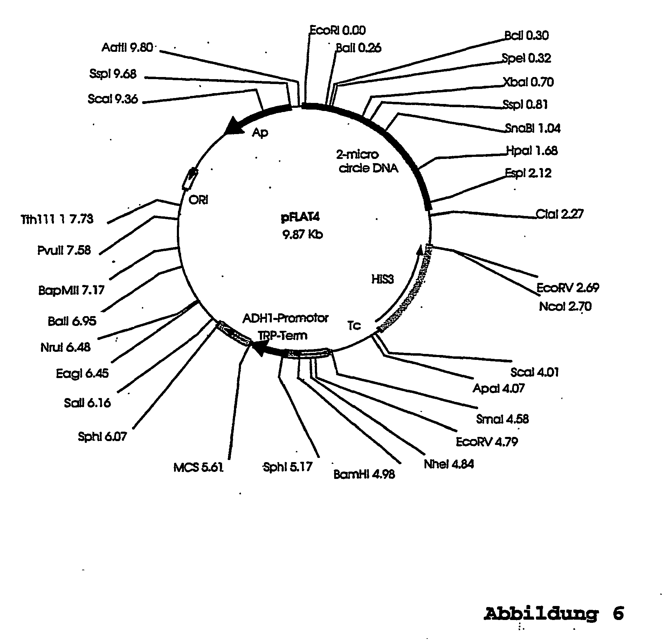

Die hierbei verwendeten Primer sind die DNA-Oligomere D24R-5' (SEQ. ID. No. 47) und D24R-3' (SEQ. ID. No. 48). Das erhaltene DNA-Fragment wurde mit den Restriktionsenzymen *NotI* und *XhoI* behandelt und anschliessend in den Vektor pFlat4 (Abbildung 6), der zuvor ebenfalls mit den Enzymen *NotI* und *XhoI* behandelt wurde, mittels einer Ligase-Reaktion integriert. Der resultierende Vektor pFlat4-*D24R* (Abbildung 5d) enthält das *D24R*-Gen unter der Kontrolle des *ADH1*-Promotors und des Tryptophan-Terminators.

Der Expressionsvektor pFlat4-*D24R* wurde anschliessend in die Hefestämme I bis VI der Tabelle 1 aus Beispiel 1 sowie in den Stamm GRFura3 transformiert. Die so gewonnenen Hefestämme wurden anschliessend 48 Stunden lang in WMVIII- Medium bei 28°C und 160 rpm in einem 20 ml Kulturvolumen kultiviert. Anschliessend wurden 500 µl dieser Vorkultur in eine 50 ml Hauptkultur des gleichen Mediums überführt und für 3 Tage bei 28°C und 160 rpm in einem Schikanekolben kultiviert.

Die Sterole wurden, wie in Beispiel 1 beschrieben, nach 3 Tagen extrahiert und mittels Gaschromatographie analysiert. Der Einfluss der Expression einer A24-Reduktase aus *Mus musculus* in Kombination mit der Expression der transkriptionell deregulierten hefeeigenen Gene *tHMG* und/oder *ERG1* und/oder *ERG11* und/oder der Deletion der hefeeigenen Gene *ERG6* und *ERG5* ist in Tabelle 5 aufgelistet. Die Abkürzungen bedeuten: - = Abnahme; 0 = keine Veränderung; / = nicht vorhanden; +, ++, +++, ++++ = angereichert bis stark angereichert.

**Tabelle 5**

| | | Einfluß der genetischen Veränderungen auf den Sterolgehalt gegenüber dem Hefe-Stamm GRF | | | | | | | | | | |
|---|---|---|---|---|---|---|---|---|---|---|---|---|
| Nr | Stammbezeichnung | 1 | 2 | 3 | 4 | 5 | 6 | 7 | 8 | 9 | 10 | 11 |
| XIX | GRFtH1 pFlat4-D24R | 0 | 0 | 0 | 0 | 0 | 0 | / | / | / | / | 0 |
| XX | GRF1H1E1 pFlat4-D24R | 0 | - | - | - | 0 | 0 | / | / | / | + | 0 |
| XXI | GRFtH1E11 pFlat4-D24R | 0 | 0 | 0 | - | 0 | 0 | / | + | / | + | 0 |
| XXII | GRFtH1E1E11 pFlat4-D24R | 0 | 0 | 0 | - | 0 | 0 | / | + | / | + | 0 |
| XXIII | GRFtH1E1E11*erg5e rg6* pFlat4-D24R | 0 | - | - | --- | / | / | 0 | + | + | ++ + | / |
| XXIV | GRFtH1*erg5erg6* pFlat4-D24R | 0 | - | - | - | / | / | 0 | + | + | ++ | / |

| | | | | | | | | | | | | |
|---|---|---|---|---|---|---|---|---|---|---|---|---|
| - 1 =: Squalen - 2 =: Lanosterol - 3 =: Dimethyl-Zymosterol - 4 =: Zymosterol - 5 =: Fecosterol - 6 =: Episterol - 7 =: Cholesta-7,24-dienol - 8 =: Cholesta-8-enol - 9 =: Cholesta-5,7,24 trienol - 10 =: 7-Dehydrocholesterol - 11 =: Ergosterol | | | | | | | | | | | | |

### Beispiel 5

### Gemeinsame Expression der heterologen Gene kodierende eine Δ8-Δ7 Isomerase (Ebp) aus der Maus (Mus musculus) und eine C5-Desaturase (Sc5d) aus der Maus (Mus musculus) in Hefe

Die Expressionsvektoren pFlat1-*EBP* (aus Beispiel 2) und pFlat3-*SC5D* (aus Beispiel 3) wurden in die Hefestämme I bis VI der Tabelle 1 aus Beispiel 1 sowie in den Stamm *GRFura3* transformiert. Die so gewonnenen Hefestämme wurden anschliessend 48 Stunden lang in WMVIII- Medium bei 28°C und 160 rpm in einem 20 ml Kulturvolumen kultiviert. Anschliessend wurden 500 µl dieser Vorkultur in eine 50 ml Hauptkultur des gleichen Mediums überführt und für 3 Tage bei 28°C und 160 rpm in einem Schikanekolben kultiviert.

Die Sterole wurden, wie in Beispiel 1 beschrieben, nach 3 Tagen extrahiert und mittels Gaschromatographie analysiert. Der Einfluss der Expression einer Δ8-Δ7 Isomerase und einer CS-Desaturase aus *Mus musculus* in Kombination mit der Expression der transkriptionell deregulierten hefeeigenen Gene *tHMG* und/oder *ERG1* und/oder *ERG11* und/oder der Deletion der hefeeigenen Gene *ERG6* und *ERG5* ist in Tabelle 6 aufgelistet. Die Abkürzungen bedeuten: - = Abnahme; 0 = keine Veränderung; / = nicht vorhanden; +, ++, +++, ++++ = angereichert bis stark angereichert.

**Tabelle 6**

| | | Einfluß der genetischen Veränderungen auf den Sterolgehalt gegenüber dem Hefe-Stamm GRF | | | | | | | | | | |
|---|---|---|---|---|---|---|---|---|---|---|---|---|
| Nr | Stammbezeichnung | 1 | 2 | 3 | 4 | 5 | 6 | 7 | 8 | 9 | 10 | 11 |
| XXV | GRFtH1 pFlat3-Ebp/ pFlat1-Sc5d | 0 | 0 | 0 | - | 0 | 0 | / | / | + | / | 0 |
| XXVI | GRFtH1E1 pFlat3-Ebp/ pFlat1-Sc5d | 0 | 0 | 0 | -- | 0 | 0 | / | / | + | / | 0 |
| XXVII | GRFtH1E11 pFlat3-Ebp/ pFlat1-Sc5d | 0 | 0 | 0 | -- | 0 | 0 | / | / | + | / | 0 |
| XXVIII | GRFtH1E1E11 pFlat3-Ebp/ pFlat1-Sc5d | 0 | - | - | -- | 0 | 0 | / | / | ++ | / | 0 |
| XXIX | GRFtH1E1E11*erg5e rg6* pFlat3-Ebp/ pFlat1-Sc5d | 0 | - | 0 | -- | / | / | / | / | ++ + | + | / |
| XXX | GRFtH1*erg5erg6* pFlat3-Ebp/ pFlat1-Sc5d | 0 | 0 | 0 | - | / | / | / | / | ++ | + | / |

| | | | | | | | | | | | | |
|---|---|---|---|---|---|---|---|---|---|---|---|---|
| - 1 =: Squalen - 2 =: Lanosterol - 3 =: Dimethyl-Zymosterol - 4 =: Zymosterol - 5 =: Fecosterol - 6 =: Episterol - 7 =: Cholesta-7,24-dienol - 8 =: Cholesta-8-enol - 9 =: Cholesta-5,7,24 trienol - 10 =: 7-Dehydrocholesterol - 11 =: Ergosterol | | | | | | | | | | | | |

### Beispiel 6

### Gemeinsame Expression der heterologen Gene kodierend eine Δ8-Δ7 Isomerase (Ebp) aus der Maus (Mus musculus), kodierend eine C5-Desaturase (Sc5d) aus der Maus (Mus musculus) und eine Δ24-Reduktase aus der Maus (Mus musculus) in Hefe

Die Expressionsvektoren pFlat1-*EBP* (aus Beispiel 2) und pFlat3-*SC5D* (aus Beispiel 3) und pFlat4-*D24R* (aus Beispiel 4) wurden in die Hefestämme I bis VI der Tabelle 1 aus Beispiel 1 sowie in den Stamm GRFura3 transformiert. Die so gewonnenen Hefestämme wurden anschliessend 48 Stunden lang in WMVIII- Medium bei 28°C und 160 rpm in einem 20 ml Kulturvolumen kultiviert. Anschliessend wurden 500 µl dieser Vorkultur in eine 50 ml Hauptkultur des gleichen Mediums überführt und für 3 Tage bei 28°C und 160 rpm in einem Schikanekolben kultiviert.

Die Sterole wurden, wie in Beispiel 1 beschrieben, nach 3 Tagen extrahiert und mittels Gaschromatographie analysiert. Der Einfluss der Expression einer A8-A7 Isomerase, einer C5-Desaturase und einer A24-Reduktase aus *Mus musculus* in Kombination mit der Expression der transkriptionell deregulierten hefeeigenen Gene *tHMG* und/oder *ERG1* und/oder *ERG11* und/oder der Deletion der hefeeigenen Gene *ERG6* und *ERG5* ist in Tabelle 7 aufgelistet. Die Abkürzungen bedeuten: - = Abnahme; 0 = keine Veränderung; / = nicht vorhanden; +, ++, +++, ++++ = angereichert bis stark angereichert.

**Tabelle 7**

| | | Einfluß der genetischen Veränderungen auf den Sterolgehalt gegenüber dem Hefe-Stamm GRF | | | | | | | | | | |
|---|---|---|---|---|---|---|---|---|---|---|---|---|
| Nr | Stammbezeichnung | 1 | 2 | 3 | 4 | 5 | 6 | 7 | 8 | 9 | 10 | 11 |
| | | | | | | | | | | | | |
| XXXI | GRFtH1 pFlat3-Ebp/ pFlat1-Sc5d/ pFlat4-*D24R* | 0 | 0 | 0 | - | 0 | 0 | / | / | / | + | 0 |
| XXXII | GRFtH1E1 pFlat3-Ebp/ pFlat1-Sc5d/ pFlat4-*D24R* | 0 | - | 0 | -- | 0 | 0 | / | / | / | + | 0 |
| XXXIII | GRFtH1E11 pFlat3-Ebp/ pFlat1-Sc5d/ pFlat4-*D24R* | 0 | 0 | 0 | -- | 0 | 0 | / | / | / | + | 0 |
| XXXIV | GRFtH1E1E11 pFlat3-Ebp/ pFlat1-Sc5d/ pFlat4-*D24R* | 0 | - | - | -- | 0 | 0 | / | / | / | ++ | 0 |
| XXXV | GRFtH1E1E11*erg5e rg6* pFlat3-Ebp/ pFlat1-Sc5d/ pFlat4-*D24R* | 0 | - | 0 | --- | / | / | / | / | + | ++ ++ | / |
| XXXVI | GRFtH1*erg5erg6* pFlat3-Ebp/ pFlat1-Sc5d/ pFlat4-*D24R* | 0 | 0 | 0 | - | / | / | / | / | ++ | ++ + | / |

| | | | | | | | | | | | | |
|---|---|---|---|---|---|---|---|---|---|---|---|---|
| - 1 =: Squalen - 2 =: Lanosterol - 3 =: Dimethyl-Zymosterol - 4 =: Zymosterol - 5 =: Fecosterol - 6 =: Episterol - 7 =: Cholesta-7,24-dienol - 8 =: Cholesta-8-enol - 9 =: Cholesta-5,7,24 trienol - 10 =: 7-Dehydrocholesterol - 11 =: Ergosterol | | | | | | | | | | | | |

## Patentansprüche

1. Verfahren zur Herstellung von 7-Dehydrocholesterol durch Kultivierung von Organismen die gegenüber dem Wildtyp eine erhöhte delta24-Reduktase-Aktivität und HMG-CoA-Reduktase-Aktivität aufweisen, sowie entwedereine erhöhte delta8-delta7-Isomerase-Aktivität und delta5-Desaturase-Aktivität, oder eine erhöhte Squalenepoxidase-Aktivität aufweisen.

2. Verfahren nach Anspruch 1, **dadurch gekennzeichnet, dass** man zur Erhöhung der delta8-delta7-Isomerase-Aktivität die Genexpression einer Nukleinsäure codierend eine delta8-delta7-Isomerase gegenüber dem Wildtyp erhöht.

3. Verfahren nach Anspruch 2, **dadurch gekennzeichnet, dass** man zur Erhöhung der Genexpression eine oder mehrere Nukleinsäuren, codierend eine delta8-delta7-Isomerase, in den Organismus einbringt.

4. Verfahren nach Anspruch 3, **dadurch gekennzeichnet, dass** man Nukleinsäuren einbringt, die Proteine kodieren, enthaltend die Aminosäuresequenz SEQ. ID. NO. 2 oder eine von dieser Sequenz durch Substitution, Insertion oder Deletion von Aminosäuren abgeleitete Sequenz, die eine Identität von mindestens 30% auf Aminosäureebene mit der Sequenz SEQ. ID. NO. 2, und die die enzymatische Eigenschaft einer delta8-delta7-Isomerase aufweisen.

5. Verfahren nach Anspruch 4, **dadurch gekennzeichnet, dass** man eine Nukleinsäure, enthaltend die Sequenz SEQ. ID. NO. 1 einbringt.

6. Verfahren nach einem der Ansprüche 1 bis 5, **dadurch gekennzeichnet, dass** man zur Erhöhung der delta5-Desaturase-Aktivität die Genexpression einer Nukleinsäure, codierend eine delta5-Desaturase, gegenüber dem Wildtyp erhöht.

7. Verfahren nach Anspruch 6, **dadurch gekennzeichnet, dass** man zur Erhöhung der Genexpression eine oder mehrere Nukleinsäuren codierend eine delta5-Desaturase, in den Organismus einbringt.

8. Verfahren nach Anspruch 7, **dadurch gekennzeichnet, dass** man Nukleinsäuren einbringt, die Proteine kodieren, enthaltend die Aminosäuresequenz SEQ. ID. NO. 4 oder eine von dieser Sequenz durch Substitution, Insertion oder Deletion von Aminosäuren abgeleitete Sequenz, die eine Identität von mindestens 30% auf Aminosäureebene mit der Sequenz SEQ. ID. NO. 4, und die die enzymatische Eigenschaft einer delta5-Desaturase aufweisen.

9. Verfahren nach Anspruch 8, **dadurch gekennzeichnet, dass** man eine Nukleinsäure, enthaltend die Sequenz SEQ. ID. NO. 3 einbringt.

10. Verfahren nach einem der Ansprüche 1 bis 9, **dadurch gekennzeichnet, dass** man zur Erhöhung der delta24-Reduktase-Aktivität die Genexpression einer Nukleinsäure, codierend eine delta24-Reduktase, gegenüber dem Wildtyp erhöht.

11. Verfahren nach Anspruch 10, **dadurch gekennzeichnet, dass** man zur Erhöhung der Genexpression eine oder mehrere Nukleinsäuren codierend eine delta24-Reduktase, in den Organismus einbringt.

12. Verfahren nach Anspruch 11, **dadurch gekennzeichnet, dass** man Nukleinsäuren einbringt, die Proteine kodieren, enthaltend die Aminosäuresequenz SEQ. ID. NO. 6 oder eine von dieser Sequenz durch Substitution, Insertion oder Deletion von Aminosäuren abgeleitete Sequenz, die eine Identität von mindestens 30% auf Aminosäureebene mit der Sequenz SEQ. ID. NO. 6, und die die enzymatische Eigenschaft einer delta24-Reduktase aufweisen.

13. Verfahren nach Anspruch 12, **dadurch gekennzeichnet, dass** man eine Nukleinsäure, enthaltend die Sequenz SEQ. ID. NO. 5 einbringt.

14. Verfahren nach einem der Ansprüche 1 bis 13, **dadurch gekennzeichnet, dass** die Organismen gegenüber dem Wildtyp zusätzlich eine reduzierte Aktivität mindestens einer der Aktivitäten, ausgewählt aus der Gruppe C24-Methyltransferase-Aktivität und delta22-Desaturase-Aktivität aufweisen.

15. Verfahren gemäß Anspruch 14, **dadurch gekennzeichnet, dass** die Organismen gegenüber dem Wildtyp eine reduzierte C24-Methyltransferase-Aktivität und eine reduzierte delta22-Desaturase-Aktivität aufweisen.

16. Verfahren nach Anspruch 14 oder 15, **dadurch gekennzeichnet, dass** man zur Reduzierung der C24-Methyltransferase-Aktivität die Genexpression einer Nukleinsäure codierend eine C24-Methyltransferase gegenüber dem Wildtyp reduziert.

17. Verfahren nach Anspruch 16, **dadurch gekennzeichnet, dass** man einen Organismus verwendet, der kein funktionelles C24-Methyltransferase-Gen aufweist.

18. Verfahren nach einem der Ansprüche 14 bis 17, **dadurch gekennzeichnet, dass** man zur Reduzierung der delta22-Desaturase-Aktivität die Genexpression einer Nukleinsäure codierend eine delta22-Desaturase gegenüber dem Wildtyp reduziert.

19. Verfahren nach Anspruch 18, **dadurch gekennzeichnet, dass** man einen Organismus verwendet, der kein funktionelles delta22-Desaturase-Gen aufweist.

20. Verfahren nach einem der Ansprüche 1 bis 19, **dadurch gekennzeichnet, dass** die Organismen zusätzlich gegenüber dem Wildtyp eine erhöhte Aktivität mindestens einer der Aktivitäten, ausgewählt aus der Gruppe Lanosterol-C14-Demethylase-Aktivität, Squalensynthetase-Aktivität und Sterol-Acyltransferase-Aktivität aufweisen.

21. Verfahren nach Anspruch 20, **dadurch gekennzeichnet, dass** die Organismen zusätzlich gegenüber dem Wildtyp eine erhöhte Lanosterol-C14-Demethylase-Aktivität aufweisen.

22. Verfahren nach Anspruch 20 oder 21, **dadurch gekennzeichnet, dass** man zur Erhöhung der Lanosterol-C14-Demethylase-Aktivität die Genexpression einer Nukleinsäure codierend eine Lanosterol-C14-Demethylase gegenüber dem Wildtyp erhöht.

23. Verfahren nach Anspruch 22, **dadurch gekennzeichnet, dass** man zur Erhöhung der Genexpression eine oder mehrere Nukleinsäuren codierend eine Lanosterol-C14-Demethylase, in den Organismus einbringt.

24. Verfahren nach Anspruch 23, **dadurch gekennzeichnet, dass** man Nukleinsäuren einbringt, die Proteine kodieren, enthaltend die Aminosäuresequenz SEQ. ID. NO. 8 oder eine von dieser Sequenz durch Substitution, Insertion oder Deletion von Aminosäuren abgeleitete Sequenz, die eine Identität von mindestens 30% auf Aminosäureebene mit der Sequenz SEQ. ID. NO. 8, und die die enzymatische Eigenschaft einer Lanosterol-C14-Demethylase aufweisen.

25. Verfahren nach Anspruch 24, **dadurch gekennzeichnet, dass** man eine Nukleinsäure, enthaltend die Sequenz SEQ. ID. NO. 7 einbringt.

26. Verfahren nach einem der Ansprüche 1 bis 25, **dadurch gekennzeichnet, dass** man zur Erhöhung der HMG-CoA-Reduktase-Aktivität die Genexpression einer Nukleinsäure codierend eine HMG-CoA-Reduktase gegenüber dem Wildtyp erhöht.

27. Verfahren nach Anspruch 26, **dadurch gekennzeichnet, dass** man zur Erhöhung der Genexpression ein Nukleinsäurekonstrukt, enthaltend eine Nukleinsäure codierend eine HMG-CoA-Reduktase in den Organismus einbringt, deren Expression in dem Organismus, verglichen mit dem Wildtyp, einer reduzierten Regulation unterliegt.

28. Verfahren nach Anspruch 27, **dadurch gekennzeichnet, dass** das Nukleinsäurekonstrukt einen Promotor enthält, der in dem Organismus, verglichen mit dem Wildtyp-Promotor, einer reduzierten Regulation unterliegt.

29. Verfahren nach Anspruch 27 oder 28, **dadurch gekennzeichnet, dass** man als Nukleinsäure codierend eine HMG-CoA-Reduktase eine Nukleinsäure verwendet, deren Expression in dem Organismus, verglichen mit der Organismus eigenen, orthologen Nukleinsäure, einer reduzierten Regulation unterliegt.

30. Verfahren nach Anspruch 29, **dadurch gekennzeichnet, dass** man als Nukleinsäure codierend eine HMG-CoA-Reduktase eine Nukleinsäure verwendet, die den katalytischen Bereich der HMG-CoA-Reduktase kodiert.

31. Verfahren nach Anspruch 30, **dadurch gekennzeichnet, dass** man Nukleinsäuren einbringt, die Proteine kodieren, enthaltend die Aminosäuresequenz SEQ. ID. NO. 10 oder eine von dieser Sequenz durch Substitution, Insertion oder Deletion von Aminosäuren abgeleitete Sequenz, die eine Identität von mindestens 30% auf Aminosäureebene mit der Sequenz SEQ. ID. NO. 10, und die die enzymatische Eigenschaft einer HMG-CoA-Reduktase aufweisen.

32. Verfahren nach Anspruch 31, **dadurch gekennzeichnet, dass** man eine Nukleinsäure, enthaltend die Sequenz SEQ. ID. NO. 9 einbringt.

33. Verfahren nach einem der Ansprüche 1 bis 32, **dadurch gekennzeichnet, dass** man einen Organismus verwendet, der zusätzlich gegenüber dem Wildtyp eine erhöhte Squalenepoxidase-Aktivität aufweist.

34. Verfahren nach Anspruch 33, **dadurch gekennzeichnet, dass** man zur Erhöhung der Squalenepoxidase-Aktivität die Genexpression einer Nukleinsäure codierend eine Squalenepoxidase gegenüber dem Wildtyp erhöht.

35. Verfahren nach Anspruch 34, **dadurch gekennzeichnet, dass** man zur Erhöhung der Genexpression eine oder mehrere Nukleinsäuren codierend eine Squalenepoxidase, in den Organismus einbringt.

36. Verfahren nach Anspruch 35, **dadurch gekennzeichnet, dass** man Nukleinsäuren einbringt, die Proteine kodieren, enthaltend die Aminosäuresequenz SEQ. ID. NO. 12 oder eine von dieser Sequenz durch Substitution, Insertion oder Deletion von Aminosäuren abgeleitete Sequenz, die eine Identität von mindestens 30% auf Aminosäureebene mit der Sequenz SEQ. ID. NO. 12, und die die enzymatische Eigenschaft einer Squalenepoxidase aufweisen.

37. Verfahren nach Anspruch 36, **dadurch gekennzeichnet, dass** man eine Nukleinsäure, enthaltend die Sequenz SEQ. ID. NO. 11 einbringt.

38. Verfahren nach einem der Ansprüche 1 bis 38, **dadurch gekennzeichnet, dass** man als Organismus Hefe verwendet.

39. Verfahren nach einem der Ansprüche 1 bis 38, **dadurch gekennzeichnet, dass** man nach dem Kultivieren den Organismus erntet und anschließend 7-Dehydrocholesterol aus dem Organismus isoliert.

40. Nukleinsäurekonstrukt, enthaltend a) mindestens eine Nukleinsäure codierend eine delta24-Reduktase, die mit einem oder mehreren Regulationssignalen funktionell verknüpft sind, die die Transkription und Translation in Organismen gewährleisten, b) mindestens eine Nukleinsäuren codierend eine HMG-CoA-Reduktase, und entweder c1) mindestens eine Nukleinsäure codierend eine delta8-delta7-Isomerase und eine Nukleinsäure codierend eine delta5-Desaturase, oder c2) eine Nukleinsäure codierend eine Squalenepoxidase, wobei die Komponenten b) sowie c1) oder c2) mit einem oder mehreren Regulationssignalen funktionell verknüpft sind, die die Transkription und Translation in Organismen gewährleisten.

41. Nukleinsäurekonstrukt nach Anspruch 40, enthaltend zusätzlich mindestens eine Nukleinsäure, ausgewählt aus der Gruppe Nukleinsäuren codierend eine Lanosterol-C14-Demethylase, , Nukleinsäuren codierend eine Squalensynthetase und Nukleinsäuren codierend eine Sterol-Acyltransferase, die mit einem oder mehreren Regulationssignalen funktionell verknüpft sind, die die Transkription und Translation in Organismen gewährleisten.

42. Kombination aus Nukleinsäurekonstrukten, wobei die Kombination mindestens ein Nukleinsäurekonstrukt, ausgewählt aus der Gruppe A bis C
A Nukleinsäurekonstrukt, enthaltend Nukleinsäuren codierend eine delta8-delta7-Isomerase, die mit einem oder mehreren Regulationssignalen funktionell verknüpft sind, die die Transkription und Translation in Organismen gewährleisten,
B Nukleinsäurekonstrukt, enthaltend Nukleinsäuren codierend eine delta5-Desaturase, die mit einem oder mehreren Regulationssignalen funktionell verknüpft sind, die die Transkription und Translation in Organismen gewährleisten und
C Nukleinsäurekonstrukt, enthaltend Nukleinsäuren codierend eine delta24-Reduktase, die mit einem oder mehreren Regulationssignalen funktionell verknüpft sind, die die Transkription und Translation in Organismen gewährleisten,
und mindestens ein Nukleinsäurekonstrukt, ausgewählt aus der Gruppe D bis H
D Nukleinsäurekonstrukt, enthaltend Nukleinsäuren codierend eine HMG-CoA-Reduktase, die mit einem oder mehreren Regulationssignalen funktionell verknüpft sind, die die Transkription und Translation in Organismen gewährleisten,
E Nukleinsäurekonstrukt, enthaltend Nukleinsäuren codierend eine Lanosterol-C14-Demethylase, die mit einem oder mehreren Regulationssignalen funktionell verknüpft sind, die die Transkription und Translation in Organismen gewährleisten,
F Nukleinsäurekonstrukt, enthaltend Nukleinsäuren codierend eine Squalenepoxidase, die mit einem oder mehreren Regulationssignalen funktionell verknüpft sind, die die Transkription und Translation in Organismen gewährleisten,
G Nukleinsäurekonstrukt, enthaltend Nukleinsäuren codierend eine Squalensynthetase, die mit einem oder mehreren Regulationssignalen funktionell verknüpft sind, die die Transkription und Translation in Organismen gewährleisten,
H Nukleinsäurekonstrukt, enthaltend Nukleinsäuren codierend eine Sterol-Acyltransferase, die mit einem oder mehreren Regulationssignalen funktionell verknüpft sind, die die Transkription und Translation in Organismen gewährleisten
umfasst, wobei die Gruppen C und D sowie entweder A und B einerseits oder F andererseits zwingend eingerichtet sind.

43. Nukleinsäurekonstrukte oder Kombination von Nukleinsäurekonstrukten gemäß einem der Ansprüche 40 bis 42, **dadurch gekennzeichnet, dass** die Regulationssignale einen oder mehrere Promotoren und einen oder mehrere Terminatoren enthalten, die die Transkription und Translation in Organismen gewährleisten.

44. Nukleinsäurekonstrukte oder Kombination von Nukleinsäurekonstrukten gemäß Anspruch 43, **dadurch gekennzeichnet, dass** man Regulationssignale verwendet, die die Transkription und Translation in Hefen gewährleisten.

45. Genetisch veränderter Organismus, wobei die genetische Veränderung zumindest die delta24-Reduktase-Aktivität und die HMG-CoA-Reduktase-Aktivität gegenüber einem Wildtyp erhöht, wobei die Erhöhung der delta24-Reduktase-Aktivität durch eine Erhöhung der Genexpression mindestens einer Nukleinsäure, kodierend eine delta24-Reduktase, gegenüber dem Wildtyp bewirkt wird, wobei die genetische Veränderunge zusätzlich
entweder die Squalenepoxidase-Aktivität erhöht, oder wobei der Organismus zwei oder mehr Nukleinsäuren codierend eine delta8-delta7-Isomerase und/oder zwei oder mehr Nukleinsäuren codierend eine delta5-Desaturase enthält.

46. Genetisch veränderter Organismus nach Anspruch 45, **dadurch gekennzeichnet, dass** der Organismus zwei oder mehr Nukleinsäuren kodierend eine delta24-Reduktase enthält.

47. Genetisch veränderter Organismus nach einem der Ansprüche 45 bis 46, **dadurch gekennzeichnet, dass** die genetische Veränderung zusätzlich mindestens eine der Aktivitäten, ausgewählt aus der Gruppe C24-Methyltransferase-Aktivität und delta22-Desaturase-Aktivität gegenüber einem Wildtyp reduziert.

48. Genetisch veränderter Organismus nach Anspruch 47, **dadurch gekennzeichnet, dass** die Reduzierung mindestens einer der Aktivitäten durch eine Reduzierung der Genexpression mindestens einer Nukleinsäure, ausgewählt aus der Gruppe Nukleinsäuren codierend eine C24-Methyltransferase und Nukleinsäuren kodierend eine Delta22-Desaturase, gegenüber dem Wildtyp bewirkt wird.

49. Genetisch veränderter Organismus nach Anspruch 48, **dadurch gekennzeichnet, dass** der Organismus kein funktionelles C24-Methyltransferase-Gen und/oder delta22-Desaturase-Gen aufweist.

50. Genetisch veränderter Organismus nach einem der Ansprüche 45 bis 49, **dadurch gekennzeichnet, dass** die genetische Veränderung zusätzlich mindestens eine der Aktivitäten, ausgewählt aus der Gruppe Lanosterol-C14-Demethylase-Aktivität, , Squalensynthetase-Aktivität und Sterol-Acyltransferase-Aktivität, gegenüber einem Wildtyp erhöht.

51. Genetisch veränderter Organismus nach Anspruch 50, **dadurch gekennzeichnet, dass** die Erhöhung mindestens einer der Aktivitäten durch eine Erhöhung der Genexpression mindestens einer Nukleinsäure, ausgewählt aus der Gruppe Nukleinsäuren codierend eine Lanosterol-C14-Demethylase, Nukleinsäuren codierend eine Squalensynthetase und Nukleinsäuren codierend eine Sterol-Acyltransferase, gegenüber dem Wildtyp bewirkt wird.

52. Genetisch veränderter Organismus nach Anspruch 51, **dadurch gekennzeichnet, dass** der Organismus zwei oder mehr Nukleinsäuren codierend eine HMG-CoA-Reduktase und/oder zwei oder mehr Nukleinsäuren codierend eine Lanosterol-C14-Demethylase und/ oder zwei oder mehr Nukleinsäuren codierend eine Squalenepoxidase und/oder zwei oder mehr Nukleinsäuren codierend eine Squalensynthetase und/oder zwei oder mehr Nukleinsäuren codierend eine Sterol-Acyltransferase enthält.

53. Genetisch veränderter Organismus nach einem der Ansprüche 45 bis 52, **dadurch gekennzeichnet, dass** der genetisch veränderte Organismus gegenüber dem Wildtyp einen erhöhten Gehalt an 7-Dehydrocholesterol aufweist.

54. Genetisch veränderter Organismus nach einem der Ansprüche 45 bis 53, **dadurch gekennzeichnet dass** man als Organismus Hefe verwendet.

55. Verwendung eines genetisch veränderten Organismus nach einem der Ansprüche 45 bis 54 zur Herstellung von 7-Dehydrocholesterol.

## Claims

1. A method for preparing 7-dehydrocholesterol by culturing organism, which, compared to the wild type, have an increased activity of delta24-reductase and an increased activity of HMG-CoA-reductase, and either an increased activity of delta8-delta7-isomerase and an increased activity of delta5-desaturase, or an increased activity of squalene epoxidase.

2. The method according to claim 1, **characterized by** that for increasing the activity of delta8-delta7-isomerase, the gene expression of a nucleic acid coding a delta8-delta7-isomerase, compared to the wild type, is increased.

3. The method according to claim 2, **characterized by** that for increasing the gene expression, one or several nucleic acids coding a delta8-delta7-isomerase, are introduced into the organism.

4. The method according to claim 3, **characterized by** that nucleic acids are introduce, which code proteins containing the amino acid sequence SEQ. ID. NO. 2 or a sequence derived from this sequence by substitution, insertion, or deletion of amino acids, which have an amino acid identity of at least 30% with the sequence SEQ. ID. NO. 2, and which have the enzymatic property of a delta8-delta7-isomerase.

5. The method according to claim 4, **characterized by** that a nucleic acid containing the sequence SEQ. ID. NO. 1 is introduced.

6. The method according to one of claims 1 to 5, **characterized by** that for increasing the activity of delta5-desaturase, the gene expression of a nucleic acid coding a delta5-desaturase, compared to the wild type, is increased.

7. The method according to claim 6, **characterized by** that for increasing the gene expression, one or several nucleic acids coding a delta5-desaturase, are introduced into the organism.

8. The method according to claim 7, **characterized by** that nucleic acids are introduced, which code proteins containing the amino acid sequence SEQ. ID. NO.4 or a sequence derived from this sequence by substitution, insertion, or deletion of amino acids, which have an amino acid identity of at least 30% with the sequence SEQ. ID. NO. 4, and which have the enzymatic property of a delta5-desaturase,

9. The method according to claim 8, **characterized by** that a nucleic acid containing the sequence SEQ. ID. NO. 3 is introduced.

10. The method according to one of claims 1 to 9, **characterized by** that for increasing the activity of delta24-reductase, the gene expression of a nucleic acid coding a delta24-reductase, compared to the wild type, is increased.

11. The method according to claim 10, **characterized by** that for increasing the gene expression, one or several nucleic acids coding a delta24-reductase are introduced into the organism.

12. The method according to claim 11, **characterized by** that nucleic acids are introduced, which code proteins containing the amino acid sequence SEQ. ID. NO. 6 or a sequence derived from this sequence by substitution, insertion, or deletion of amino acids, which have an amino acid identity of at least 30% with the sequence SEQ. ID. NO. 6, and which have the enzymatic property of a delta24-reductase.

13. The method according to claim 12, **characterized by** that a nucleic acid containing the sequence SEQ. ID. NO. 5 is introduced.

14. The method according to one of claims 1 to 13, **characterized by** that the organisms, compared to the wild type, in addition have a reduced activity of at least one of the activities selected from the group: activity of C24-methyltransferase and activity of delta22-desaturase.

15. The method according to claim 14, **characterized by** that the organisms, compared to the wild type, have a reduced activity of C24-methyltransferase and a reduced activity of delta22-desaturase.

16. The method according to claim 14 or 15, **characterized by** that for reducing the activity of C24-methyltransferase, the gene expression of a nucleic acid coding a C24-methyltransferase, compared to the wild type, is reduced.

17. The method according to claim 16, **characterized by** that an organism is used, which has no functional C24-methyltransferase gene.

18. The method according to one of claims 14 to 17, **characterized by** that for reducing the activity of delta22-desaturase, the gene expression of a nucleic acid coding a delta22-desaturase, compared to the wild type, is reduced.

19. The method according to claim 18, **characterized by** that an organism is used, which has no functional delta22-desaturase gene.

20. The method according to one of claims 1 to 19, **characterized by** that the organisms in addition, compared to the wild type, have an increased activity of at least one of the activities selected from the group: activity of lanosterol-C14-demethylase, activity of squalene synthase and activity of sterol acyltransferase.

21. The method according to claim 20, **characterized by** that the organism in addition, compared to the wild type, have an increased activity of lanosterol-C14-demethylase.

22. The method according to claim 20 or 21, **characterized by** that for increasing the activity of lanosterol-C14-demethylase, the gene expression of a nucleic acid coding a lanosterol-C14-demethylase, compared to the wild type, is increased.

23. The method according to claim 22, **characterized by** that for increasing the gene expression, one or several nucleic acids coding a lanosterol-C14-demethylase, are introduced into the organism.

24. The method according to claim 23, charactersized by that nucleic acids are introduced, which code proteins containing the amino acid sequence SEQ. ID. NO. 8 or a sequence derived from this sequence by substitution, insertion, or deletion of amino acids, which have an amino acid identity of at least 30% with the sequence SEQ. ID. NO. 8, and which have the enzymatic property of a lanosterol-C14-demethylase.

25. The method according to claim 24, **characterized by** that a nucleic acid containing the sequence SEQ. ID. NO. 7 is introduced.

26. The method according to one of claims 1 to 25, **characterized by** that for increasing the activity of HMG-CoA reductase, the gene expression of a nucleic acid coding a HMG-CoA reductase, compared to the wild type, is increased.

27. The method according to claim 26, **characterized by** that for increasing the gene expression, a nucleic acid construct containing a nucleic acid coding a HMG-CoA reductase is introduced into the organism, the expression of which in the organism, compared to the wild type, is subject to a reduced regulation.

28. The method according to claim 27, **characterized by** that the nucleic acid construct contains a promoter, which in the organism, compared to the wild type promoter, is subject to a reduced regulation.

29. The method according to claim 27 or 28, **characterized by** that as a nucleic acid coding a HMG-CoA reductase, a nucleic acid is used, the expression of which in the organism, compared to the organism's own, orthologous nucleic acid, is subject to a reduced regulation.

30. The method according to claim 29, **characterized by** that as a nucleic acid coding a HMG-CoA reductase, a nucleic acid is used, which codes the catalytic section of the HMG-CoA reductase.

31. The method according to claim 30, **characterized by** that nucleic acids are introduced, which code proteins containing the amino acid sequence SEQ. ID. NO. 10 or a sequence derived from this sequence by substitution, insertion, or deletion of amino acids, which have an amino acid identity of at least 30% with the sequence SEQ. ID. NO. 10, and which have the enzymatic property of a HMG-CoA reductase.

32. The method according to claim 31, **characterized by** that a nucleic acid containing the sequence SEQ. ID. NO. 9 is introduced.

33. The method according to one of claims 1 to 32, **characterized by** that an organism is used, which in addition, compared to the wild type, has an increased activity of squalene epoxidase.

34. The method according to claim 33, **characterized by** that for increasing the activity of squalene epoxidase, the gene expression of a nucleic acid coding a squalene epoxidase, compared to the wild type, is increased.

35. The method according to claim 34, **characterized by** that for increasing the gene expression, one or several nucleic acids coding a squalene epoxidase are introduced into the organism.

36. The method according to claim 35, **characterized by** that nucleic acids are introduced, which code proteins containing the amino acid sequence SEQ. ID. NO. 12 or a sequence derived from this sequence by substitution, insertion, or deletion of amino acids, which have an amino acid identity of at least 30% with the sequence SEQ. ID. NO. 12, and which have the enzymatic property of a squalene epoxidase.

37. The method according to claim 36, **characterized by** that a nucleic acid containing the sequence SEQ. ID. NO. 11 is introduced.

38. The method according to one of claims 1 to 38, **characterized by** that as an organism, yeast is used.

39. The method according to one of claims 1 to 38, **characterized by** that after culturing, the organism is harvested, and then 7-dehydrocholesterol is isolated from the organism.

40. A nucleic acid construct containing a) at least one nucleic acid coding a delta24-reductase being functionally linked with one or several regulation signals guaranteeing the transcription and translation in organisms, b) at least one nucleic acid coding a HMG-CoA reductase, and either c1) at least one nucleic acid coding a delta8-delta7-isomerase and a nucleic acid coding a delta5-desaturase, or c2) a nucleic acid coding a squalene epoxidase, the components b) and c1) or c2) being functionally linked with one or several regulation signals guaranteeing the transcription and translation in organisms.

41. The nucleic acid construct according to claim 40 containing in addition at least one nucleic acid selected from the group nucleic acids coding a lanostorol-C14-demethylase, nucleic acids coding a squalene epoxidase, and nucleic acids coding a sterol acyltransferase being functionally linked with one or several regulation signals guaranteeing the transcription and translation in organisms.

42. A combination of nucleic acid constructs, wherein the combination comprises at least one nucleic acid construct selected from the group A to C,
A nucleic acid construct containing nucleic acids coding a delta8-delta7-isomerase being functionally linked with one or several regulation signals guaranteeing the transcription and translation in organisms,
B nucleic acid construct containing nucleic acids coding a delta5-desaturase being functionally linked with one or several regulation signals guaranteeing the transcription and translation in organisms, and
C nucleic acid construct containing nucleic acids coding a delta24-reductase being functionally linked with one or several regulation signals guaranteeing the transcription and translation in organisms,
and at least one nucleic acid construct selected from the group D to H,
D nucleic acid construct containing nucleic acids coding a HMG-CoA reductase being functionally linked with one or several regulation signals guaranteeing the transcription and translation in organisms,
E nucleic acid construct containing nucleic acids coding a lanosterol-C14-demethylase being functionally linked with one or several regulation signals guaranteeing the transcription and translation in organisms,
F nucleic acid construct containing nucleic acids coding a squalene epoxidase being functionally linked with one or several regulation signals guaranteeing the transcription and translation in organisms,
G nucleic acid construct containing nucleic acids coding a squalene synthase being functionally linked with one or several regulation signals guaranteeing the transcription and translation in organisms,
H nucleic acid construct containing nucleic acids coding a sterol acyltransferase being functionally linked with one or several regulation signals guaranteeing the transcription and translation in organisms,
wherein the groups C and D and either A and B on the one hand or F on the other hand are mandatorily provided.

43. Nucleic acid constructs or a combination of nucleic acid constructs according to one of claims 40 to 42, **characterized by** that the regulation signals contain one or several promoters and one or several terminators guaranteeing the transcription and translation in organisms.

44. Nucleic acid constructs or a combination of nucleic acid constructs according to claim 43, **characterized by** that regulation signals are used that guarantee the transcription and translation in yeasts.

45. A genetically modified organism, wherein the genetic modification increases at least the activity of delta24-reductase and the activity of HMG-CoA-reductase, compared to the wild type, wherein the increase of the activity of delta24-reductase is effected by an increase of the gene expression of at least one nucleic acid coding a delta24-reductase, compared to the wild type, wherein the genetic modification in addition either increases the activity of squalene epoxidase, or wherein the organism contains two or more nucleic acids coding a delta8-delta7-isomerase and/or two or more nucleic acids coding a delta5-desaturase.

46. The genetically modified organism according to claim 45, **characterized by** that the organism contains two or more nucleic acids coding a delta24-reductase.

47. The genetically modified organism according to one of claims 45 to 46, **characterized by** that the genetic modification in addition reduces at least one of the activities selected from the group: activity of C24-methyltransferase and activity of delta22-desaturase, compared to the wild type.

48. The genetically modified organism according to claim 47, **characterized by** that the reduction of at least one of the activities is effected by a reduction of the gene expression of at least one nucleic acid selected from the group nucleic acids coding a C24-methyltransferase and nucleic acids coding a delta22-desaturase, compared to the wild type.

49. The genetically modified organism according to claim 48, **characterized by** that the organism has no functional C24-methyltransferase gene and/or delta22-desaturase gene.

50. The genetically modified organism according to one of claims 45 to 49, **characterized by** that the genetic modification in addition increases at least one of the activities selected from the group: activity of lanosterol-C14-demethylase, activity of squalene synthase and activity of sterol acyltransferase, compared to the wild type.

51. The genetically modified organism according to claim 50, **characterised by** that the increase of at least one of the activities is effected by an increase of the gene expression of at least one nucleic acid selected from the group nucleic acids coding a lanosterol-C14-demethylase, nucleic acids coding a squalene synthase and nucleic acids coding a sterol acyltransferase, compared to the wild type.

52. The genetically modified organism according to claim 51, **characterized by** that the organism contains two or more nucleic acids coding a HMG-CoA reductase and/or two or more nucleic acids coding a lanosterol-C14-demethylase and/or two or more nucleic acids coding a squalene epoxidase and/or two or more nucleic acids coding a squalene synthase and/or two or more nucleic acids coding a sterol acyltransferase.

53. The genetically modified organism according to one of claims 45 to 52, **characterized by** that the genetically modified organism, compared to the wild type, has an increased content of 7-dehydrocholesterol.

54. The genetically modified organism according to one of claims 45 to 53, **characterized by** that as an organism, yeast is used.

55. Use of a genetically modified organism according to one of claims 45 to 54 for preparing 7-dehydrocholesterol.

## Revendications

1. Procédé de production de 7-déhydrocholestérol par mise en culture d'organismes, qui présentent une activité accrue de la delta24-réductase et une activité accrue de la HMG-CoA-réductase, et ou bien une activité accrue de la delta8-delta7-isomerase et une activité accrue de la delta5-désaturase, ou bien une activité accrue de la squaléne époxidase par rapport au type sauvage.

2. Procédé selon la revendication 1, **caractérisé en ce que** pour accroître l'activité de la delta8-delta7-isomérase, l'expression génétique d'un acide nucléique codant une delta8-delta7-isomérase est accrue par rapport au type sauvage.

3. Procédé selon la revendication 2, **caractérisé en ce que** pour accroître l'expression génétique, un ou plusieurs acides nucléiques, codant une delta8-delta7-isomérase sont introduits dans l'organisme.

4. Procédé selon la revendication 3, **caractérisé en ce que** des acides nucléiques sont introduits, qui codent des protéines contenant la séquence d'acides aminés SEQ. ID. N° 2 ou une séquence dérivée à partir de cette séquence par substitution, insertion, ou délétion d'acides aminés, qui ont une identité en acides aminés d'au moins 30% avec la séquence SEQ. ID. N° 2, et qui ont la propriété enzymatique d'une delta8-delta7-isomérase.

5. Procédé selon la revendication 4, **caractérisé en ce qu'**un acide nucléique contenant la séquence SEQ. ID. N° 1 est introduit.

6. Procédé selon une des revendications 1 à 5, **caractérisé en ce que** pour accroître l'activité de la delta5-désaturase, l'expression génétique d'un acide nucléique codant une delta5-désaturase est accrue par rapport au type sauvage.

7. Procédé selon la revendication 6, **caractérisé en ce que** pour accroître l'expression génétique, un ou plusieurs acides nucléiques codant une delta5-désaturase sont introduits dans l'organisme.

8. Procédé selon la revendication 7, **caractérisé en ce que** des acides nucléiques sont introduits, qui codent des protéines contenant la séquence d'acides aminés SEQ. ID. N° 4 ou une séquence dérivée à partir de cette séquence par substitution, insertion, ou délétion d'acides aminés, qui ont une identité en acides aminés d'au moins 30% avec la séquence SEQ. ID. N° 4, et qui ont la propriété enzymatique d'une delta5-désaturase.

9. Procédé selon la revendication 8, **caractérisé en ce qu'**un acide nucléique contenant la séquence SEQ. ID. N° 3 est introduit.

10. Procédé selon une des revendications 1 à 9, **caractérisé en ce que** pour accroître l'activité de la delta24-réductase, l'expression génétique d'un acide nucléique codant une delta24-réductase est accrue par rapport au type sauvage.

11. Procédé selon la revendication 10, **caractérisé en ce que** pour accroître l'expression génétique, un ou plusieurs acides nucléiques codant une delta24-réductase sont introduits dans l'organisme.

12. Procédé selon la revendication 11, **caractérisé en ce que** des acides nucléiques sont introduits, qui codent des protéines contenant la séquence d'acides aminés SEQ. ID. N° 6 ou une séquence dérivée à partir de cette séquence par substitution, insertion, ou délétion d'acides aminés, qui ont une identité en acides aminés d'au moins 30% avec la séquence SEQ. ID. N° 6, et qui ont la propriété enzymatique d'une delta24-réductase.

13. Procédé selon la revendication 12, **caractérisé en ce qu'**un acide nucléique contenant la séquence SEQ. ID. N° 5 est introduit.

14. Procédé selon une des revendications 1 à 13, **caractérisé en ce que** les organismes en plus ont une activité réduite, par rapport au type sauvage, d'au moins une des activités, sélectionnée à partir du groupe: activité de C24-méthyltransférase et activité de delta22-désaturase.

15. Procédé selon la revendication 14, **caractérisé en ce que** les organismes ont une activité réduite de la C24-méthyltransférase et une activité réduite de la delta22-désaturase par rapport au type sauvage.

16. Procédé selon la revendication 14 ou 15, **caractérisé en ce que** pour réduire l'activité de la C24-méthyltransférase, l'expression génétique d'un acide nucléique codant une C24-méthyltransférase est réduite par rapport au type sauvage.

17. Procédé selon la revendication 16, **caractérisé en ce qu'**un organisme est utilisé, qui n'a pas de gène fonctionnel de la C24-méthyltransférase.

18. Procédé selon une des revendications 14 à 17, **caractérisé en ce que** pour réduire l'activité de la delta22-désaturase, l'expression génétique d'un acide nucléique codant une delta22-désaturase est réduite par rapport au type sauvage.

19. Procédé selon la revendication 18, **caractérisé en ce qu'**un organisme est utilisé, qui n'a pas de gène fonctionnel de la delta22-désaturase.

20. Procédé selon une des revendications 1 à 19, **caractérisé en ce que** les organismes en plus présentent une activités accrue, par rapport au type sauvage, d'au moins une des activités, sélectionnée à partir du groupe: activité de la lanostérol-C14-déméthylase, activité de la squalène synthétase et activité de la stérol acyltransférase.

21. Procédé selon la revendication 20, **caractérisé en ce que** les organismes en plus présentent une activité accrue, par rapport au type sauvage, de la lanostérol-C14-déméthylase.

22. Procédé selon la revendication 20 ou 21, **caractérisé en ce que** pour accroître l'activité de la lanostérol-C14-déméthylase, l'expression génétique d'un acide nucléique codant une lanostérol-C14-déméthylase est accrue par rapport au type sauvage.

23. Procédé selon la revendication 22, **caractérisé en ce que** pour accroître l'expression génétique, une ou plusieurs acides nucléiques codant une lanostérol-C14-déméthylase sont introduits dans l'organisme.

24. Procédé selon la revendication 23, **caractérisé en ce que** des acides nucléiques sont introduits, qui codent des protéines contenant la séquence d'acides aminés SEQ. ID. N° 8 ou une séquence dérivée à partir de cette séquence par substitution, insert.i,on, ou délétion d'acides aminés, qui ont une identité en acides aminés d'au moins 30% avec la séquence SEQ. ID. N° 8, et qui ont la propriété enzymatique d'une lanostérol-C14-déméthylase.

25. Procédé selon la revendication 24, **caractérisé en ce qu'**un acide nucléique contenant la séquence SEQ. ID. N° 7 est introduit.

26. Procédé selon une des revendications 1 à 25, **caractérisé en ce que** pour accroître l'activité de la HMG-CoA réductase, l'expression génétique d'un acide nucléique codant une HMG-CoA réductase est accrue par rapport au type sauvage.

27. Procédé selon la revendication 26, **caractérisé en ce que** pour accroître l'expression génétique, une construction d'acides nucléiques contenant un acide nucléique codant une HMG-CoA réductase est introduite dans l'organisme, l'expression duquel dans l'organisme est soumise à une régulation réduite par rapport au type sauvage.

28. Procédé selon la revendication 27, **caractérisé en ce que** la construction d'acides nucléiques contient un promoteur, qui dans l'organisme est soumis à une régulation réduite par rapport au promoteur du type sauvage.

29. Procédé selon la revendication 27 ou 28, **caractérisé en ce que** comme acide nucléique codant une HMG-CoA réductase, un acide nucléique est utilisé, l'expression duquel dans l'organisme est soumise à une régulation réduite par rapport à l'acide nucléique orthologue propre de l'organisme.

30. Procédé selon la revendication 29, **caractérisé en ce que** comme acide nucléique codant une HMG-CoA réductase, un acide nucléique est utilisé, qui code la partie catalytique de la HMG-CoA réductase.

31. Procédé selon la revendication 30, **caractérisé en ce que** des acides nucléiques sont introduits, qui codent des protéines contenant la séquence d'acides aminés SEQ. ID. N° 10 ou une séquence dérivée à partir de cette séquence par substitution, insertion, ou délétion d'acides aminés, qui ont une identité en acides aminés d'au moins 30% avec la séquence SEQ. ID. N° 10, et qui ont la propriété enzymatique d'une HMG-CoA réductase.

32. Procédé selon la revendication 31, **caractérisé en ce qu'**un acide nucléique contenant la séquence SEQ. ID. N° 9 est introduit.

33. Procédé selon une des revendications 1 à 32, **caractérisé en ce qu'**un organisme est utilisé, qui en plus a une activité accrue, par rapport au type sauvage, de la squalène époxidase.

34. Procédé selon la revendication 33, **caractérisé en ce que** pour accroître l'activité de la squalène epoxidase, l'expression génétique d'un acide nucléique codant une squalène époxidase est accrue par rapport au type sauvage.

35. Procédé selon la revendication 34, **caractérisé en ce que** pour accroître l'expression génétique, un ou plusieurs acides nucléiques codant une squalène époxidase sont introduits dans l'organisme.

36. Procédé selon la revendication 35, **caractérisé en ce que** des acides nucléiques sont introduits, qui codent des protéines contenant la séquence d'acides aminés SEQ. ID. N° 12 ou une séquence dérivée à partir de cette séquence par substitution, insertion, ou délétion d'acides aminés, qui ont une identité en acides aminés d'au moins 30% avec la séquence SEQ. ID. N° 12, et qui ont la propriété enzymatique d'une squalène époxidase.

37. Procédé selon la revendication 36, **caractérisé en ce qu'**un acide nucléique contenant la séquence SEQ. ID. N° 11 est introduit.

38. Procédé selon une des revendications 1 à 38, **caractérisé en ce que** comme organisme, la levure est utilisée.

39. Procédé selon une des revendications 1 à 38, **caractérisé en ce qu'**après la mise en culture, l'organisme est récolté, et puis le 7-déhydrocholestérol est isolé de l'organisme.

40. Construction d'acides nucléiques, contenant a) au moins un acide nucléique codant une delta24-réductase et étant fonctionnellement lié avec un ou plusieurs signaux de régulation, qui garantissent la transcription et translation dans des organismes, b) au moins un acide nucléique codant une HMG-CoA réductase, et c1) au moins un acide nucléique codant une delta8-delta7-isomérase et un acide nucléique codant une delta5-désaturase, ou bien c2) un acide nucléique codant une squalène epoxidase, les composants b) et c1) ou c2) étant fonctionnellement liés avec un ou plusieurs signaux de régulation, qui garantissent la transcription et translation dans des organismes.

41. Construction d'acides nucléiques selon la revendication 40, contenant en plus au moins un acide nucléique, sélectionné à partir du groupe des acides nucléiques codant une lanostérol-C14-déméthylase, acide nucléiques codant une squalène époxidase et acides nucléique codant une stérol acyltransférase et étant fonctionnellement liés avec un ou plusieurs signaux de régulation, qui garantissent la transcription et translation dans des organismes.

42. Combinaison de constructions d'acides nucléiques, dans laquelle la combinaison comprend au moins une construction d'acides nucléiques, sélectionnée à partir du groupe A à C:
A construction d'acides nucléiques, contenant des acides nucléiques codant une delta8-delta7-isomérase et étant fonctionnellement liés avec un ou plusieurs signaux de régulation, qui garantissent la transcription et translation dans des organismes,
B construction d'acides nucléiques, contenant des acides nucléiques codant une delta5-désaturase et étant fonctionnellement liés avec un ou plusieurs signaux de régulation, qui garantissent la transcription et translation dans des organismes et
C construction d'acides nucléiques, contenant des acides nucléiques codant une delta24-réductase et étant fonctionnellement liés avec un ou plusieurs signaux de régulation, qui garantissent la transcription et translation dans des organismes,
et au moins une construction d'acides nucléiques, sélectionnée à partir du groupe D à H:
D construction d'acides nucléiques, contenant des acides nucléiques codant une HMG-CoA réductase et étant fonctionnellement liés avec un ou plusieurs signaux de régulation, qui garantissent la transcription et translation dans des organismes,
E construction d'acides nucléiques, contenant des acides nucléiques codant une lanostérol-C14-déméthylase et étant fonctionnellement liés avec un ou plusieurs signaux de régulation, qui garantissent la transcription et translation dans des organismes,
F construction d'acides nucléiques, contenant des acides nucléiques codant une squalène époxidase et étant fonctionnellement liés avec un ou plusieurs signaux de régulation, qui garantissent la transcription et translation dans des organismes,
G construction d'acides nucléiques, contenant des acides nucléiques codant une squalène synthétase et étant fonctionnellement liés avec un ou plusieurs signaux de régulation, qui garantissent la transcription et translation dans des organismes,
H construction d'acides nucléiques, contenant des acides nucléiques codant une stérol acyltransferase et étant fonctionnellement liés avec un ou plusieurs signaux de régulation, qui garantissent la transcription et translation dans des organismes,
dans laquelle les groupes C et D et ou bien A et B à l'un côté ou bien F à l'autre côté sont impérativement prévus.

43. Constructions d'acides nucléiques ou combinaison de constructions d'acides nucléiques selon une des revendications 40 à 42, **caractérisées en ce que** les signaux de régulation contiennent un ou plusieurs promoteurs et un ou plusieurs terminateurs, qui garantissent la transcription et translation dans des organismes.

44. Constructions d'acides nucléiques ou combinaison de constructions d'acides nucléiques selon la revendication 43, **caractérisées en ce que** des signaux de régulation sont utilisés, qui garantissent la transcription et translation dans les levures.

45. Organisme génétiquement modifié, dans lequel la modification génétique accroît au moins l'activité de la delta24-réductase et l'activité de la HMG-CoA réductase par rapport au type sauvage, dans lequel l'accroissement de l'activité de la delta24-réductase est effectué par accroissement de l'expression génétique d'au moins un acide nucléique codant une delta24-réductase par rapport au type sauvage, dans lequel la modification génétique en plus accroît l'activité de la squalène époxidase, ou bien dans lequel l'organisme contient deux ou plus d'acides nucléiques codant une delta8-delta7-isomérase et/ou deux ou plus d'acides nucléiques codant une delta5-désaturase.

46. Organisme génétiquement modifié selon la revendication 45, **caractérisé en ce que** l'organisme contient deux ou plus d'acides nucléiques codant une delta24-réductase.

47. Organisme génétiquement modifié selon une des revendications 45 à 46, **caractérisé en ce que** la modification génétique en plus réduit au moins une des activités, sélectionnée à partir du groupe: activité de C24-méthyltransférase et activité de delta22-désaturase, par rapport au type sauvage.

48. Organisme génétiquement modifié selon la revendication 47, **caractérisé en ce que** la réduction d'au moins une des activités est effectuée par une réduction de l'expression génétique d'au moins un acide nucléique, sélectionné à partir du groupe des acides nucléiques codant une C24-méthyltransférase et acides nucléiques codant une delta22-désaturase, par rapport au type sauvage.

49. Organise génétiquement modifié selon la revendication 48, **caractérisé en ce que** l'organisme ne contient pas de gène fonctionnel de la C24-méthyltransférase et/ou de la delta22-désaturase.

50. Organisme génétiquement modifié selon une des revendications 45 à 49, **caractérisé en ce que** la modification génétique en plus accroît au moins une des activités, sélectionnée à partir du groupe: activité de la lanostérol-C14-déméthylase, activité de la squalène synthétase et activité de la stérol acyltransférase, par rapport au type sauvage.

51. Organisme génétiquement modifié selon la revendication 50, **caractérisé en ce que** l'accroissement d'au moins une des activités est effectué par accroissement de l'expression génétique d'au moins un acide nucléiques, sélectionné à partir du groupe des acides nucléique codant une lanostérol-C14-déméthylase, acides nucléiques codant une squalène synthétase est acides nucléiques codant une stérol acyltransférase, par rapport au type sauvage.

52. Organisme génétiquement modifié selon la revendication 51, **caractérisé en ce que** l'organisme contient deux ou plus d'acides nucléiques codant une HMG-CoA réductase et/ou deux ou plus d'acides nucléiques codant une lanostérol-C14-déméthylase et/ou deux ou plus d'acides nucléiques codant une squalène époxidase et/ou deux ou plus d'acides nucléiques codant une squalène synthétase et/ou deux ou plus d'acides nucléiques codant une stérol acyltransférase.

53. Organisme génétiquement modifié selon une des revendications 45 à 52, **caractérisé en ce que** l'organisme génétiquement modifié a un contenu accru de 7-déhydrocholestérol par rapport au type sauvage.

54. Organisme génétiquement modifié selon une des revendications 45 à 53, **caractérisé en ce que** comme organisme, la levure est utilisée.

55. Utilisation d'un organisme génétiquement modifié selon une des revendications 45 à 54 pour la production de 7-déhydrocholestérol.


### SEQUENZPROTOKOLL

<110> BASF Aktiengesellschaft
<120> Verfahren zur Herstellung von 7-Dehydrocholesterol und/oder dessen biosynthetischen Zwischen- und/oder Folgeprodukten in transgenen Organismen
<130> 0050
<140>
   <141>
<160> 60
<170> PatentIn Vers. 2.0
<210> 1
   <211> 693
   <212> DNA
   <213> Mus musculus
<220>
   <221> CDS
   <222> (1)..(693)
<400> 1
   atg acc acc aat acg gtc ccc ttg cac ccg tac tgg ccc agg cac ctg    48
   Met Thr Thr Asn Thr Val Pro Leu His Pro Tyr Trp Pro Arg His Leu
     1               5                  10                  15
   aag ctg gac aac ttc gtg cct aat gac ctc ccg act tcg cat atc ctg    96
   Lys Leu Asp Asn Phe Val Pro Asn Asp Leu Pro Thr Ser His Ile Leu
                20                  25                  30
   gtt ggc ctc ttc tcc atc tct ggg ggc cta att gtg atc acg tgg ctg   144
   Val Gly Leu Phe Ser Ile Ser Gly Gly Leu Ile Val Ile Thr Trp Leu
            35                  40                  45
   ttg tct agc cga gct tcc gtc gtc cca ctt gga gct ggg cgg cga ctg   192
   Leu Ser Ser Arg Ala Ser Val Val Pro Leu Gly Ala Gly Arg Arg Leu
        50                  55                  60
   gcc ttg tgc tgg ttt gct gtg tgt acc ttc att cac ctt gtg atc gag   240
   Ala Leu Cys Trp Phe Ala Val Cys Thr Phe Ile His Leu Val Ile Glu
    65                  70                  75                  80
   ggc tgg ttc tct ctc tac aat ggc atc ctt tta gaa gac caa gcc ttc   288
   Gly Trp Phe Ser Leu Tyr Asn Gly Ile Leu Leu Glu Asp Gln Ala Phe
                    85                  90                  95
   tta tcc caa ctc tgg aaa gag tat tcc aag gga gat agc cga tat atc   336
   Leu Ser Gln Leu Trp Lys Glu Tyr Ser Lys Gly Asp Ser Arg Tyr Ile
               100                 105                 110
   ctt agt gac agc ttc gtc gtc tgt atg gag act gtc aca gct tgt ctc   384
   Leu Ser Asp Ser Phe Val Val Cys Met Glu Thr Val Thr Ala Cys Leu
   115 120 125
   tgg gga cca ctc agc cta tgg gta gtg att gcc ttt ctc cgc caa cag 432
   Trp Gly Pro Leu Ser Leu Trp Val Val Ile Ala Phe Leu Arg Gln Gln
   130 135 140
   ccc ttc cgc ttt gtc cta cag ctt gtg gtg tct atg ggc cag ata tac 480
   Pro Phe Arg Phe Val Leu Gln Leu Val Val Ser Met Gly Gln Ile Tyr
   145 150 155 160
   ggg gat gtg ctg tac ttc ctg aca gag cta cac gaa gga ctc cag cat 528
   Gly Asp Val Leu Tyr Phe Leu Thr Glu Leu His Glu Gly Leu Gln His
   165 170 175
   ggg gag ata ggc cac ccc gtt tat ttc tgg ttc tat ttt gtt ttc ctg 576
   Gly Glu Ile Gly His Pro Val Tyr Phe Trp Phe Tyr Phe Val Phe Leu
   180 185 190
   aat gct gta tgg ttg gtg ata cca agc atc ctt gtg ctt gat gcc ata 624
   Asn Ala Val Trp Leu Val Ile Pro Ser Ile Leu Val Leu Asp Ala Ile
   195 200 205
   aag cat ctc act agt gcc cag agc gtg ctg gac agc aaa gtc atg aaa 672
   Lys His Leu Thr Ser Ala Gln Ser Val Leu Asp Ser Lys Val Met Lys
   210 215 220
   att aag agc aag cat aac taa 693
   Ile Lys Ser Lys His Asn
   225 230
<210> 2
   <211> 230
   <212> PRT
   <213> Mus musculus
<400> 2
   Met Thr Thr Asn Thr Val Pro Leu His Pro Tyr Trp Pro Arg His Leu
   1               5                   10                  15
   Lys Leu Asp Asn Phe Val Pro Asn Asp Leu Pro Thr Ser His Ile Leu
               20                  25                  30
   Val Gly Leu Phe Ser Ile Ser Gly Gly Leu Ile Val Ile Thr Trp Leu
           35                  40                  45
   Leu Ser Ser Arg Ala Ser Val Val Pro Leu Gly Ala Gly Arg Arg Leu
       50                  55                  60
   Ala Leu Cys Trp Phe Ala Val Cys Thr Phe Ile His Leu Val Ile Glu
   65                  70                  75                  80
   Gly Trp Phe Ser Leu Tyr Asn Gly Ile Leu Leu Glu Asp Gln Ala Phe
                   85                  90                  95
   Leu Ser Gln Leu Trp Lys Glu Tyr Ser Lys Gly Asp Ser Arg Tyr Ile
               100                 105                 110
   Leu Ser Asp Ser Phe Val Val Cys Met Glu Thr Val Thr Ala Cys Leu
           115                 120                 125
   Trp Gly Pro Leu Ser Leu Trp Val Val Ile Ala Phe Leu Arg Gln Gln
       130                 135                 140
   Pro Phe Arg Phe Val Leu Gln Leu Val Val Ser Met Gly Gln Ile Tyr
   145                 150                 155                 160
   Gly Asp Val Leu Tyr Phe Leu Thr Glu Leu His Glu Gly Leu Gln His
                   165                 170                 175
   Gly Glu Ile Gly His Pro Val Tyr Phe Trp Phe Tyr Phe Val Phe Leu
               180                 185                 190
   Asn Ala Val Trp Leu Val Ile Pro Ser Ile Leu Val Leu Asp Ala Ile
           195                 200                 205
   Lys His Leu Thr Ser Ala Gln Ser Val Leu Asp Ser Lys Val Met Lys
       210                 215                 220
   Ile Lys Ser Lys His Asn
   225                 230
<210> 3
   <211> 693
   <212> DNA
   <213> Homo sapiens
<220>
   <221> CDS
   <222> (1)..(693)
<400> 3
   atg act acc aac gcg ggc ccc ttg cac cca tac tgg cct cag cac cta     48
   aga ctg gac aac ttt gta cct aat gac cgc ccc acc tgg cat ata ctg     96
   gct ggc ctc ttc tct gtc aca ggg gtc tta gtc gtg acc aca tgg ctg    144
   ttg tca ggt cgt gct gcg gtt gtc cca ttg ggg act tgg cgg cga ctg    192
   tcc ctg tgc tgg ttt gca gtg tgt ggg ttc att cac ctg gtg atc gag    240
   ggc tgg ttc gtt ctc tac tac gaa gac ctg ctt gga gac caa gcc ttc    288
   tta tct caa ctc tgg aaa gag tat gcc aag gga gac agc cga tac atc     336
   ctg ggt gac aac ttc aca gtg tgc atg gaa acc atc aca gct tgc ctg     384
   tgg gga cca ctc agc ctg tgg gtg gtg atc gcc ttt ctc cgc cag cat     432
   ccc ctc cgc ttc att cta cag ctt gtg gtc tct gtg ggc cag atc tat     480
   ggg gat gtg ctc tac ttc ctg aca gag cac cgc gac gga ttc cag cac     528
   gga gag ctg ggc cac cct ctc tac ttc tgg ttt tac ttt gtc ttc atg     576
   aat gcc ctg tgg ctg gtg ctg cct gga gtc ctt gtg ctt gat gct gtg     624
   aag cac ctc act cat gcc cag agc acg ctg gat gcc aag gcc aca aaa     672
   gcc aag agc aag aag aac tga                                         693
<210> 4
   <211> 230
   <212> PRT
   <213> Homo sapiens
<400> 4
<210> 5
   <211> 669
   <212> DNA
   <213> Saccharomyces cerevisiae
<220>
   <221> CDS
   <222> (1)..(669)
<400> 5
   atg aag ttt ttc cca ctc ctt ttg ttg att ggt gtt gta ggc tac att    48
   atg aac gta ttg ttc act acc tgg ttg cca acc aat tac atg ttc gat    96
   cca aaa act ttg aac gaa ata tgt aac tcg gtg att agc aaa cac aac   144
   gca gca gaa ggt tta tcc act gaa gac ctg tta cag gat gtc aga gac   192
   gca ctt gcc tct cat tac ggg gac gaa tac atc aac agg tac gtc aaa   240
   gaa gaa tgg gtc ttc aac aat gct ggt ggt gcg atg ggc caa atg atc   288
   atc cta cac gct tcc gta tcc gag tac tta att cta ttc gga acc gct   336
   gtt ggt act gaa ggg cac aca ggt gtt cac ttt gct gac gac tat ttt   384
   acc atc tta cat ggt acg caa atc gca gca ttg cca tat gcc act gaa   432
   gcc gaa gtt tac act cct ggt atg act cat cac ttg aag aag gga tac   480
   gcc aag caa tac agc atg cca ggt ggt tcc ttt gcc ctt gaa ttg gct   528
   caa ggc tgg att cca tgt atg ttg cca ttc ggg ttt ttg gac act ttc   576
   tcc agt act ctt gat tta tac act cta tat aga act gtc tac ctg act   624
   gcc agg gac atg ggt aag aac ttg ttg caa aac aaa aag ttc taa       669
<210> 6
   <211> 222
   <212> PRT
   <213> Saccharomyces cerevisiae
<400> 6
<210> 7
   <211> 900
   <212> DNA
   <213> Mus musculus
<220>
   <221> CDS
   <222> (1)..(900)
<400> 7
   atg gac ctg gtt ctc agt gcc gcc gat tac tac ttc ttc act ccg tat    48
   Met Asp Leu Val Leu Ser Ala Ala Asp Tyr Tyr Phe Phe Thr Pro Tyr
    1               5                  10                  15
   gta tat cca gcc acg tgg ccc gag gac aac atc atc cga caa act att    96
   Val Tyr Pro Ala Thr Trp Pro Glu Asp Asn Ile Ile Arg Gln Thr Ile
               20                  25                  30
   agc ctc ctg att gtc aca aac ctg ggt gct tac att ctc tac ttc ttc    144
   Ser Leu Leu Ile Val Thr Asn Leu Gly Ala Tyr Ile Leu Tyr Phe Phe
               35                  40                  45
   tgt gca acc ctc agc tat tat ttt gtc tat gat cat tcc tta atg aaa    192
   Cys Ala Thr Leu Ser Tyr Tyr Phe Val Tyr Asp His Ser Leu Met Lys
        50                  55                  60
   cac cca cag ttt tta aag aac caa gtc tcg cgt gag atc gtg ttc act    240
   His Pro Gln Phe Leu Lys Asn Gln Val Ser Arg Glu Ile Val Phe Thr
    65                  70                  75                  80
   gtc aag tct ttg cct tgg atc agc atc ccc acc gtc tca cta ttc ctg    288
   Val Lys Ser Leu Pro Trp Ile Ser Ile Pro Thr Val Ser Leu Phe Leu
                    85                  90                  95
   ctg gag ctg agg ggt tac agc aaa ctc tac gat gac atc gga gac ttt    336
   Leu Glu Leu Arg Gly Tyr Ser Lys Leu Tyr Asp Asp Ile Gly Asp Phe
               100                 105                 110
   cca aat ggc tgg att cat ctc atg gtt agc gtc gta tcc ttc ctc ttt    384
   Pro Asn Gly Trp Ile His Leu Met Val Ser Val Val Ser Phe Leu Phe
           115                 120                 125
   ttc aca gac atg ttg atc tac agg att cat agg ggc ctg cac cac aga    432
   Phe Thr Asp Met Leu Ile Tyr Arg Ile His Arg Gly Leu His His Arg
       130                 135                 140
   ctg gtc tac aag cgc ata cat aaa cca cat cat att tgg aag atc ccc    480
   Leu Val Tyr Lys Arg Ile His Lys Pro His His Ile Trp Lys Ile Pro
   145                 150                 155                 160
   acg ccg ttt gca agt cat gct ttt cac cct gtg gac ggc ttc ctt cag    528
   Thr Pro Phe Ala Ser His Ala Phe His Pro Val Asp Gly Phe Leu Gln
                   165                 170                 175
   agt ctg cct tac cat ata tac ccc ttt gtc ttt cca ctg cac aag gtg    576
   Ser Leu Pro Tyr His Ile Tyr Pro Phe Val Phe Pro Leu His Lys Val
               180                 185                 190
   gtc tac tta ggt tta tat gtc ttg gtt aat gtc tgg aca att tct att    624
   Val Tyr Leu Gly Leu Tyr Val Leu Val Asn Val Trp Thr Ile Ser Ile
           195                 200                 205
   cat gat ggt gat ttt cgg gtt ccc cag atc tta agg cca ttt att aac    672
   His Asp Gly Asp Phe Arg Val Pro Gln Ile Leu Arg Pro Phe Ile Asn
       210                 215                 220
   ggg tca gct cac cac aca gac cac cac atg ttc ttt gac tat aac tat    720
   Gly Ser Ala His His Thr Asp His His Met Phe Phe Asp Tyr Asn Tyr
   225                 230                 235                 240
   gga cag tat ttc aca ttg tgg gat aga att gga ggc tct ttt aaa cat    768
   Gly Gln Tyr Phe Thr Leu Trp Asp Arg Ile Gly Gly Ser Phe Lys His
                   245                 250                 255
   cct tcc tct ttt gaa ggg aaa gga cca cat agt tac gtg aag aac atg    816
   Pro Ser Ser Phe Glu Gly Lys Gly Pro His Ser Tyr Val Lys Asn Met
               260                 265                 270
   aca gaa aaa gaa tct aac agc ttt gca gaa aac ggc tgt aaa ggc aaa    864
   Thr Glu Lys Glu Ser Asn Ser Phe Ala Glu Asn Gly Cys Lys Gly Lys
           275                 280                 285
   aaa gta agc aat gga gag ttt aca aag aat aag tag                    900
   Lys Val Ser Asn Gly Glu Phe Thr Lys Asn Lys
       290                 295                 300
<210> 8
   <211> 299
   <212> PRT
   <213> Mus musculus
<400> 8
   Met Asp Leu Val Leu Ser Ala Ala Asp Tyr Tyr Phe Phe Thr Pro Tyr
   1 5 10 15
   Val Tyr Pro Ala Thr Trp Pro Glu Asp Asn Ile Ile Arg Gln Thr Ile
   20 25 30
   Ser Leu Leu Ile Val Thr Asn Leu Gly Ala Tyr Ile Leu Tyr Phe Phe
   35 40 45
   Cys Ala Thr Leu Ser Tyr Tyr Phe Val Tyr Asp His Ser Leu Met Lys
   50 55 60
   His Pro Gln Phe Leu Lys Asn Gln Val Ser Arg Glu Ile Val Phe Thr
   65 70 75 80
   Val Lys Ser Leu Pro Trp Ile Ser Ile Pro Thr Val Ser Leu Phe Leu
   85 90 95
   Leu Glu Leu Arg Gly Tyr Ser Lys Leu Tyr Asp Asp Ile Gly Asp Phe
   100 105 110
   Pro Asn Gly Trp Ile His Leu Met Val Ser Val Val Ser Phe Leu Phe
   115 120 125
   Phe Thr Asp Met Leu Ile Tyr Arg Ile His Arg Gly Leu His His Arg
   130 135 140
   Leu Val Tyr Lys Arg Ile His Lys Pro His His Ile Trp Lys Ile Pro
   145 150 155 160
   Thr Pro Phe Ala Ser His Ala Phe His Pro Val Asp Gly Phe Leu Gln
   165 170 175
   Ser Leu Pro Tyr His Ile Tyr Pro Phe Val Phe Pro Leu His Lys Val
   180 185 190
   Val Tyr Leu Gly Leu Tyr Val Leu Val Asn Val Trp Thr Ile Ser Ile
   195 200 205
   His Asp Gly Asp Phe Arg Val Pro Gln Ile Leu Arg Pro Phe Ile Asn
   210 215 220
   Gly Ser Ala His His Thr Asp His His Met Phe Phe Asp Tyr Asn Tyr
   225 230 235 240
   Gly Gln Tyr Phe Thr Leu Trp Asp Arg Ile Gly Gly Ser Phe Lys His
   245 250 255
   Pro Ser Ser Phe Glu Gly Lys Gly Pro His Ser Tyr Val Lys Asn Met
   260 265 270
   Thr Glu Lys Glu Ser Asn Ser Phe Ala Glu Asn Gly Cys Lys Gly Lys
   275 280 285
   Lys Val Ser Asn Gly Glu Phe Thr Lys Asn Lys
   290 295
<210> 9
   <211> 900
   <212> DNA
   <213> Homo sapiens
<220>
   <221> CDS
   <222> (1)..(900)
<400> 9
   atg gat ctt gta ctc cgt gtt gca gat tac tat ttt ttt aca cca tac      48
   Met Asp Leu Val Leu Arg Val Ala Asp Tyr Tyr Phe Phe Thr Pro Tyr
    1               5                  10                  15
   gtg tat cca gcc aca tgg cca gaa gat gac atc ttc cga caa gct att      96
   Val Tyr Pro Ala Thr Trp Pro Glu Asp Asp Ile Phe Arg Gln Ala Ile
               20                  25                  30
   agt ctt ctg att gta aca aat gtt ggt gct tac atc ctt tat ttc ttc     144
   Ser Leu Leu Ile Val Thr Asn Val Gly Ala Tyr Ile Leu Tyr Phe Phe
           35                  40                  45
   tgt gca aca ctg agc tat tat ttt gtc ttc gat cat gca tta atg aaa     192
   Cys Ala Thr Leu Ser Tyr Tyr Phe Val Phe Asp His Ala Leu Met Lys
       50                  55                  60
   cat cca caa ttt tta aag aat caa gtc cgt cga gag att aag ttt act     240
   His Pro Gln Phe Leu Lys Asn Gln Val Arg Arg Glu Ile Lys Phe Thr
   65                  70                  75                  80
   gtc cag gca ttg cca tgg ata agt att ctt act gtt gca ctg ttc ttg     288
   Val Gln Ala Leu Pro Trp Ile Ser Ile Leu Thr Val Ala Leu Phe Leu
                   85                  90                  95
   ctg gag ata aga ggt tac agc aaa tta cat gat gac cta gga gag ttt     336
   Leu Glu Ile Arg Gly Tyr Ser Lys Leu His Asp Asp Leu Gly Glu Phe
               100                 105                 110
   cca tat gga ttg ttt gaa ctt gtc gtt agt ata ata tct ttc ctc ttt     384
   Pro Tyr Gly Leu Phe Glu Leu Val Val Ser Ile Ile Ser Phe Leu Phe
           115                 120                 125
   ttc act gac atg ttc atc tac tgg att cac aga ggc ctt cat cat aga     432
   Phe Thr Asp Met Phe Ile Tyr Trp Ile His Arg Gly Leu His His Arg
       130                 135                 140
   ctg gta tat aag cgc cta cat aaa cct cac cat att tgg aag att cct     480
   Leu Val Tyr Lys Arg Leu His Lys Pro His His Ile Trp Lys Ile Pro
   145                 150                 155                 160
   act cca ttt gca agt cat gct ttt cac cct att gat ggc ttt ctt cag     528
   Thr Pro Phe Ala Ser His Ala Phe His Pro Ile Asp Gly Phe Leu Gln
                   165                 170                 175
   agt cta cct tac cat ata tac cct ttt atc ttt cca tta cac aag gtg     576
   Ser Leu Pro Tyr His Ile Tyr Pro Phe Ile Phe Pro Leu His Lys Val
               180                 185                 190
   gtt tat tta agt ctg tac atc ttg gtt aat atc tgg aca att tcc att     624
   Val Tyr Leu Ser Leu Tyr Ile Leu Val Asn Ile Trp Thr Ile Ser Ile
           195                 200                 205
   cat gac ggt gat ttt cgt gtc ccc caa atc tta cag cca ttt att aat     672
   His Asp Gly Asp Phe Arg Val Pro Gln Ile Leu Gln Pro Phe Ile Asn
       210                 215                 220
   ggc tca gct cat cat aca gac cac cat atg ttc ttt gac tat aat tat     720
   Gly Ser Ala His His Thr Asp His His Met Phe Phe Asp Tyr Asn Tyr
   225                 230                 235                 240
   gga caa tat ttc act ttg tgg gat agg att ggc ggc tca ttc aaa aat     768
   Gly Gln Tyr Phe Thr Leu Trp Asp Arg Ile Gly Gly Ser Phe Lys Asn
                   245                 250                 255
   cct tca tcc ttt gag ggg aag gga ccg ctc agt tat gtg aag gag atg     816
   Pro Ser Ser Phe Glu Gly Lys Gly Pro Leu Ser Tyr Val Lys Glu Met
               260                 265                 270
   aca gag gga aag cgc agc agc cct tca gga aat ggc tgt aag aat gaa     864
   Thr Glu Gly Lys Arg Ser Ser Pro Ser Gly Asn Gly Cys Lys Asn Glu
           275                 280                 285
   aaa tta ttc aat gga gag ttt aca aag act gaa tag                     900
   Lys Leu Phe Asn Gly Glu Phe Thr Lys Thr Glu
       290                 295                 300
<210> 10
   <211> 299
   <212> PRT
   <213> Homo sapiens
<400> 10
   Met Asp Leu Val Leu Arg Val Ala Asp Tyr Tyr Phe Phe Thr Pro Tyr
   1 5 10 15
   Val Tyr Pro Ala Thr Trp Pro Glu Asp Asp Ile Phe Arg Gln Ala Ile
   20 25 30
   Ser Leu Leu Ile Val Thr Asn Val Gly Ala Tyr Ile Leu Tyr Phe Phe
   35 40 45
   Cys Ala Thr Leu Ser Tyr Tyr Phe Val Phe Asp His Ala Leu Met Lys
   50 55 60
   His Pro Gln Phe Leu Lys Asn Gln Val Arg Arg Glu Ile Lys Phe Thr
   65 70 75 80
   Val Gln Ala Leu Pro Trp Ile Ser Ile Leu Thr Val Ala Leu Phe Leu
   85 90 95
   Leu Glu Ile Arg Gly Tyr Ser Lys Leu His Asp Asp Leu Gly Glu Phe
   100 105 110
   Pro Tyr Gly Leu Phe Glu Leu Val Val Ser Ile Ile Ser Phe Leu Phe
   115 120 125
   Phe Thr Asp Met Phe Ile Tyr Trp Ile His Arg Gly Leu His His Arg
   130 135 140
   Leu Val Tyr Lys Arg Leu His Lys Pro His His Ile Trp Lys Ile Pro
   145 150 155 160
   Thr Pro Phe Ala Ser His Ala Phe His Pro Ile Asp Gly Phe Leu Gln
   165 170 175
   Ser Leu Pro Tyr His Ile Tyr Pro Phe Ile Phe Pro Leu His Lys Val
   180 185 190
   Val Tyr Leu Ser Leu Tyr Ile Leu Val Asn Ile Trp Thr Ile Ser Ile
   195 200 205
   His Asp Gly Asp Phe Arg Val Pro Gln Ile Leu Gln Pro Phe Ile Asn
   210 215 220
   Gly Ser Ala His His Thr Asp His His Met Phe Phe Asp Tyr Asn Tyr
   225 230 235 240
   Gly Gln Tyr Phe Thr Leu Trp Asp Arg Ile Gly Gly Ser Phe Lys Asn
   245 250 255
   Pro Ser Ser Phe Glu Gly Lys Gly Pro Leu Ser Tyr Val Lys Glu Met
   260 265 270
   Thr Glu Gly Lys Arg Ser Ser Pro Ser Gly Asn Gly Cys Lys Asn Glu
   275 280 285
   Lys Leu Phe Asn Gly Glu Phe Thr Lys Thr Glu
   290 295
<210> 11
   <211> 1098
   <212> DNA
   <213> Saccharomyces cerevisiae
<220>
   <221> CDS
   <222> (1)..(1098)
<400> 11
   atg gat ttg gtc tta gaa gtc gct gac cat tat gtc tta gac gac ttg      48
   Met Asp Leu Val Leu Glu Val Ala Asp His Tyr Val Leu Asp Asp Leu
    1               5                  10                  15
   tac gct aaa gtt ctg ccc gct tcg ttg gca gct aat att cct gtc aag      96
   Tyr Ala Lys Val Leu Pro Ala Ser Leu Ala Ala Asn Ile Pro Val Lys
               20                  25                  30
   tgg cag aaa ttg cta ggg ttg aac agt ggg ttc agc aat tct acg att     144
   Trp Gln Lys Leu Leu Gly Leu Asn Ser Gly Phe Ser Asn Ser Thr Ile
           35                  40                  45
   ttg cag gag act ttg aac tcc aag aat gcc gtc aaa gaa tgt aga agg     192
   Leu Gln Glu Thr Leu Asn Ser Lys Asn Ala Val Lys Glu Cys Arg Arg
       50                  55                  60
   ttc tac ggg cag gtg cca ttc ctg ttt gat atg tcg acg acg tct ttt     240
   Phe Tyr Gly Gln Val Pro Phe Leu Phe Asp Met Ser Thr Thr Ser Phe
   65                  70                  75                  80
   gca tcg cta ttg cct cgt tcc agc atc ttg aga gaa ttc ctc tca cta     288
   Ala Ser Leu Leu Pro Arg Ser Ser Ile Leu Arg Glu Phe Leu Ser Leu
                   85                  90                  95
   tgg gtt att gtt acg atc ttt ggt tta cta ctt tac tta ttc acg gct     336
   Trp Val Ile Val Thr Ile Phe Gly Leu Leu Leu Tyr Leu Phe Thr Ala
               100                 105                 110
   agt ctc agc tac gtg ttt gtg ttt gac aag tcg att ttc aac cat cct     384
   Ser Leu Ser Tyr Val Phe Val Phe Asp Lys Ser Ile Phe Asn His Pro
           115                 120                 125
   cgt tac ttg aaa aac caa atg gca atg gaa atc aag ttg gca gtc agt     432
   Arg Tyr Leu Lys Asn Gln Met Ala Met Glu Ile Lys Leu Ala Val Ser
       130                 135                 140
   gct atc cca tgg atg tcg atg ttg acc gtt cca tgg ttt gtt atg gaa     480
   Ala Ile Pro Trp Met Ser Met Leu Thr Val Pro Trp Phe Val Met Glu
   145                 150                 155                 160
   ttg aac ggc cat tct aaa cta tac atg aag att gat tat gaa aac cac     528
   Leu Asn Gly His Ser Lys Leu Tyr Met Lys Ile Asp Tyr Glu Asn His
                   165                 170                 175
   ggt gta agg aag ctc att atc gag tac ttc act ttc atc ttt ttc act     576
   Gly Val Arg Lys Leu Ile Ile Glu Tyr Phe Thr Phe Ile Phe Phe Thr
               180                 185                 190
   gat tgc ggt gtg tat tta gcg cac aga tgg ttg cat tgg cca agg gtc     624
   Asp Cys Gly Val Tyr Leu Ala His Arg Trp Leu His Trp Pro Arg Val
   195 200 205
   tac cgt gct ctg cac aag cct cat cac aag tgg ctg gtc tgc aca cct 672
   Tyr Arg Ala Leu His Lys Pro His His Lys Trp Leu Val Cys Thr Pro
   210 215 220
   ttc gca tct cat tct ttc cat cct gta gac ggg ttt ttg caa tcc atc 720
   Phe Ala Ser His Ser Phe His Pro Val Asp Gly Phe Leu Gln Ser Ile
   225 230 235 240
   tcg tac cac atc tac cca ttg att ctg cca tta cac aag gtt tct tat 768
   Ser Tyr His Ile Tyr Pro Leu Ile Leu Pro Leu His Lys Val Ser Tyr
   245 250 255
   ttg att ctg ttc act ttt gtt aac ttt tgg act gtt atg att cat gac 816
   Leu Ile Leu Phe Thr Phe Val Asn Phe Trp Thr Val Met Ile His Asp
   260 265 270
   ggt caa tac cta tca aac aat cct gcc gtc aac ggt act gcc tgc cac 864
   Gly Gln Tyr Leu Ser Asn Asn Pro Ala Val Asn Gly Thr Ala Cys His
   275 280 285
   acg gtt cac cat cta tat ttc aac tac aac tac ggt caa ttc acc act 912
   Thr Val His His Leu Tyr Phe Asn Tyr Asn Tyr Gly Gln Phe Thr Thr
   290 295 300
   ctg tgg gac aga cta ggg ggt tct tac cgt aga cca gat gac tca ttg 960
   Leu Trp Asp Arg Leu Gly Gly Ser Tyr Arg Arg Pro Asp Asp Ser Leu
   305 310 315 320
   ttt gat cct aag tta aga gat gct aag gag acc tgg gac gct caa gtt 1008
   Phe Asp Pro Lys Leu Arg Asp Ala Lys Glu Thr Trp Asp Ala Gln Val
   325 330 335
   aag gaa gtt gaa cat ttc atc aag gag gtc gaa ggt gat gat aat gat 1056
   Lys Glu Val Glu His Phe Ile Lys Glu Val Glu Gly Asp Asp Asn Asp
   340 345 350
   aga atc tat gaa aac gac cca aat acc aag aag aac aac tga 1098
   Arg Ile Tyr Glu Asn Asp Pro Asn Thr Lys Lys Asn Asn
   355 360 365
<210> 12
   <211> 365
   <212> PRT
   <213> Saccharomyces cerevisiae
<400> 12
   Met Asp Leu Val Leu Glu Val Ala Asp His Tyr Val Leu Asp Asp Leu
    1              5                   10                  15
   Tyr Ala Lys Val Leu Pro Ala Ser Leu Ala Ala Asn Ile Pro Val Lys
               20                  25                  30
   Trp Gln Lys Leu Leu Gly Leu Asn Ser Gly Phe Ser Asn Ser Thr Ile
           35                  40                  45
   Leu Gln Glu Thr Leu Asn Ser Lys Asn Ala Val Lys Glu Cys Arg Arg
        50                  55                  60
   Phe Tyr Gly Gln Val Pro Phe Leu Phe Asp Met Ser Thr Thr Ser Phe
   65                  70                  75                  80
   Ala Ser Leu Leu Pro Arg Ser Ser Ile Leu Arg Glu Phe Leu Ser Leu
                   85                  90                  95
   Trp Val Ile Val Thr Ile Phe Gly Leu Leu Leu Tyr Leu Phe Thr Ala
               100                 105                 110
   Ser Leu Ser Tyr Val Phe Val Phe Asp Lys Ser Ile Phe Asn His Pro
           115                 120                 125
   Arg Tyr Leu Lys Asn Gln Met Ala Met Glu Ile Lys Leu Ala Val Ser
       130                 135                 140
   Ala Ile Pro Trp Met Ser Met Leu Thr Val Pro Trp Phe Val Met Glu
   145                 150                 155                 160
   Leu Asn Gly His Ser Lys Leu Tyr Met Lys Ile Asp Tyr Glu Asn His
                   165                 170                 175
   Gly Val Arg Lys Leu Ile Ile Glu Tyr Phe Thr Phe Ile Phe Phe Thr
               180                 185                 190
   Asp Cys Gly Val Tyr Leu Ala His Arg Trp Leu His Trp Pro Arg Val
           195                 200                 205
   Tyr Arg Ala Leu His Lys Pro His His Lys Trp Leu Val Cys Thr Pro
       210                 215                 220
   Phe Ala Ser His Ser Phe His Pro Val Asp Gly Phe Leu Gln Ser Ile
   225                 230                 235                 240
   Ser Tyr His Ile Tyr Pro Leu Ile Leu Pro Leu His Lys Val Ser Tyr
                   245                 250                 255
   Leu Ile Leu Phe Thr Phe Val Asn Phe Trp Thr Val Met Ile His Asp
               260                 265                 270
   Gly Gln Tyr Leu Ser Asn Asn Pro Ala Val Asn Gly Thr Ala Cys His
           275                 280                 285
   Thr Val His His Leu Tyr Phe Asn Tyr Asn Tyr Gly Gln Phe Thr Thr
       290                 295                 300
   Leu Trp Asp Arg Leu Gly Gly Ser Tyr Arg Arg Pro Asp Asp Ser Leu
   305                 310                 315                 320
   Phe Asp Pro Lys Leu Arg Asp Ala Lys Glu Thr Trp Asp Ala Gln Val
                   325                 330                 335
   Lys Glu Val Glu His Phe Ile Lys Glu Val Glu Gly Asp Asp Asn Asp
               340                 345                 350
   Arg Ile Tyr Glu Asn Asp Pro Asn Thr Lys Lys Asn Asn
   355 360 365
<210> 13
   <211> 1557
   <212> DNA
   <213> Mus musculus
<220>
   <221> CDS
   <222> (1)..(1557)
<400> 13
   atg gag ccc gcc gtg tcg ctg gcc gtg tgc gcg ctg ctc ttt ctg ctc     48
   Met Glu Pro Ala Val Ser Leu Ala Val Cys Ala Leu Leu Phe Leu Leu
    1               5                  10                  15
   tgg gtg cga gtg aag ggg ttg gag ttc gtt ctc atc cac cag cgc tgg     96
   Trp Val Arg Val Lys Gly Leu Glu Phe Val Leu Ile His Gln Arg Trp
                   20                  25                  30
   gtg ttc gtg tgc ctc ttc ttg ctg ccg ctc tcg ctc atc ttc gat atc    144
   Val Phe Val Cys Leu Phe Leu Leu Pro Leu Ser Leu Ile Phe Asp Ile
               35                  40                  45
   tac tac tac gtg cgc gcc tgg gtg gtg ttc aag ctg agc agt gcg ccg    192
   Tyr Tyr Tyr Val Arg Ala Trp Val Val Phe Lys Leu Ser Ser Ala Pro
           50                  55                  60
   cgc ctg cac gag cag cgc gtg cgg gac atc cag aaa cag gtc cgg gaa    240
   Arg Leu His Glu Gln Arg Val Arg Asp Ile Gln Lys Gln Val Arg Glu
   65                  70                  75                  80
   tgg aag gaa cag ggc agt aag acc ttc atg tgc acg ggg cgc cca ggc    288
   Trp Lys Glu Gln Gly Ser Lys Thr Phe Met Cys Thr Gly Arg Pro Gly
                   85                  90                  95
   tgg ctc act gtc tcg ctg cga gtc gga aag tac aag aag acc cat aag    336
   Trp Leu Thr Val Ser Leu Arg Val Gly Lys Tyr Lys Lys Thr His Lys
               100                 105                 110
   aac atc atg atc aac ctg atg gac atc ctg gag gtg gac acc aag aaa    384
   Asn Ile Met Ile Asn Leu Met Asp Ile Leu Glu Val Asp Thr Lys Lys
           115                 120                 125
   cag att gtt cga gtg gag ccc ttg gtg tct atg ggt cag gtg aca gct    432
   Gln Ile Val Arg Val Glu Pro Leu Val Ser Met Gly Gln Val Thr Ala
       130                 135                 140
   ttg ctg aac tcc att ggc tgg acc ctg cct gtg ttg cct gag ctt gat    480
   Leu Leu Asn Ser Ile Gly Trp Thr Leu Pro Val Leu Pro Glu Leu Asp
   145                 150                 155                 160
   gac ctc aca gtg ggg ggc ctg atc atg ggc aca ggc atc gag tca tcg    528
   Asp Leu Thr Val Gly Gly Leu Ile Met Gly Thr Gly Ile Glu Ser Ser
                   165                 170                 175
   tcc cac aag tat ggc ctg ttc caa cac att tgc act gcc tac gag ctg      576
   Ser His Lys Tyr Gly Leu Phe Gln His Ile Cys Thr Ala Tyr Glu Leu
               180                 185                 190
   atc ctg gca gac ggc agc ttt gtg cgc tgc aca ccg tct gaa aac tca      624
   Ile Leu Ala Asp Gly Ser Phe Val Arg Cys Thr Pro Ser Glu Asn Ser
           195                 200                 205
   gac ctg ttc tat gcc gtg ccc tgg tcc tgt ggg acc ctg ggc ttc ctg      672
   Asp Leu Phe Tyr Ala Val Pro Trp Ser Cys Gly Thr Leu Gly Phe Leu
       210                 215                 220
   gtg gct gcc gag atc cgg atc atc ccg gcc aag aag tat gtc aag ctg      720
   Val Ala Ala Glu Ile Arg Ile Ile Pro Ala Lys Lys Tyr Val Lys Leu
   225                 230                 235                 240
   cgg ttt gag cct gtt cgg ggc ctg gag gcc atc tgt gaa aaa ttc acc      768
   Arg Phe Glu Pro Val Arg Gly Leu Glu Ala Ile Cys Glu Lys Phe Thr
                   245                 250                 255
   cgc gag tcc cag cgg ctg gag aac cac ttc gtg gaa ggg ttg ctg tac      816
   Arg Glu Ser Gln Arg Leu Glu Asn His Phe Val Glu Gly Leu Leu Tyr
               260                 265                 270
   tcc ctg gat gag gct gtg gct gtc atc atg aca ggg gtc atg acg gac      864
   Ser Leu Asp Glu Ala Val Ala Val Ile Met Thr Gly Val Met Thr Asp
           275                 280                 285
   gac gta gag tcc agc aag ctg aat agc att ggc agt tac tac aag ccc      912
   Asp Val Glu Ser Ser Lys Leu Asn Ser Ile Gly Ser Tyr Tyr Lys Pro
       290                 295                 300
   tgg ttc ttc aag cat gtg gag aac tac ctg aag aca aac cgg gag ggc      960
   Trp Phe Phe Lys His Val Glu Asn Tyr Leu Lys Thr Asn Arg Glu Gly
   305                 310                 315                 320
   ctc gaa tac att ccc ctg aga cac tac tac cac cga cac acg cgc agc      1008
   Leu Glu Tyr Ile Pro Leu Arg His Tyr Tyr His Arg His Thr Arg Ser
                   325                 330                 335
   atc ttc tgg gag ctc cag gac atc atc cct ttc ggc aac aac ccc atc      1056
   Ile Phe Trp Glu Leu Gln Asp Ile Ile Pro Phe Gly Asn Asn Pro Ile
               340                 345                 350
   ttc cgc tac ctc ttc ggc tgg atg gtg cct ccc aag atc tcc ctc ctg      1104
   Phe Arg Tyr Leu Phe Gly Trp Met Val Pro Pro Lys Ile Ser Leu Leu
           355                 360                 365
   aag ctg acc cag ggc gag acg cta cgc aag ctg tac gag cag cac cac      1152
   Lys Leu Thr Gln Gly Glu Thr Leu Arg Lys Leu Tyr Glu Gln His His
       370                 375                 380
   gtg gtg cag gac atg ctg gtg ccc atg aag tgc atg tca cag gcc ctg      1200
   Val Val Gln Asp Met Leu Val Pro Met Lys Cys Met Ser Gln Ala Leu
   385                 390                 395                 400
   cat acc ttc caa aat gac atc cac gtc tac ccc atc tgg ctg tgc cca      1248
   His Thr Phe Gln Asn Asp Ile His Val Tyr Pro Ile Trp Leu Cys Pro
                   405                 410                 415
   ttc atc ctg ccc agc cag cca gga cta gtg cat ccc aag gga gat gaa      1296
   Phe Ile Leu Pro Ser Gln Pro Gly Leu Val His Pro Lys Gly Asp Glu
               420                 425                 430
   gca gag ctc tac gtg gac atc ggg gca tac ggg gag cca cgt gtg aag      1344
   Ala Glu Leu Tyr Val Asp Ile Gly Ala Tyr Gly Glu Pro Arg Val Lys
           435                 440                 445
   cac ttc gag gcc agg tcc tgc atg agg cag ctg gag aag ttt gtg cgg      1392
   His Phe Glu Ala Arg Ser Cys Met Arg Gln Leu Glu Lys Phe Val Arg
       450                 455                 460
   agt gtg cac ggg ttc caa atg tta tac gcc gat tgc tat atg aac cgc      1440
   Ser Val His Gly Phe Gln Met Leu Tyr Ala Asp Cys Tyr Met Asn Arg
   465                 470                 475                 480
   gag gaa ttc tgg gag atg ttc gat ggc tcc ttg tac cac aag ctg cgc      1488
   Glu Glu Phe Trp Glu Met Phe Asp Gly Ser Leu Tyr His Lys Leu Arg
                   485                 490                 495
   aag cag ctg ggc tgc cag gac gcc ttc cct gag gtg tac gac aag atc      1536
   Lys Gln Leu Gly Cys Gln Asp Ala Phe Pro Glu Val Tyr Asp Lys Ile
               500                 505                 510
   tgc aag gcg gca agg cac tga                                          1557
   Cys Lys Ala Ala Arg His
           515
<210> 14
   <211> 518
   <212> PRT
   <213> Mus musculus
<400> 14
   Met Glu Pro Ala Val Ser Leu Ala Val Cys Ala Leu Leu Phe Leu Leu
   1               5                   10                  15
   Trp Val Arg Val Lys Gly Leu Glu Phe Val Leu Ile His Gln Arg Trp
               20                  25                  30
   Val Phe Val Cys Leu Phe Leu Leu Pro Leu Ser Leu Ile Phe Asp Ile
           35                  40                  45
   Tyr Tyr Tyr Val Arg Ala Trp Val Val Phe Lys Leu Ser Ser Ala Pro
       50                  55                  60
   Arg Leu His Glu Gln Arg Val Arg Asp Ile Gln Lys Gln Val Arg Glu
   65                  70                  75                  80
   Trp Lys Glu Gln Gly Ser Lys Thr Phe Met Cys Thr Gly Arg Pro Gly
                   85                  90                  95
   Trp Leu Thr Val Ser Leu Arg Val Gly Lys Tyr Lys Lys Thr His Lys
               100                 105                 110
   Asn Ile Met Ile Asn Leu Met Asp Ile Leu Glu Val Asp Thr Lys Lys
           115                 120                 125
   Gln Ile Val Arg Val Glu Pro Leu Val Ser Met Gly Gln Val Thr Ala
       130                 135                 140
   Leu Leu Asn Ser Ile Gly Trp Thr Leu Pro Val Leu Pro Glu Leu Asp
   145                 150                 155                 160
   Asp Leu Thr Val Gly Gly Leu Ile Met Gly Thr Gly Ile Glu Ser Ser
                   165                 170                 175
   Ser His Lys Tyr Gly Leu Phe Gln His Ile Cys Thr Ala Tyr Glu Leu
               180                 185                 190
   Ile Leu Ala Asp Gly Ser Phe Val Arg Cys Thr Pro Ser Glu Asn Ser
           195                 200                 205
   Asp Leu Phe Tyr Ala Val Pro Trp Ser Cys Gly Thr Leu Gly Phe Leu
       210                 215                 220
   Val Ala Ala Glu Ile Arg Ile Ile Pro Ala Lys Lys Tyr Val Lys Leu
   225                 230                 235                 240
   Arg Phe Glu Pro Val Arg Gly Leu Glu Ala Ile Cys Glu Lys Phe Thr
                   245                 250                 255
   Arg Glu Ser Gln Arg Leu Glu Asn His Phe Val Glu Gly Leu Leu Tyr
               260                 265                 270
   Ser Leu Asp Glu Ala Val Ala Val Ile Met Thr Gly Val Met Thr Asp
           275                 280                 285
   Asp Val Glu Ser Ser Lys Leu Asn Ser Ile Gly Ser Tyr Tyr Lys Pro
       290                 295                 300
   Trp Phe Phe Lys His Val Glu Asn Tyr Leu Lys Thr Asn Arg Glu Gly
   305                 310                 315                 320
   Leu Glu Tyr Ile Pro Leu Arg His Tyr Tyr His Arg His Thr Arg Ser
                   325                 330                 335
   Ile Phe Trp Glu Leu Gln Asp Ile Ile Pro Phe Gly Asn Asn Pro Ile
               340                 345                 350
   Phe Arg Tyr Leu Phe Gly Trp Met Val Pro Pro Lys Ile Ser Leu Leu
           355                 360                 365
   Lys Leu Thr Gln Gly Glu Thr Leu Arg Lys Leu Tyr Glu Gln His His
       370                 375                 380
   Val Val Gln Asp Met Leu Val Pro Met Lys Cys Met Ser Gln Ala Leu
   385                 390                 395                 400
   His Thr Phe Gln Asn Asp Ile His Val Tyr Pro Ile Trp Leu Cys Pro
   405 410 415
   Phe Ile Leu Pro Ser Gln Pro Gly Leu Val His Pro Lys Gly Asp Glu
   420 425 430
   Ala Glu Leu Tyr Val Asp Ile Gly Ala Tyr Gly Glu Pro Arg Val Lys
   435 440 445
   His Phe Glu Ala Arg Ser Cys Met Arg Gln Leu Glu Lys Phe Val Arg
   450 455 460
   Ser Val His Gly Phe Gln Met Leu Tyr Ala Asp Cys Tyr Met Asn Arg
   465 470 475 480
   Glu Glu Phe Trp Glu Met Phe Asp Gly Ser Leu Tyr His Lys Leu Arg
   485 490 495
   Lys Gln Leu Gly Cys Gln Asp Ala Phe Pro Glu Val Tyr Asp Lys Ile
   500 505 510
   Cys Lys Ala Ala Arg His
   515
<210> 15
   <211> 1551
   <212> DNA
   <213> Homo sapiens
<220>
   <221> CDS
   <222> (1)..(1551)
<400> 15
atg gag ccc gcc gtg tcg ctg gcc gtg tgc gcg ctg ctc ttc ctg ctg    48
   tgg gtg cgc ctg aag ggg ctg gag ttc gtg ctc atc cac cag cgc tgg    96
   gtg ttc gtg tgc ctc ttc ctc ctg ccg ctc tcg ctt atc ttc gat atc   144
   tac tac tac gtg cgc gcc tgg gtg gtg ttc aag ctc agc agc gct ccg   192
   cgc ctg cac gag cag cgc gtg cgg gac atc cag aag cag gtg cgg gaa   240
   tgg aag gag cag ggt agc aag acc ttc atg tgc acg ggg cgc cct ggc   288
   tgg ctc act gtc tca cta cgt gtc ggg aag tac aag aag aca cac aaa    336
   aac atc atg atc aac ctg atg gac att ctg gaa gtg gac acc aag aaa    384
   cag att gtc cgt gtg gag ccc ttg gtg acc atg ggc cag gtg act gcc    432
   ctg ctg acc tcc att ggc tgg act ctc ccc gtg ttg cct gag ctt gat    480
   gac ctc aca gtg ggg ggc ttg atc atg ggc aca ggc atc gag tca tca    528
   tcc cac aag tac ggc ctg ttc caa cac atc tgc act gct tac gag ctg    576
   gtc ctg gct gat ggc agc ttt gtg cga tgc act ccg tcc gaa aac tca    624
   gac ctg ttc tat gcc gta ccc tgg tcc tgt ggg acg ctg ggt ttc ctg    672
   gtg gcc gct gag atc cgc atc atc cct gcc aag aag tac gtc aag ctg    720
   cgt ttc gag cca gtg cgg ggc ctg gag gct atc tgt gcc aag ttc acc    768
   cac gag tcc cag cgg cag gag aac cac ttc gtg gaa ggg ctg ctc tac    816
   tcc ctg gat gag gct gtc att atg aca ggg gtc atg aca gat gag gca    864
   gag ccc agc aag ctg aat agc att ggc aat tac tac aag ccg tgg ttc    912
   ttt aag cat gtg gag aac tat ctg aag aca aac cga gag ggc ctg gag    960
   tac att ccc ttg aga cac tac tac cac cgc cac acg cgc agc atc ttc 1008
   tgg gag ctc cag gac atc atc ccc ttt ggc aac aac ccc atc ttc cgc 1056
   tac ctc ttt ggc tgg atg gtg cct ccc aag atc tcc ctc ctg aag ctg 1104
   acc cag ggt gag acc ctg cgc aag ctg tac gag cag cac cac gtg gtg 1152
   cag gac atg ctg gtg ccc atg aag tgc ctg cag cag gcc ctg cac acc 1200
   ttc caa aac gac atc cac gtc tac ccc atc tgg ctg tgt ccg ttc atc 1248
   ctg ccc agc cag cca ggc cta gtg cac ccc aaa gga aat gag gca gag 1296
   ctc tac atc gac att gga gca tat ggg gag ccg cgt gtg aaa cac ttt 1344
   gaa gcc agg tcc tgc atg agg cag ctg gag aag ttt gtc cgc agc gtg 1392
   cat ggc ttc cag atg ctg tat gcc gac tgc tac atg aac cgg gag gag 1440
   ttc tgg gag atg ttt gat ggc tcc ttg tac cac aag ctg cga gag aag 1488
   ctg ggt tgc cag gac gcc ttc ccc gag gtg tac gac aag atc tgc aag 1536
   gcc gcc agg cac tga 1551
<210> 16
   <211> 516
   <212> PRT
   <213> Homo sapiens
<400> 16
<210> 17
   <211> 1422
   <212> DNA
   <213> Saccharomyces cerevisiae
<220>
   <221> CDS
   <222> (1)..(1422)
<400> 17
   atg gca aag gat aat agt gag aag ctg cag gtg cag gga gag gag aaa    48
   Met Ala Lys Asp Asn Ser Glu Lys Leu Gln Val Gln Gly Glu Glu Lys
    1               5                  10                  15
   aag tcc aag caa ccg gtt aat ttc ctg cct cag ggt aaa tgg ctg aag    96
   Lys Ser Lys Gln Pro Val Asn Phe Leu Pro Gln Gly Lys Trp Leu Lys
               20                  25                  30
   cca aat gaa atc gaa tat gag ttt ggt ggg act act ggt gtt att ggt   144
   Pro Asn Glu Ile Glu Tyr Glu Phe Gly Gly Thr Thr Gly Val Ile Gly
           35                  40                  45
   atg ctg atc ggg ttt cca ctg cta atg tac tat atg tgg att tgt gcg   192
   Met Leu Ile Gly Phe Pro Leu Leu Met Tyr Tyr Met Trp Ile Cys Ala
       50                  55                  60
   gaa ttt tat cac ggt aag gtt gcc cta ccc aag gct ggt gaa tcg tgg   240
   Glu Phe Tyr His Gly Lys Val Ala Leu Pro Lys Ala Gly Glu Ser Trp
   65                  70                  75                  80
   atg cac ttt atc aag cac cta tac cag tta gtc ttg gag aac ggt atc   288
   Met His Phe Ile Lys His Leu Tyr Gln Leu Val Leu Glu Asn Gly Ile
                   85                  90                  95
   cca gaa aag tat gac tgg act att ttc tta aca ttt tgg gtg ttt cag   336
   Pro Glu Lys Tyr Asp Trp Thr Ile Phe Leu Thr Phe Trp Val Phe Gln
               100                 105                 110
   atc att ttc tac tat acg ttg ccc ggg att tgg aca aaa ggt caa cca   384
   Ile Ile Phe Tyr Tyr Thr Leu Pro Gly Ile Trp Thr Lys Gly Gln Pro
           115                 120                 125
   ttg tct cat ttg aag gga aaa caa ttg cct tac ttt tgt aat gcc atg   432
   Leu Ser His Leu Lys Gly Lys Gln Leu Pro Tyr Phe Cys Asn Ala Met
       130                 135                 140
   tgg acc ttg tat gta act acc act ttg gtc ttg gtt ttg cac ttt acc   480
   Trp Thr Leu Tyr Val Thr Thr Thr Leu Val Leu Val Leu His Phe Thr
   145                 150                 155                 160
   aat ctt ttt aga ttg tat gtc att att gac cgt ttt ggg agg atc atg   528
   Asn Leu Phe Arg Leu Tyr Val Ile Ile Asp Arg Phe Gly Arg Ile Met
                   165                 170                 175
   aca tgt gcc att att tca ggg ttt gcc ttc tcc atc ata ttg tac tta   576
   Thr Cys Ala Ile Ile Ser Gly Phe Ala Phe Ser Ile Ile Leu Tyr Leu
               180                 185                 190
   tgg act tta ttt atc tca cat gac tat cat aga atg aca gga aac cat   624
   Trp Thr Leu Phe Ile Ser His Asp Tyr His Arg Met Thr Gly Asn His
           195                 200                 205
   cta tat gat ttc ttc atg gga gct cca cta aac cct agg tgg ggg att   672
   Leu Tyr Asp Phe Phe Met Gly Ala Pro Leu Asn Pro Arg Trp Gly Ile
       210                 215                 220
   ttg gac ttg aag atg ttt ttc gag gtt aga tta cct tgg ttc acc ctt   720
   Leu Asp Leu Lys Met Phe Phe Glu Val Arg Leu Pro Trp Phe Thr Leu
   225                 230                 235                 240
   tac ttt atc act ttg ggt gcc tgt ttg aag cag tgg gag act tac ggc      768
   Tyr Phe Ile Thr Leu Gly Ala Cys Leu Lys Gln Trp Glu Thr Tyr Gly
                   245                 250                 255
   tat gtg aca cca caa ttg ggg gtt gtc atg tta gct cat tgg ttg tac      816
   Tyr Val Thr Pro Gln Leu Gly Val Val Met Leu Ala His Trp Leu Tyr
               260                 265                 270
   gcg aac gca tgt gct aaa ggt gaa gaa ttg att gtt cca acc tgg gac      864
   Ala Asn Ala Cys Ala Lys Gly Glu Glu Leu Ile Val Pro Thr Trp Asp
           275                 280                 285
   atg gct tac gaa aag ttt gga ttt atg ctg atc ttc tgg aat att gcc      912
   Met Ala Tyr Glu Lys Phe Gly Phe Met Leu Ile Phe Trp Asn Ile Ala
       290                 295                 300
   ggt gtc cca tac act tac tgt cat tgt acg ttg tat ttg tac tac cat      960
   Gly Val Pro Tyr Thr Tyr Cys His Cys Thr Leu Tyr Leu Tyr Tyr His
   305                 310                 315                 320
   gac cca tct gaa tat cac tgg tct aca ctg tac aat gtt tcg ctg tac      1008
   Asp Pro Ser Glu Tyr His Trp Ser Thr Leu Tyr Asn Val Ser Leu Tyr
                   325                 330                 335
   gtt gtt cta tta tgc gcc tac tac ttc ttt gac acg gca aat gct cag      1056
   Val Val Leu Leu Cys Ala Tyr Tyr Phe Phe Asp Thr Ala Asn Ala Gln
               340                 345                 350
   aaa aat gcc ttc aga aag caa atg tct ggt gac aag aca ggt agg aag      1104
   Lys Asn Ala Phe Arg Lys Gln Met Ser Gly Asp Lys Thr Gly Arg Lys
           355                 360                 365
   act ttc cca ttt ttg cca tac caa att ttg aag aat cca aag tat atg      1152
   Thr Phe Pro Phe Leu Pro Tyr Gln Ile Leu Lys Asn Pro Lys Tyr Met
       370                 375                 380
   gtt acc tcc aat gga tcg tac cta ttg att gat ggt tgg tac act ttg      1200
   Val Thr Ser Asn Gly Ser Tyr Leu Leu Ile Asp Gly Trp Tyr Thr Leu
   385                 390                 395                 400
   gct aga aaa att cac tac act gcc gat tgg act caa tct ctc gtt tgg      1248
   Ala Arg Lys Ile His Tyr Thr Ala Asp Trp Thr Gln Ser Leu Val Trp
                   405                 410                 415
   gcc ttg tct tgc ggg ttc aac tcg gtg ttc cca tgg ttt ttc cca gta      1296
   Ala Leu Ser Cys Gly Phe Asn Ser Val Phe Pro Trp Phe Phe Pro Val
               420                 425                 430
   ttc ttc ctt gtt gtc ctg att cac aga gcc ttc aga gac caa gca aaa      1344
   Phe Phe Leu Val Val Leu Ile His Arg Ala Phe Arg Asp Gln Ala Lys
           435                 440                 445
   tgt aag aga aag tac gga aaa gat tgg gat gag tat tgt aaa cat tgc      1392
   Cys Lys Arg Lys Tyr Gly Lys Asp Trp Asp Glu Tyr Cys Lys His Cys
   450 455 460
   cct tac gtc ttt att cct tat gtt ttc tag 1422
   Pro Tyr Val Phe Ile Pro Tyr Val Phe
   465 470
<210> 18
   <211> 473
   <212> PRT
   <213> Saccharomyces cerevisiae
<400> 18
   Met Ala Lys Asp Asn Ser Glu Lys Leu Gln Val Gln Gly Glu Glu Lys
   1               5                   10                  15
   Lys Ser Lys Gln Pro Val Asn Phe Leu Pro Gln Gly Lys Trp Leu Lys
               20                  25                  30
   Pro Asn Glu Ile Glu Tyr Glu Phe Gly Gly Thr Thr Gly Val Ile Gly
           35                  40                  45
   Met Leu Ile Gly Phe Pro Leu Leu Met Tyr Tyr Met Trp Ile Cys Ala
       50                  55                  60
   Glu Phe Tyr His Gly Lys Val Ala Leu Pro Lys Ala Gly Glu Ser Trp
   65                  70                  75                  80
   Met His Phe Ile Lys His Leu Tyr Gln Leu Val Leu Glu Asn Gly Ile
                   85                  90                  95
   Pro Glu Lys Tyr Asp Trp Thr Ile Phe Leu Thr Phe Trp Val Phe Gln
               100                 105                 110
   Ile Ile Phe Tyr Tyr Thr Leu Pro Gly Ile Trp Thr Lys Gly Gln Pro
           115                 120                 125
   Leu Ser His Leu Lys Gly Lys Gln Leu Pro Tyr Phe Cys Asn Ala Met
       130                 135                 140
   Trp Thr Leu Tyr Val Thr Thr Thr Leu Val Leu Val Leu His Phe Thr
   145                 150                 155                 160
   Asn Leu Phe Arg Leu Tyr Val Ile Ile Asp Arg Phe Gly Arg Ile Met
                   165                 170                 175
   Thr Cys Ala Ile Ile Ser Gly Phe Ala Phe Ser Ile Ile Leu Tyr Leu
               180                 185                 190
   Trp Thr Leu Phe Ile Ser His Asp Tyr His Arg Met Thr Gly Asn His
           195                 200                 205
   Leu Tyr Asp Phe Phe Met Gly Ala Pro Leu Asn Pro Arg Trp Gly Ile
       210                 215                 220
   Leu Asp Leu Lys Met Phe Phe Glu Val Arg Leu Pro Trp Phe Thr Leu
   225                 230                 235                 240
   Tyr Phe Ile Thr Leu Gly Ala Cys Leu Lys Gln Trp Glu Thr Tyr Gly
   245 250 255
   Tyr Val Thr Pro Gln Leu Gly Val Val Met Leu Ala His Trp Leu Tyr
   260 265 270
   Ala Asn Ala Cys Ala Lys Gly Glu Glu Leu Ile Val Pro Thr Trp Asp
   275 280 285
   Met Ala Tyr Glu Lys Phe Gly Phe Met Leu Ile Phe Trp Asn Ile Ala
   290 295 300
   Gly Val Pro Tyr Thr Tyr Cys His Cys Thr Leu Tyr Leu Tyr Tyr His
   305 310 315 320
   Asp Pro Ser Glu Tyr His Trp Ser Thr Leu Tyr Asn Val Ser Leu Tyr
   325 330 335
   Val Val Leu Leu Cys Ala Tyr Tyr Phe Phe Asp Thr Ala Asn Ala Gln
   340 345 350
   Lys Asn Ala Phe Arg Lys Gln Met Ser Gly Asp Lys Thr Gly Arg Lys
   355 360 365
   Thr Phe Pro Phe Leu Pro Tyr Gln Ile Leu Lys Asn Pro Lys Tyr Met
   370 375 380
   Val Thr Ser Asn Gly Ser Tyr Leu Leu Ile Asp Gly Trp Tyr Thr Leu
   385 390 395 400
   Ala Arg Lys Ile His Tyr Thr Ala Asp Trp Thr Gln Ser Leu Val Trp
   405 410 415
   Ala Leu Ser Cys Gly Phe Asn Ser Val Phe Pro Trp Phe Phe Pro Val
   420 425 430
   Phe Phe Leu Val Val Leu Ile His Arg Ala Phe Arg Asp Gln Ala Lys
   435 440 445
   Cys Lys Arg Lys Tyr Gly Lys Asp Trp Asp Glu Tyr Cys Lys His Cys
   450 455 460
   Pro Tyr Val Phe Ile Pro Tyr Val Phe
   465 470
<210> 19
   <211> 1152
   <212> DNA
   <213> Saccharomyces cerevisiae
<220>
   <221> CDS
   <222> (1)..(1152)
<400> 19
   atg agt gaa aca gaa ttg aga aaa aga cag gcc caa ttc act agg gag      48
   Met Ser Glu Thr Glu Leu Arg Lys Arg Gln Ala Gln Phe Thr Arg Glu
    1               5                  10                  15
   tta cat ggt gat gat att ggt aaa aag aca ggt ttg agt gca ttg atg      96
   Leu His Gly Asp Asp Ile Gly Lys Lys Thr Gly Leu Ser Ala Leu Met
               20                  25                  30
   tcg aag aac aac tct gcc caa aag gaa gcc gtt cag aag tac ttg aga     144
   Ser Lys Asn Asn Ser Ala Gln Lys Glu Ala Val Gln Lys Tyr Leu Arg
           35                  40                  45
   aat tgg gat ggt aga acc gat aaa gat gcc gaa gaa cgt cgt ctt gag     192
   Asn Trp Asp Gly Arg Thr Asp Lys Asp Ala Glu Glu Arg Arg Leu Glu
       50                  55                  60
   gat tat aat gaa gcc aca cat tcc tac tat aac gtc gtt aca gat ttc     240
   Asp Tyr Asn Glu Ala Thr His Ser Tyr Tyr Asn Val Val Thr Asp Phe
   65                  70                  75                  80
   tat gaa tat ggt tgg ggt tcc tct ttc cat ttc agc aga ttt tat aaa     288
   Tyr Glu Tyr Gly Trp Gly Ser Ser Phe His Phe Ser Arg Phe Tyr Lys
                   85                  90                  95
   ggt gag agt ttc gct gcc tcg ata gca aga cat gaa cat tat tta gct     336
   Gly Glu Ser Phe Ala Ala Ser Ile Ala Arg His Glu His Tyr Leu Ala
               100                 105                 110
   tac aag gct ggt att caa aga ggc gat tta gtt ctc gac gtt ggt tgt     384
   Tyr Lys Ala Gly Ile Gln Arg Gly Asp Leu Val Leu Asp Val Gly Cys
           115                 120                 125
   ggt gtt ggg ggc cca gca aga gag att gca aga ttt acc ggt tgt aac     432
   Gly Val Gly Gly Pro Ala Arg Glu Ile Ala Arg Phe Thr Gly Cys Asn
       130                 135                 140
   gtc atc ggt cta aac aat aac gat tac caa att gcc aag gca aaa tat     480
   Val Ile Gly Leu Asn Asn Asn Asp Tyr Gln Ile Ala Lys Ala Lys Tyr
   145                 150                 155                 160
   tac gct aaa aaa tac aat ttg agt gac caa atg gac ttt gta aag ggt     528
   Tyr Ala Lys Lys Tyr Asn Leu Ser Asp Gln Met Asp Phe Val Lys Gly
                   165                 170                 175
   gat ttc atg aaa atg gat ttc gaa gaa aac act ttc gac aaa gtt tat     576
   Asp Phe Met Lys Met Asp Phe Glu Glu Asn Thr Phe Asp Lys Val Tyr
               180                 185                 190
   gca att gag gcc aca tgt cac gct cca aaa tta gaa ggt gta tac agc     624
   Ala Ile Glu Ala Thr Cys His Ala Pro Lys Leu Glu Gly Val Tyr Ser
           195                 200                 205
   gaa atc tac aag gtt ttg aaa ccg ggt ggt acc ttt gct gtt tac gaa     672
   Glu Ile Tyr Lys Val Leu Lys Pro Gly Gly Thr Phe Ala Val Tyr Glu
       210                 215                 220
   tgg gta atg act gat aaa tat gac gaa aac aat cct gaa cat aga aag     720
   Trp Val Met Thr Asp Lys Tyr Asp Glu Asn Asn Pro Glu His Arg Lys
   225                 230                 235                 240
   atc gct tat gaa att gaa cta ggt gat ggt atc cca aag atg ttc cat     768
   Ile Ala Tyr Glu Ile Glu Leu Gly Asp Gly Ile Pro Lys Met Phe His
                   245                 250                 255
   gtc gac gtg gct agg aaa gca ttg aag aac tgt ggt ttc gaa gtc ctc     816
   Val Asp Val Ala Arg Lys Ala Leu Lys Asn Cys Gly Phe Glu Val Leu
               260                 265                 270
   gtt agc gaa gac ctg gcg gac aat gat gat gaa atc cct tgg tat tac     864
   Val Ser Glu Asp Leu Ala Asp Asn Asp Asp Glu Ile Pro Trp Tyr Tyr
           275                 280                 285
   cca tta act ggt gag tgg aag tac gtt caa aac tta gct aat ttg gcc     912
   Pro Leu Thr Gly Glu Trp Lys Tyr Val Gln Asn Leu Ala Asn Leu Ala
       290                 295                 300
   aca ttt ttc aga act tct tac ttg ggt aga caa ttt act aca gca atg     960
   Thr Phe Phe Arg Thr Ser Tyr Leu Gly Arg Gln Phe Thr Thr Ala Met
   305                 310                 315                 320
   gtt act gta atg gaa aaa tta ggt cta gcc cca gaa ggt tcc aag gaa     1008
   Val Thr Val Met Glu Lys Leu Gly Leu Ala Pro Glu Gly Ser Lys Glu
                   325                 330                 335
   gtt act gct gct cta gaa aat gct gcg gtt ggt tta gtt gcc ggt ggt     1056
   Val Thr Ala Ala Leu Glu Asn Ala Ala Val Gly Leu Val Ala Gly Gly
               340                 345                 350
   aag tcc aag tta ttc act cca atg atg ctt ttc gtc gct agg aag cca     1104
   Lys Ser Lys Leu Phe Thr Pro Met Met Leu Phe Val Ala Arg Lys Pro
           355                 360                 365
   gaa aac gcc gaa acc ccc tcc caa act tcc caa gaa gca act caa taa     1152
   Glu Asn Ala Glu Thr Pro Ser Gln Thr Ser Gln Glu Ala Thr Gln
       370                 375                 380
<210> 20
   <211> 383
   <212> PRT
   <213> Saccharomyces cerevisiae
<400> 20 .
   Met Ser Glu Thr Glu Leu Arg Lys Arg Gln Ala Gln Phe Thr Arg Glu
   1               5                   10                  15
   Leu His Gly Asp Asp Ile Gly Lys Lys Thr Gly Leu Ser Ala Leu Met
               20                  25                  30
   Ser Lys Asn Asn Ser Ala Gln Lys Glu Ala Val Gln Lys Tyr Leu Arg
           35                  40                  45
   Asn Trp Asp Gly Arg Thr Asp Lys Asp Ala Glu Glu Arg Arg Leu Glu
       50                  55                  60
   Asp Tyr Asn Glu Ala Thr His Ser Tyr Tyr Asn Val Val Thr Asp Phe
   65 70 75 80
   Tyr Glu Tyr Gly Trp Gly Ser Ser Phe His Phe Ser Arg Phe Tyr Lys
   85 90 95
   Gly Glu Ser Phe Ala Ala Ser Ile Ala Arg His Glu His Tyr Leu Ala
   100 105 110
   Tyr Lys Ala Gly Ile Gln Arg Gly Asp Leu Val Leu Asp Val Gly Cys
   115 120 125
   Gly Val Gly Gly Pro Ala Arg Glu Ile Ala Arg Phe Thr Gly Cys Asn
   130 135 140
   Val Ile Gly Leu Asn Asn Asn Asp Tyr Gln Ile Ala Lys Ala Lys Tyr
   145 150 155 160
   Tyr Ala Lys Lys Tyr Asn Leu Ser Asp Gln Met Asp Phe Val Lys Gly
   165 170 175
   Asp Phe Met Lys Met Asp Phe Glu Glu Asn Thr Phe Asp Lys Val Tyr
   180 185 190
   Ala Ile Glu Ala Thr Cys His Ala Pro Lys Leu Glu Gly Val Tyr Ser
   195 200 205
   Glu Ile Tyr Lys Val Leu Lys Pro Gly Gly Thr Phe Ala Val Tyr Glu
   210 215 220
   Trp Val Met Thr Asp Lys Tyr Asp Glu Asn Asn Pro Glu His Arg Lys
   225 230 235 240
   Ile Ala Tyr Glu Ile Glu Leu Gly Asp Gly Ile Pro Lys Met Phe His
   245 250 255
   Val Asp Val Ala Arg Lys Ala Leu Lys Asn Cys Gly Phe Glu Val Leu
   260 265 270
   Val Ser Glu Asp Leu Ala Asp Asn Asp Asp Glu Ile Pro Trp Tyr Tyr
   275 280 285
   Pro Leu Thr Gly Glu Trp Lys Tyr Val Gln Asn Leu Ala Asn Leu Ala
   290 295 300
   Thr Phe Phe Arg Thr Ser Tyr Leu Gly Arg Gln Phe Thr Thr Ala Met
   305 310 315 320
   Val Thr Val Met Glu Lys Leu Gly Leu Ala Pro Glu Gly Ser Lys Glu
   325 330 335
   Val Thr Ala Ala Leu Glu Asn Ala Ala Val Gly Leu Val Ala Gly Gly
   340 345 350
   Lys Ser Lys Leu Phe Thr Pro Met Met Leu Phe Val Ala Arg Lys Pro
   355 360 365
   Glu Asn Ala Glu Thr Pro Ser Gln Thr Ser Gln Glu Ala Thr Gln
   370 375 380
<210> 21
   <211> 1617
   <212> DNA
   <213> Saccharomyces cerevisiae
<220>
   <221> CDS
   <222> (2)..(1617)
<400> 21
   atg agt tct gtc gca gaa aat ata ata caa cat gcc act cat aat tct    48
   Met Ser Ser Val Ala Glu Asn Ile Ile Gln His Ala Thr His Asn Ser
    1               5                  10                  15
   acg cta cac caa ttg gct aaa gac cag ccc tct gta ggc gtc act act    96
   Thr Leu His Gln Leu Ala Lys Asp Gln Pro Ser Val Gly Val Thr Thr
               20                  25                  30
   gcc ttc agt atc ctg gat aca ctt aag tct atg tca tat ttg aaa ata   144
   Ala Phe Ser Ile Leu Asp Thr Leu Lys Ser Met Ser Tyr Leu Lys Ile
           35                  40                  45
   ttt gct act tta atc tgt att ctt ttg gtt tgg gac caa gtt gca tat   192
   Phe Ala Thr Leu Ile Cys Ile Leu Leu Val Trp Asp Gln Val Ala Tyr
       50                  55                  60
   caa atc aag aaa ggt tcc atc gca ggt cca aag ttt aag ttc tgg ccc   240
   Gln Ile Lys Lys Gly Ser Ile Ala Gly Pro Lys Phe Lys Phe Trp Pro
   65                  70                  75                  80
   atc atc ggt cca ttt ttg gaa tcc tta gat cca aag ttt gaa gaa tat   288
   Ile Ile Gly Pro Phe Leu Glu Ser Leu Asp Pro Lys Phe Glu Glu Tyr
                   85                  90                  95
   aag gct aag tgg gca tcc ggt cca ctt tca tgt gtt tct att ttc cat   336
   Lys Ala Lys Trp Ala Ser Gly Pro Leu Ser Cys Val Ser Ile Phe His
               100                 105                 110
   aaa ttt gtt gtt atc gca tct act aga gac ttg gca aga aag atc ttg   384
   Lys Phe Val Val Ile Ala Ser Thr Arg Asp Leu Ala Arg Lys Ile Leu
           115                 120                 125
   caa tct tcc aaa ttc gtc aaa cct tgc gtt gtc gat gtt gct gtg aag   432
   Gln Ser Ser Lys Phe Val Lys Pro Cys Val Val Asp Val Ala Val Lys
       130                 135                 140
   atc tta aga cct tgc aat tgg gtt ttt ttg gac ggt aaa gct cat act   480
   Ile Leu Arg Pro Cys Asn Trp Val Phe Leu Asp Gly Lys Ala His Thr
   145                 150                 155                 160
   gat tac aga aaa tca tta aac ggt ctt ttc act aaa caa gct ttg gct   528
   Asp Tyr Arg Lys Ser Leu Asn Gly Leu Phe Thr Lys Gln Ala Leu Ala
               165                 170                 175
   caa tac tta cct tca ttg gaa caa atc atg gat aag tac atg gat aag      576
   Gln Tyr Leu Pro Ser Leu Glu Gln Ile Met Asp Lys Tyr Met Asp Lys
                   180                 185                 190
   ttt gtt cgt tta tct aag gag aat aac tac gag ccc cag gtc ttt ttc      624
   Phe Val Arg Leu Ser Lys Glu Asn Asn Tyr Glu Pro Gln Val Phe Phe
           195                 200                 205
   cat gaa atg aga gaa att ctt tgc gcc tta tca ttg aac tct ttc tgt      672
   His Glu Met Arg Glu Ile Leu Cys Ala Leu Ser Leu Asn Ser Phe Cys
       210                 215                 220
   ggt aac tat att acc gaa gat caa gtc aga aag att gct gat gat tac      720
   Gly Asn Tyr Ile Thr Glu Asp Gln Val Arg Lys Ile Ala Asp Asp Tyr
   225                 230                 235                 240
   tat ttg gtt aca gca gca ttg gaa tta gtc aac ttc cca att att atc      768
   Tyr Leu Val Thr Ala Ala Leu Glu Leu Val Asn Phe Pro Ile Ile Ile
                   245                 250                 255
   cct tac act aaa aca tgg tat ggt aag aaa act gca gac atg gcc atg      816
   Pro Tyr Thr Lys Thr Trp Tyr Gly Lys Lys Thr Ala Asp Met Ala Met
               260                 265                 270
   aag att ttc gaa aac tgt gct caa atg gct aag gat cat att gct gca      864
   Lys Ile Phe Glu Asn Cys Ala Gln Met Ala Lys Asp His Ile Ala Ala
           275                 280                 285
   ggt ggt aag cca gtt tgt gtt atg gat gct tgg tgt aag ttg atg cac      912
   Gly Gly Lys Pro Val Cys Val Met Asp Ala Trp Cys Lys Leu Met His
       290                 295                 300
   gat gca aag aat agt aac gat gat gat tct aga atc tac cac aga gag      960
   Asp Ala Lys Asn Ser Asn Asp Asp Asp Ser Arg Ile Tyr His Arg Glu
   305                 310                 315                 320
   ttt act aac aag gaa atc tcc gaa gct gtt ttc act ttc tta ttt gct      1008
   Phe Thr Asn Lys Glu Ile Ser Glu Ala Val Phe Thr Phe Leu Phe Ala
                   325                 330                 335
   tct caa gat gcc tct tct tct tta gct tgt tgg ttg ttc caa att gtt      1056
   Ser Gln Asp Ala Ser Ser Ser Leu Ala Cys Trp Leu Phe Gln Ile Val
               340                 345                 350
   gct gac cgt cca gat gtc tta gct aag atc aga gaa gaa caa ttg gct      1104
   Ala Asp Arg Pro Asp Val Leu Ala Lys Ile Arg Glu Glu Gln Leu Ala
           355                 360                 365
   gtt cgt aac aat gac atg tct acc gaa ttg aac ttg gat ttg att gag      1152
   Val Arg Asn Asn Asp Met Ser Thr Glu Leu Asn Leu Asp Leu Ile Glu
       370                 375                 380
   aaa atg aag tac acc aat atg gtc ata aaa gaa act ttg cgt tac aga      1200
   Lys Met Lys Tyr Thr Asn Met Val Ile Lys Glu Thr Leu Arg Tyr Arg
   385                 390                 395                 400
   cct cct gtc ttg atg gtt cca tat gtt gtt aag aag aat ttc cca gtt      1248
   Pro Pro Val Leu Met Val Pro Tyr Val Val Lys Lys Asn Phe Pro Val
                   405                 410                 415
   tcc cct aac tat acc gca cca aag ggc gct atg tta att cca acc tta      1296
   Ser Pro Asn Tyr Thr Ala Pro Lys Gly Ala Met Leu Ile Pro Thr Leu
               420                 425                 430
   tac cca gct tta cat gat cct gaa gtt tac gaa aat cct gat gag ttc      1344
   Tyr Pro Ala Leu His Asp Pro Glu Val Tyr Glu Asn Pro Asp Glu Phe
           435                 440                 445
   atc cct gaa aga tgg gta gaa ggc tct aag gct agt gaa gca aag aag      1392
   Ile Pro Glu Arg Trp Val Glu Gly Ser Lys Ala Ser Glu Ala Lys Lys
       450                 455                 460
   aat tgg ttg gtt ttt ggt tgt ggt cca cac gtt tgc tta ggt caa aca      1440
   Asn Trp Leu Val Phe Gly Cys Gly Pro His Val Cys Leu Gly Gln Thr
   465                 470                 475                 480
   tat gtc atg att acc ttc gcc gct ttg ttg ggt aaa ttt gca cta tat      1488
   Tyr Val Met Ile Thr Phe Ala Ala Leu Leu Gly Lys Phe Ala Leu Tyr
                   485                 490                 495
   act gat ttc cat cat aca gtg act cca tta agt gaa aaa atc aag gtt      1536
   Thr Asp Phe His His Thr Val Thr Pro Leu Ser Glu Lys Ile Lys Val
               500                 505                 510
   ttc gct aca att ttc cca aaa gat gat ttg tta ctg act ttc aaa aag      1584
   Phe Ala Thr Ile Phe Pro Lys Asp Asp Leu Leu Leu Thr Phe Lys Lys
           515                 520                 525
   aga gac cca att act gga gaa gtc ttc gaa taa                          1617
   Arg Asp Pro Ile Thr Gly Glu Val Phe Glu
       530                 535
<210> 22
   <211> 538
   <212> PRT
   <213> Saccharomyces cerevisiae
<400> 22
   Met Ser Ser Val Ala Glu Asn Ile Ile Gln His Ala Thr His Asn Ser
   1 5 10 15
   Thr Leu His Gln Leu Ala Lys Asp Gln Pro Ser Val Gly Val Thr Thr
   20 25 30
   Ala Phe Ser Ile Leu Asp Thr Leu Lys Ser Met Ser Tyr Leu Lys Ile
   35 40 45
   Phe Ala Thr Leu Ile Cys Ile Leu Leu Val Trp Asp Gln Val Ala Tyr
   50 55 60
   Gln Ile Lys Lys Gly Ser Ile Ala Gly Pro Lys Phe Lys Phe Trp Pro
   65 70 75 80
   Ile Ile Gly Pro Phe Leu Glu Ser Leu Asp Pro Lys Phe Glu Glu Tyr
   85 90 95
   Lys Ala Lys Trp Ala Ser Gly Pro Leu Ser Cys Val Ser Ile Phe His
   100 105 110
   Lys Phe Val Val Ile Ala Ser Thr Arg Asp Leu Ala Arg Lys Ile Leu
   115 120 125
   Gln Ser Ser Lys Phe Val Lys Pro Cys Val Val Asp Val Ala Val Lys
   130 135 140
   Ile Leu Arg Pro Cys Asn Trp Val Phe Leu Asp Gly Lys Ala His Thr
   145 150 155 160
   Asp Tyr Arg Lys Ser Leu Asn Gly Leu Phe Thr Lys Gln Ala Leu Ala
   165 170 175
   Gln Tyr Leu Pro Ser Leu Glu Gln Ile Met Asp Lys Tyr Met Asp Lys
   180 185 190
   Phe Val Arg Leu Ser Lys Glu Asn Asn Tyr Glu Pro Gln Val Phe Phe
   195 200 205
   His Glu Met Arg Glu Ile Leu Cys Ala Leu Ser Leu Asn Ser Phe Cys
   210 215 220
   Gly Asn Tyr Ile Thr Glu Asp Gln Val Arg Lys Ile Ala Asp Asp Tyr
   225 230 235 240
   Tyr Leu Val Thr Ala Ala Leu Glu Leu Val Asn Phe Pro Ile Ile Ile
   245 250 255
   Pro Tyr Thr Lys Thr Trp Tyr Gly Lys Lys Thr Ala Asp Met Ala Met
   260 265 270
   Lys Ile Phe Glu Asn Cys Ala Gln Met Ala Lys Asp His Ile Ala Ala
   275 280 285
   Gly Gly Lys Pro Val Cys Val Met Asp Ala Trp Cys Lys Leu Met His
   290 295 300
   Asp Ala Lys Asn Ser Asn Asp Asp Asp Ser Arg Ile Tyr His Arg Glu
   305 310 315 320
   Phe Thr Asn Lys Glu Ile Ser Glu Ala Val Phe Thr Phe Leu Phe Ala
   325 330 335
   Ser Gln Asp Ala Ser Ser Ser Leu Ala Cys Trp Leu Phe Gln Ile Val
   340 345 350
   Ala Asp Arg Pro Asp Val Leu Ala Lys Ile Arg Glu Glu Gln Leu Ala
   355 360 365
   Val Arg Asn Asn Asp Met Ser Thr Glu Leu Asn Leu Asp Leu Ile Glu
   370 375 380
   Lys Met Lys Tyr Thr Asn Met Val Ile Lys Glu Thr Leu Arg Tyr Arg
   385 390 395 400
   Pro Pro Val Leu Met Val Pro Tyr Val Val Lys Lys Asn Phe Pro Val
   405 410 415
   Ser Pro Asn Tyr Thr Ala Pro Lys Gly Ala Met Leu Ile Pro Thr Leu
   420 425 430
   Tyr Pro Ala Leu His Asp Pro Glu Val Tyr Glu Asn Pro Asp Glu Phe
   435 440 445
   Ile Pro Glu Arg Trp Val Glu Gly Ser Lys Ala Ser Glu Ala Lys Lys
   450 455 460
   Asn Trp Leu Val Phe Gly Cys Gly Pro His Val Cys Leu Gly Gln Thr
   465 470 475 480
   Tyr Val Met Ile Thr Phe Ala Ala Leu Leu Gly Lys Phe Ala Leu Tyr
   485 490 495
   Thr Asp Phe His His Thr Val Thr Pro Leu Ser Glu Lys Ile Lys Val
   500 505 510
   Phe Ala Thr Ile Phe Pro Lys Asp Asp Leu Leu Leu Thr Phe Lys Lys
   515 520 525
   Arg Asp Pro Ile Thr Gly Glu Val Phe Glu
   530 535
<210> 23
   <211> 1578
   <212> DNA
   <213> Artificial Sequence
<220>
   <223> Die Beschreibung von Knstliche Sequenz: trunkierte HMG
<220>
   <221> CDS
   <222> (1)..(1578)
<400> 23
   atg gac caa ttg gtg aaa act gaa gtc acc aag aag tct ttt act gct    48
   Met Asp Gln Leu Val Lys Thr Glu Val Thr Lys Lys Ser Phe Thr Ala
    1               5                  10                  15
   cct gta caa aag gct tct aca cca gtt tta acc aat aaa aca gtc att    96
   Pro Val Gln Lys Ala Ser Thr Pro Val Leu Thr Asn Lys Thr Val Ile
               20                  25                  30
   tct gga tcg aaa gtc aaa agt tta tca tct gcg caa tcg agc tca tca    144
   Ser Gly Ser Lys Val Lys Ser Leu Ser Ser Ala Gln Ser Ser Ser Ser
                   35                  40                  45
   gga cct tca tca tct agt gag gaa gat gat tcc cgc gat att gaa agc    192
   Gly Pro Ser Ser Ser Ser Glu Glu Asp Asp Ser Arg Asp Ile Glu Ser
           50                  55                  60
   ttg gat aag aaa ata cgt cct tta gaa gaa tta gaa gca tta tta agt    240
   Leu Asp Lys Lys Ile Arg Pro Leu Glu Glu Leu Glu Ala Leu Leu Ser
   65                  70                  75                  80
   agt gga aat aca aaa caa ttg aag aac aaa gag gtc gct gcc ttg gtt    288
   Ser Gly Asn Thr Lys Gln Leu Lys Asn Lys Glu Val Ala Ala Leu Val
                   85                  90                  95
   att cac ggt aag tta cct ttg tac gct ttg gag aaa aaa tta ggt gat    336
   Ile His Gly Lys Leu Pro Leu Tyr Ala Leu Glu Lys Lys Leu Gly Asp
               100                 105                 110
   act acg aga gcg gtt gcg gta cgt agg aag gct ctt tca att ttg gca    384
   Thr Thr Arg Ala Val Ala Val Arg Arg Lys Ala Leu Ser Ile Leu Ala
           115                 120                 125
   gaa gct cct gta tta gca tct gat cgt tta cca tat aaa aat tat gac    432
   Glu Ala Pro Val Leu Ala Ser Asp Arg Leu Pro Tyr Lys Asn Tyr Asp
       130                 135                 140
   tac gac cgc gta ttt ggc gct tgt tgt gaa aat gtt ata ggt tac atg    480
   Tyr Asp Arg Val Phe Gly Ala Cys Cys Glu Asn Val Ile Gly Tyr Met
   145                 150                 155                 160
   cct ttg ccc gtt ggt gtt ata ggc ccc ttg gtt atc gat ggt aca tct    528
   Pro Leu Pro Val Gly Val Ile Gly Pro Leu Val Ile Asp Gly Thr Ser
                   165                 170                 175
   tat cat ata cca atg gca act aca gag ggt tgt ttg gta gct tct gcc    576
   Tyr His Ile Pro Met Ala Thr Thr Glu Gly Cys Leu Val Ala Ser Ala
               180                 185                 190
   atg cgt ggc tgt aag gca atc aat gct ggc ggt ggt gca aca act gtt    624
   Met Arg Gly Cys Lys Ala Ile Asn Ala Gly Gly Gly Ala Thr Thr Val
           195                 200                 205
   tta act aag gat ggt atg aca aga ggc cca gta gtc cgt ttc cca act    672
   Leu Thr Lys Asp Gly Met Thr Arg Gly Pro Val Val Arg Phe Pro Thr
       210                 215                 220
   ttg aaa aga tct ggt gcc tgt aag ata tgg tta gac tca gaa gag gga    720
   Leu Lys Arg Ser Gly Ala Cys Lys Ile Trp Leu Asp Ser Glu Glu Gly
   225                 230                 235                 240
   caa aac gca att aaa aaa gct ttt aac tct aca tca aga ttt gca cgt    768
   Gln Asn Ala Ile Lys Lys Ala Phe Asn Ser Thr Ser Arg Phe Ala Arg
                   245                 250                 255
   ctg caa cat att caa act tgt cta gca gga gat tta ctc ttc atg aga    816
   Leu Gln His Ile Gln Thr Cys Leu Ala Gly Asp Leu Leu Phe Met Arg
               260                 265                 270
   ttt aga aca act act ggt gac gca atg ggt atg aat atg att tct aaa    864
   Phe Arg Thr Thr Thr Gly Asp Ala Met Gly Met Asn Met Ile Ser Lys
           275                 280                 285
   ggt gtc gaa tac tca tta aag caa atg gta gaa gag tat ggc tgg gaa    912
   Gly Val Glu Tyr Ser Leu Lys Gln Met Val Glu Glu Tyr Gly Trp Glu
       290                 295                 300
   gat atg gag gtt gtc tcc gtt tct ggt aac tac tgt acc gac aaa aaa    960
   Asp Met Glu Val Val Ser Val Ser Gly Asn Tyr Cys Thr Asp Lys Lys
   305                 310                 315                 320
   cca gct gcc atc aac tgg atc gaa ggt cgt ggt aag agt gtc gtc gca    1008
   Pro Ala Ala Ile Asn Trp Ile Glu Gly Arg Gly Lys Ser Val Val Ala
                   325                 330                 335
   gaa gct act att cct ggt gat gtt gtc aga aaa gtg tta aaa agt gat    1056
   Glu Ala Thr Ile Pro Gly Asp Val Val Arg Lys Val Leu Lys Ser Asp
               340                 345                 350
   gtt tcc gca ttg gtt gag ttg aac att gct aag aat ttg gtt gga tct    1104
   Val Ser Ala Leu Val Glu Leu Asn Ile Ala Lys Asn Leu Val Gly Ser
           355                 360                 365
   gca atg gct ggg tct gtt ggt gga ttt aac gca cat gca gct aat tta    1152
   Ala Met Ala Gly Ser Val Gly Gly Phe Asn Ala His Ala Ala Asn Leu
       370                 375                 380
   gtg aca gct gtt ttc ttg gca tta gga caa gat cct gca caa aat gtt    1200
   Val Thr Ala Val Phe Leu Ala Leu Gly Gln Asp Pro Ala Gln Asn Val
   385                 390                 395                 400
   gaa agt tcc aac tgt ata aca ttg atg aaa gaa gtg gac ggt gat ttg    1248
   Glu Ser Ser Asn Cys Ile Thr Leu Met Lys Glu Val Asp Gly Asp Leu
                   405                 410                 415
   aga att tcc gta tcc atg cca tcc atc gaa gta ggt acc atc ggt ggt    1296
   Arg Ile Ser Val Ser Met Pro Ser Ile Glu Val Gly Thr Ile Gly Gly
               420                 425                 430
   ggt act gtt cta gaa cca caa ggt gcc atg ttg gac tta tta ggt gta    1344
   Gly Thr Val Leu Glu Pro Gln Gly Ala Met Leu Asp Leu Leu Gly Val
           435                 440                 445
   aga ggc ccg cat gct acc gct cct ggt acc aac gca cgt caa tta gca    1392
   Arg Gly Pro His Ala Thr Ala Pro Gly Thr Asn Ala Arg Gln Leu Ala
       450                 455                 460
   aga ata gtt gcc tgt gcc gtc ttg gca ggt gaa tta tcc tta tgt gct    1440
   Arg Ile Val Ala Cys Ala Val Leu Ala Gly Glu Leu Ser Leu Cys Ala
   465                 470                 475                 480
   gcc cta gca gcc ggc cat ttg gtt caa agt cat atg acc cac aac agg    1488
   Ala Leu Ala Ala Gly His Leu Val Gln Ser His Met Thr His Asn Arg
                   485                 490                 495
   aaa cct gct gaa cca aca aaa cct aac aat ttg gac gcc act gat ata    1536
   Lys Pro Ala Glu Pro Thr Lys Pro Asn Asn Leu Asp Ala Thr Asp Ile
               500                 505                 510
   aat cgt ttg aaa gat ggg tcc gtc acc tgc att aaa tcc taa            1578
   Asn Arg Leu Lys Asp Gly Ser Val Thr Cys Ile Lys Ser
           515                 520                 525
<210> 24
   <211> 525
   <212> PRT
   <213> Artificial Sequence
<400> 24
   Met Asp Gln Leu Val Lys Thr Glu Val Thr Lys Lys Ser Phe Thr Ala
   1               5                   10                  15
   Pro Val Gln Lys Ala Ser Thr Pro Val Leu Thr Asn Lys Thr Val Ile
               20                  25                  30
   Ser Gly Ser Lys Val Lys Ser Leu Ser Ser Ala Gln Ser Ser Ser Ser
           35                  40                  45
   Gly Pro Ser Ser Ser Ser Glu Glu Asp Asp Ser Arg Asp Ile Glu Ser
       50                  55                  60
   Leu Asp Lys Lys Ile Arg Pro Leu Glu Glu Leu Glu Ala Leu Leu Ser
   65                  70                  75                  80
   Ser Gly Asn Thr Lys Gln Leu Lys Asn Lys Glu Val Ala Ala Leu Val
                   85                  90                  95
   Ile His Gly Lys Leu Pro Leu Tyr Ala Leu Glu Lys Lys Leu Gly Asp
               100                 105                 110
   Thr Thr Arg Ala Val Ala Val Arg Arg Lys Ala Leu Ser Ile Leu Ala
           115                 120                 125
   Glu Ala Pro Val Leu Ala Ser Asp Arg Leu Pro Tyr Lys Asn Tyr Asp
       130                 135                 140
   Tyr Asp Arg Val Phe Gly Ala Cys Cys Glu Asn Val Ile Gly Tyr Met
   145                 150                 155                 160
   Pro Leu Pro Val Gly Val Ile Gly Pro Leu Val Ile Asp Gly Thr Ser
                   165                 170                 175
   Tyr His Ile Pro Met Ala Thr Thr Glu Gly Cys Leu Val Ala Ser Ala
               180                 185                 190
   Met Arg Gly Cys Lys Ala Ile Asn Ala Gly Gly Gly Ala Thr Thr Val
           195                 200                 205
   Leu Thr Lys Asp Gly Met Thr Arg Gly Pro Val Val Arg Phe Pro Thr
       210                 215                 220
   Leu Lys Arg Ser Gly Ala Cys Lys Ile Trp Leu Asp Ser Glu Glu Gly
   225 230 235 240
   Gln Asn Ala Ile Lys Lys Ala Phe Asn Ser Thr Ser Arg Phe Ala Arg
   245 250 255
   Leu Gln His Ile Gln Thr Cys Leu Ala Gly Asp Leu Leu Phe Met Arg
   260 265 270
   Phe Arg Thr Thr Thr Gly Asp Ala Met Gly Met Asn Met Ile Ser Lys
   275 280 285
   Gly Val Glu Tyr Ser Leu Lys Gln Met Val Glu Glu Tyr Gly Trp Glu
   290 295 300
   Asp Met Glu Val Val Ser Val Ser Gly Asn Tyr Cys Thr Asp Lys Lys
   305 310 315 320
   Pro Ala Ala Ile Asn Trp Ile Glu Gly Arg Gly Lys Ser Val Val Ala
   325 330 335
   Glu Ala Thr Ile Pro Gly Asp Val Val Arg Lys Val Leu Lys Ser Asp
   340 345 350
   Val Ser Ala Leu Val Glu Leu Asn Ile Ala Lys Asn Leu Val Gly Ser
   355 360 365
   Ala Met Ala Gly Ser Val Gly Gly Phe Asn Ala His Ala Ala Asn Leu
   370 375 380
   Val Thr Ala Val Phe Leu Ala Leu Gly Gln Asp Pro Ala Gln Asn Val
   385 390 395 400
   Glu Ser Ser Asn Cys Ile Thr Leu Met Lys Glu Val Asp Gly Asp Leu
   405 410 415
   Arg Ile Ser Val Ser Met Pro Ser Ile Glu Val Gly Thr Ile Gly Gly
   420 425 430
   Gly Thr Val Leu Glu Pro Gln Gly Ala Met Leu Asp Leu Leu Gly Val
   435 440 445
   Arg Gly Pro His Ala Thr Ala Pro Gly Thr Asn Ala Arg Gln Leu Ala
   450 455 460
   Arg Ile Val Ala Cys Ala Val Leu Ala Gly Glu Leu Ser Leu Cys Ala
   465 470 475 480
   Ala Leu Ala Ala Gly His Leu Val Gln Ser His Met Thr His Asn Arg
   485 490 495
   Lys Pro Ala Glu Pro Thr Lys Pro Asn Asn Leu Asp Ala Thr Asp Ile
   500 505 510
   Asn Arg Leu Lys Asp Gly Ser Val Thr Cys Ile Lys Ser
   515 520 525
<210> 25
   <211> 1593
   <212> DNA
   <213> Saccharomyces cerevisiae
<220>
   <221> CDS
   <222> (1)..(1593)
<400> 25
   atg tct gct acc aag tca atc gtt gga gag gca ttg gaa tac gta aac    48
   Met Ser Ala Thr Lys Ser Ile Val Gly Glu Ala Leu Glu Tyr Val Asn
     1               5                  10                  15
   att ggt tta agt cat ttc ttg gct tta cca ttg gcc caa aga atc tct    96
   Ile Gly Leu Ser His Phe Leu Ala Leu Pro Leu Ala Gln Arg Ile Ser
               20                  25                  30
   ttg atc ata ata att cct ttc att tac aat att gta tgg caa tta cta   144
   Leu Ile Ile Ile Ile Pro Phe Ile Tyr Asn Ile Val Trp Gln Leu Leu
           35                  40                  45
   tat tct ttg aga aag gac cgt cca cct cta gtg ttt tac tgg att cca   192
   Tyr Ser Leu Arg Lys Asp Arg Pro Pro Leu Val Phe Tyr Trp Ile Pro
       50                  55                  60
   tgg gtc ggt agt gct gtt gtg tac ggt atg aag cca tac gag ttt ttc   240
   Trp Val Gly Ser Ala Val Val Tyr Gly Met Lys Pro Tyr Glu Phe Phe
   65                  70                  75                  80
   gaa gaa tgt caa aag aaa tac ggt gat att ttt tca ttc gtt ttg tta   288
   Glu Glu Cys Gln Lys Lys Tyr Gly Asp Ile Phe Ser Phe Val Leu Leu
                   85                  90                  95
   gga aga gtc atg act gtg tat tta gga cca aag ggt cac gaa ttt gtc   336
   Gly Arg Val Met Thr Val Tyr Leu Gly Pro Lys Gly His Glu Phe Val
               100                 105                 110
   ttc aac gct aag ttg gca gat gtt tca gca gaa gct gct tac gct cat   384
   Phe Asn Ala Lys Leu Ala Asp Val Ser Ala Glu Ala Ala Tyr Ala His
           115                 120                 125
   ttg act act cca gtt ttc ggt aaa ggt gtt att tac gat tgt cca aat   432
   Leu Thr Thr Pro Val Phe Gly Lys Gly Val Ile Tyr Asp Cys Pro Asn
       130                 135                 140
   tct aga ttg atg gag caa aag aag ttt gtt aag ggt gct cta acc aaa   480
   Ser Arg Leu Met Glu Gln Lys Lys Phe Val Lys Gly Ala Leu Thr Lys
   145                 150                 155                 160
   gaa gcc ttc aag agc tac gtt cca ttg att gct gaa gaa gtg tac aag   528
   Glu Ala Phe Lys Ser Tyr Val Pro Leu Ile Ala Glu Glu Val Tyr Lys
                   165                 170                 175
   tac ttc aga gac tcc aaa aac ttc cgt ttg aat gaa aga act act ggt   576
   Tyr Phe Arg Asp Ser Lys Asn Phe Arg Leu Asn Glu Arg Thr Thr Gly
                   180                     185                     190
   act att gac gtg atg gtt act caa cct gaa atg act att ttc acc gct    624
   Thr Ile Asp Val Met Val Thr Gln Pro Glu Met Thr Ile Phe Thr Ala
           195                     200                     205
   tca aga tca tta ttg ggt aag gaa atg aga gca aaa ttg gat acc gat    672
   Ser Arg Ser Leu Leu Gly Lys Glu Met Arg Ala Lys Leu Asp Thr Asp
       210                     215                     220
   ttt gct tac ttg tac agt gat ttg gat aag ggt ttc act cca atc aac    720
   Phe Ala Tyr Leu Tyr Ser Asp Leu Asp Lys Gly Phe Thr Pro Ile Asn
   225                     230                     235                     240
   ttc gtc ttc cct aac tta cca ttg gaa cac tat aga aag aga gat cac    768
   Phe Val Phe Pro Asn Leu Pro Leu Glu His Tyr Arg Lys Arg Asp His
                   245                     250                     255
   gct caa aag gct atc tcc ggt act tac atg tct ttg att aag gaa aga    816
   Ala Gln Lys Ala Ile Ser Gly Thr Tyr Met Ser Leu Ile Lys Glu Arg
               260                     265                     270
   aga aag aac aac gac att caa gac aga gat ttg atc gat tcc ttg atg    864
   Arg Lys Asn Asn Asp Ile Gln Asp Arg Asp Leu Ile Asp Ser Leu Met
           275                     280                     285
   aag aac tct acc tac aag gat ggt gtg aag atg act gat caa gaa atc    912
   Lys Asn Ser Thr Tyr Lys Asp Gly Val Lys Met Thr Asp Gln Glu Ile
       290                     295                     300
   gct aac ttg tta att ggt gtc tta atg ggt ggt caa cat act tct gct    960
   Ala Asn Leu Leu Ile Gly Val Leu Met Gly Gly Gln His Thr Ser Ala
   305                     310                     315                     320
   gcc act tct gct tgg att ttg ttg cac ttg gct gaa aga cca gat gtc   1008
   Ala Thr Ser Ala Trp Ile Leu Leu His Leu Ala Glu Arg Pro Asp Val
                   325                     330                     335
   caa caa gaa ttg tac gaa gaa caa atg cgt gtt ttg gat ggt ggt aag   1056
   Gln Gln Glu Leu Tyr Glu Glu Gln Met Arg Val Leu Asp Gly Gly Lys
               340                     345                     350
   aag gaa ttg acc tac gat tta tta caa gaa atg cca ttg ttg aac caa   1104
   Lys Glu Leu Thr Tyr Asp Leu Leu Gln Glu Met Pro Leu Leu Asn Gln
           355                     360                     365
   act att aag gaa act cta aga atg cac cat cca ttg cac tct ttg ttc   1152
   Thr Ile Lys Glu Thr Leu Arg Met His His Pro Leu His Ser Leu Phe
       370                     375                     380
   cgt aag gtt atg aaa gat atg cac gtt cca aac act tct tat gtc atc   1200
   Arg Lys Val Met Lys Asp Met His Val Pro Asn Thr Ser Tyr Val Ile
   385                     390                     395                     400
   cca gca ggt tat cac gtt ttg gtt tct cca ggt tac act cat tta aga   1248
   Pro Ala Gly Tyr His Val Leu Val Ser Pro Gly Tyr Thr His Leu Arg
                   405                     410                     415
   gac gaa tac ttc cct aat gct cac caa ttc aac att cac cgt tgg aac    1296
   Asp Glu Tyr Phe Pro Asn Ala His Gln Phe Asn Ile His Arg Trp Asn
               420                 425                 430
   aaa gat tct gcc tcc tct tat tcc gtc ggt gaa gaa gtc gat tac ggt    1344
   Lys Asp Ser Ala Ser Ser Tyr Ser Val Gly Glu Glu Val Asp Tyr Gly
           435                 440                 445
   ttc ggt gcc att tct aag ggt gtc agc tct cca tac tta cct ttc ggt    1392
   Phe Gly Ala Ile Ser Lys Gly Val Ser Ser Pro Tyr Leu Pro Phe Gly
       450                 455                 460
   ggt ggt aga cac aga tgt atc ggt gaa cac ttt gct tac tgt cag cta    1440
   Gly Gly Arg His Arg Cys Ile Gly Glu His Phe Ala Tyr Cys Gln Leu
   465                 470                 475                 480
   ggt gtt cta atg tcc att ttt atc aga aca tta aaa tgg cat tac cca    1488
   Gly Val Leu Met Ser Ile Phe Ile Arg Thr Leu Lys Trp His Tyr Pro
                   485                 490                 495
   gag ggt aag acc gtt cca cct cct gac ttt aca tct atg gtt act ctt    1536
   Glu Gly Lys Thr Val Pro Pro Pro Asp Phe Thr Ser Met Val Thr Leu
               500                 505                 510
   cca acc ggt cca gcc aag atc atc tgg gaa aag aga aat cca gaa caa    1584
   Pro Thr Gly Pro Ala Lys Ile Ile Trp Glu Lys Arg Asn Pro Glu Gln
           515                 520                 525
   aag atc taa                                                        1593
   Lys Ile
       530
<210> 26
   <211> 530
   <212> PRT
   <213> Saccharomyces cerevisiae
<400> 26
   Met Ser Ala Thr Lys Ser Ile Val Gly Glu Ala Leu Glu Tyr Val Asn
   1               5                   10                  15
   Ile Gly Leu Ser His Phe Leu Ala Leu Pro Leu Ala Gln Arg Ile Ser
               20                  25                  30
   Leu Ile Ile Ile Ile Pro Phe Ile Tyr Asn Ile Val Trp Gln Leu Leu
           35                  40                  45
   Tyr Ser Leu Arg Lys Asp Arg Pro Pro Leu Val Phe Tyr Trp Ile Pro
       50                  55                  60
   Trp Val Gly Ser Ala Val Val Tyr Gly Met Lys Pro Tyr Glu Phe Phe
   65                  70                  75                  80
   Glu Glu Cys Gln Lys Lys Tyr Gly Asp Ile Phe Ser Phe Val Leu Leu
                   85                  90                  95
   Gly Arg Val Met Thr Val Tyr Leu Gly Pro Lys Gly His Glu Phe Val
   100 105 110
   Phe Asn Ala Lys Leu Ala Asp Val Ser Ala Glu Ala Ala Tyr Ala His
   115 120 125
   Leu Thr Thr Pro Val Phe Gly Lys Gly Val Ile Tyr Asp Cys Pro Asn
   130 135 140
   Ser Arg Leu Met Glu Gln Lys Lys Phe Val Lys Gly Ala Leu Thr Lys
   145 150 155 160
   Glu Ala Phe Lys Ser Tyr Val Pro Leu Ile Ala Glu Glu Val Tyr Lys
   165 170 175
   Tyr Phe Arg Asp Ser Lys Asn Phe Arg Leu Asn Glu Arg Thr Thr Gly
   180 185 190
   Thr Ile Asp Val Met Val Thr Gln Pro Glu Met Thr Ile Phe Thr Ala
   195 200 205
   Ser Arg Ser Leu Leu Gly Lys Glu Met Arg Ala Lys Leu Asp Thr Asp
   210 215 220
   Phe Ala Tyr Leu Tyr Ser Asp Leu Asp Lys Gly Phe Thr Pro Ile Asn
   225 230 235 240
   Phe Val Phe Pro Asn Leu Pro Leu Glu His Tyr Arg Lys Arg Asp His
   245 250 255
   Ala Gln Lys Ala Ile Ser Gly Thr Tyr Met Ser Leu Ile Lys Glu Arg
   260 265 270
   Arg Lys Asn Asn Asp Ile Gln Asp Arg Asp Leu Ile Asp Ser Leu Met
   275 280 285
   Lys Asn Ser Thr Tyr Lys Asp Gly Val Lys Met Thr Asp Gln Glu Ile
   290 295 300
   Ala Asn Leu Leu Ile Gly Val Leu Met Gly Gly Gln His Thr Ser Ala
   305 310 315 320
   Ala Thr Ser Ala Trp Ile Leu Leu His Leu Ala Glu Arg Pro Asp Val
   325 330 335
   Gln Gln Glu Leu Tyr Glu Glu Gln Met Arg Val Leu Asp Gly Gly Lys
   340 345 350
   Lys Glu Leu Thr Tyr Asp Leu Leu Gln Glu Met Pro Leu Leu Asn Gln
   355 360 365
   Thr Ile Lys Glu Thr Leu Arg Met His His Pro Leu His Ser Leu Phe
   370 375 380
   Arg Lys Val Met Lys Asp Met His Val Pro Asn Thr Ser Tyr Val Ile
   385 390 395 400
   Pro Ala Gly Tyr His Val Leu Val Ser Pro Gly Tyr Thr His Leu Arg
                   405                     410                     415
   Asp Glu Tyr Phe Pro Asn Ala His Gln Phe Asn Ile His Arg Trp Asn
               420                     425                     430
   Lys Asp Ser Ala Ser Ser Tyr Ser Val Gly Glu Glu Val Asp Tyr Gly
           435                     440                     445
   Phe Gly Ala Ile Ser Lys Gly Val Ser Ser Pro Tyr Leu Pro Phe Gly
       450                     455                     460
   Gly Gly Arg His Arg Cys Ile Gly Glu His Phe Ala Tyr Cys Gln Leu
   465                     470                     475                     480
   Gly Val Leu Met Ser Ile Phe Ile Arg Thr Leu Lys Trp His Tyr Pro
                   485                     490                     495
   Glu Gly Lys Thr Val Pro Pro Pro Asp Phe Thr Ser Met Val Thr Leu
               500                     505                     510
   Pro Thr Gly Pro Ala Lys Ile Ile Trp Glu Lys Arg Asn Pro Glu Gln
           515                     520                     525
   Lys Ile
       530
<210> 27
   <211> 1491
   <212> DNA
   <213> Saccharomyces cerevisiae
<220>
   <221> CDS
   <222> (1)..(1491)
<400> 27
   atg tct gct gtt aac gtt gca cct gaa ttg att aat gcc gac aac aca    48
   Met Ser Ala Val Asn Val Ala Pro Glu Leu Ile Asn Ala Asp Asn Thr
    1               5                  10                  15
   att acc tac gat gcg att gtc atc ggt gct ggt gtt atc ggt cca tgt    96
   Ile Thr Tyr Asp Ala Ile Val Ile Gly Ala Gly Val Ile Gly Pro Cys
               20                  25                  30
   gtt gct act ggt cta gca aga aag ggt aag aaa gtt ctt atc gta gaa    144
   Val Ala Thr Gly Leu Ala Arg Lys Gly Lys Lys Val Leu Ile Val Glu
           35                  40                  45
   cgt gac tgg gct atg cct gat aga att gtt ggt gaa ttg atg caa cca    192
   Arg Asp Trp Ala Met Pro Asp Arg Ile Val Gly Glu Leu Met Gln Pro
       50                  55                  60
   ggt ggt gtt aga gca ttg aga agt ctg ggt atg att caa tct atc aac    240
   Gly Gly Val Arg Ala Leu Arg Ser Leu Gly Met Ile Gln Ser Ile Asn
    65                   70                   75                   80
   aac atc gaa gca tat cct gtt acc ggt tat acc gtc ttt ttc aac ggc    288
   Asn Ile Glu Ala Tyr Pro Val Thr Gly Tyr Thr Val Phe Phe Asn Gly
                    85                  90                  95
   gaa caa gtt gat att cca tac cct tac aag gcc gat atc cct aaa gtt    336
   Glu Gln Val Asp Ile Pro Tyr Pro Tyr Lys Ala Asp Ile Pro Lys Val
               100                 105                 110
   gaa aaa ttg aag gac ttg gtc aaa gat ggt aat gac aag gtc ttg gaa    384
   Glu Lys Leu Lys Asp Leu Val Lys Asp Gly Asn Asp Lys Val Leu Glu
           115                 120                 125
   gac agc act att cac atc aag gat tac gaa gat gat gaa aga gaa agg    432
   Asp Ser Thr Ile His Ile Lys Asp Tyr Glu Asp Asp Glu Arg Glu Arg
       130                 135                 140
   ggt gtt gct ttt gtt cat ggt aga ttc ttg aac aac ttg aga aac att    480
   Gly Val Ala Phe Val His Gly Arg Phe Leu Asn Asn Leu Arg Asn Ile
   145                 150                 155                 160
   act gct caa gag cca aat gtt act aga gtg caa ggt aac tgt att gag    528
   Thr Ala Gln Glu Pro Asn Val Thr Arg Val Gln Gly Asn Cys Ile Glu
                   165                 170                 175
   ata ttg aag gat gaa aag aat gag gtt gtt ggt gcc aag gtt gac att    576
   Ile Leu Lys Asp Glu Lys Asn Glu Val Val Gly Ala Lys Val Asp Ile
               180                 185                 190
   gat ggc cgt ggc aag gtg gaa ttc aaa gcc cac ttg aca ttt atc tgt    624
   Asp Gly Arg Gly Lys Val Glu Phe Lys Ala His Leu Thr Phe Ile Cys
           195                 200                 205
   gac ggt atc ttt tca cgt ttc aga aag gaa ttg cac cca gac cat gtt    672
   Asp Gly Ile Phe Ser Arg Phe Arg Lys Glu Leu His Pro Asp His Val
       210                 215                 220
   cca act gtc ggt tct tcg ttt gtc ggt atg tct ttg ttc aat gct aag    720
   Pro Thr Val Gly Ser Ser Phe Val Gly Met Ser Leu Phe Asn Ala Lys
   225                 230                 235                 240
   aat cct gct cct atg cac ggt cac gtt att ctt ggt agt gat cat atg    768
   Asn Pro Ala Pro Met His Gly His Val Ile Leu Gly Ser Asp His Met
                   245                 250                 255
   cca atc ttg gtt tac caa atc agt cca gaa gaa aca aga atc ctt tgt    816
   Pro Ile Leu Val Tyr Gln Ile Ser Pro Glu Glu Thr Arg Ile Leu Cys
               260                 265                 270
   gct tac aac tct cca aag gtc cca gct gat atc aag agt tgg atg att    864
   Ala Tyr Asn Ser Pro Lys Val Pro Ala Asp Ile Lys Ser Trp Met Ile
           275                 280                 285
   aag gat gtc caa cct ttc att cca aag agt cta cgt cct tca ttt gat    912
   Lys Asp Val Gln Pro Phe Ile Pro Lys Ser Leu Arg Pro Ser Phe Asp
       290                 295                 300
   gaa gcc gtc agc caa ggt aaa ttt aga gct atg cca aac tcc tac ttg    960
   Glu Ala Val Ser Gln Gly Lys Phe Arg Ala Met Pro Asn Ser Tyr Leu
   305                 310                 315                 320
   cca gct aga caa aac gac gtc act ggt atg tgt gtt atc ggt gac gct    1008
   Pro Ala Arg Gln Asn Asp Val Thr Gly Met Cys Val Ile Gly Asp Ala
                   325                 330                 335
   cta aat atg aga cat cca ttg act ggt ggt ggt atg act gtc ggt ttg    1056
   Leu Asn Met Arg His Pro Leu Thr Gly Gly Gly Met Thr Val Gly Leu
               340                 345                 350
   cat gat gtt gtc ttg ttg att aag aaa ata ggt gac cta gac ttc agc    1104
   His Asp Val Val Leu Leu Ile Lys Lys Ile Gly Asp Leu Asp Phe Ser
           355                 360                 365
   gac cgt gaa aag gtt ttg gat gaa tta cta gac tac cat ttc gaa aga    1152
   Asp Arg Glu Lys Val Leu Asp Glu Leu Leu Asp Tyr His Phe Glu Arg
       370                 375                 380
   aag agt tac gat tcc gtt att aac gtt ttg tca gtg gct ttg tat tct    1200
   Lys Ser Tyr Asp Ser Val Ile Asn Val Leu Ser Val Ala Leu Tyr Ser
   385                 390                 395                 400
   ttg ttc gct gct gac agc gat aac ttg aag gca tta caa aaa ggt tgt    1248
   Leu Phe Ala Ala Asp Ser Asp Asn Leu Lys Ala Leu Gln Lys Gly Cys
                   405                 410                 415
   ttc aaa tat ttc caa aga ggt ggc gat tgt gtc aac aaa ccc gtt gaa    1296
   Phe Lys Tyr Phe Gln Arg Gly Gly Asp Cys Val Asn Lys Pro Val Glu
               420                 425                 430
   ttt ctg tct ggt gtc ttg cca aag cct ttg caa ttg acc agg gtt ttc    1344
   Phe Leu Ser Gly Val Leu Pro Lys Pro Leu Gln Leu Thr Arg Val Phe
           435                 440                 445
   ttc gct gtc gct ttt tac acc att tac ttg aac atg gaa gaa cgt ggt    1392
   Phe Ala Val Ala Phe Tyr Thr Ile Tyr Leu Asn Met Glu Glu Arg Gly
       450                 455                 460
   ttc ttg gga tta cca atg gct tta ttg gaa ggt att atg att ttg atc    1440
   Phe Leu Gly Leu Pro Met Ala Leu Leu Glu Gly Ile Met Ile Leu Ile
   465                 470                 475                 480
   aca gct att aga gta ttc acc cca ttt ttg ttt ggt gag ttg att ggt    1488
   Thr Ala Ile Arg Val Phe Thr Pro Phe Leu Phe Gly Glu Leu Ile Gly
                   485                 490                 495
   taa                                                                1491
<210> 28
   <211> 496
   <212> PRT
   <213> Saccharomyces cerevisiae
<400> 28
   Met Ser Ala Val Asn Val Ala Pro Glu Leu Ile Asn Ala Asp Asn Thr
   1 5 10 15
   Ile Thr Tyr Asp Ala Ile Val Ile Gly Ala Gly Val Ile Gly Pro Cys
   20 25 30
   Val Ala Thr Gly Leu Ala Arg Lys Gly Lys Lys Val Leu Ile Val Glu
   35 40 45
   Arg Asp Trp Ala Met Pro Asp Arg Ile Val Gly Glu Leu Met Gln Pro
   50 55 60
   Gly Gly Val Arg Ala Leu Arg Ser Leu Gly Met Ile Gln Ser Ile Asn
   65 70 75 80
   Asn Ile Glu Ala Tyr Pro Val Thr Gly Tyr Thr Val Phe Phe Asn Gly
   85 90 95
   Glu Gln Val Asp Ile Pro Tyr Pro Tyr Lys Ala Asp Ile Pro Lys Val
   100 105 110
   Glu Lys Leu Lys Asp Leu Val Lys Asp Gly Asn Asp Lys Val Leu Glu
   115 120 125
   Asp Ser Thr Ile His Ile Lys Asp Tyr Glu Asp Asp Glu Arg Glu Arg
   130 135 140
   Gly Val Ala Phe Val His Gly Arg Phe Leu Asn Asn Leu Arg Asn Ile
   145 150 155 160
   Thr Ala Gln Glu Pro Asn Val Thr Arg Val Gln Gly Asn Cys Ile Glu
   165 170 175
   Ile Leu Lys Asp Glu Lys Asn Glu Val Val Gly Ala Lys Val Asp Ile
   180 185 190
   Asp Gly Arg Gly Lys Val Glu Phe Lys Ala His Leu Thr Phe Ile Cys
   195 200 205
   Asp Gly Ile Phe Ser Arg Phe Arg Lys Glu Leu His Pro Asp His Val
   210 215 220
   Pro Thr Val Gly Ser Ser Phe Val Gly Met Ser Leu Phe Asn Ala Lys
   225 230 235 240
   Asn Pro Ala Pro Met His Gly His Val Ile Leu Gly Ser Asp His Met
   245 250 255
   Pro Ile Leu Val Tyr Gln Ile Ser Pro Glu Glu Thr Arg Ile Leu Cys
   260 265 270
   Ala Tyr Asn Ser Pro Lys Val Pro Ala Asp Ile Lys Ser Trp Met Ile
   275 280 285
   Lys Asp Val Gln Pro Phe Ile Pro Lys Ser Leu Arg Pro Ser Phe Asp
           290                 295                 300
   Glu Ala Val Ser Gln Gly Lys Phe Arg Ala Met Pro Asn Ser Tyr Leu
   305                 310                 315                 320
   Pro Ala Arg Gln Asn Asp Val Thr Gly Met Cys Val Ile Gly Asp Ala
                   325                 330                 335
   Leu Asn Met Arg His Pro Leu Thr Gly Gly Gly Met Thr Val Gly Leu
               340                 345                 350
   His Asp Val Val Leu Leu Ile Lys Lys Ile Gly Asp Leu Asp Phe Ser
           355                 360                 365
   Asp Arg Glu Lys Val Leu Asp Glu Leu Leu Asp Tyr His Phe Glu Arg
       370                 375                 380
   Lys Ser Tyr Asp Ser Val Ile Asn Val Leu Ser Val Ala Leu Tyr Ser
   385                 390                 395                 400
   Leu Phe Ala Ala Asp Ser Asp Asn Leu Lys Ala Leu Gln Lys Gly Cys
                   405                 410                 415
   Phe Lys Tyr Phe Gln Arg Gly Gly Asp Cys Val Asn Lys Pro Val Glu
               420                 425                 430
   Phe Leu Ser Gly Val Leu Pro Lys Pro Leu Gln Leu Thr Arg Val Phe
           435                 440                 445
   Phe Ala Val Ala Phe Tyr Thr Ile Tyr Leu Asn Met Glu Glu Arg Gly
       450                 455                 460
   Phe Leu Gly Leu Pro Met Ala Leu Leu Glu Gly Ile Met Ile Leu Ile
   465                 470                 475                 480
   Thr Ala Ile Arg Val Phe Thr Pro Phe Leu Phe Gly Glu Leu Ile Gly
                   485                 490                 495
<210> 29
   <211> 1335
   <212> DNA
   <213> Saccharomyces cerevisiae
<220>
   <221> CDS
   <222> (1)..(1335)
<400> 29
   atg gga aag cta tta caa ttg gca ttg cat ccg gtc gag atg aag gca    48
   Met Gly Lys Leu Leu Gln Leu Ala Leu His Pro Val Glu Met Lys Ala
    1               5                  10                  15
   gct ttg aag ctg aag ttt tgc aga aca ccg cta ttc tcc atc tat gat    96
   Ala Leu Lys Leu Lys Phe Cys Arg Thr Pro Leu Phe Ser Ile Tyr Asp
               20                  25                  30
   cag tcc acg tct cca tat ctc ttg cac tgt ttc gaa ctg ttg aac ttg    144
   Gln Ser Thr Ser Pro Tyr Leu Leu His Cys Phe Glu Leu Leu Asn Leu
           35                  40                  45
   acc tcc aga tcg ttt gct gct gtg atc aga gag ctg cat cca gaa ttg    192
   Thr Ser Arg Ser Phe Ala Ala Val Ile Arg Glu Leu His Pro Glu Leu
       50                  55                  60
   aga aac tgt gtt act ctc ttt tat ttg att tta agg gct ttg gat acc    240
   Arg Asn Cys Val Thr Leu Phe Tyr Leu Ile Leu Arg Ala Leu Asp Thr
   65                  70                  75                  80
   atc gaa gac gat atg tcc atc gaa cac gat ttg aaa att gac ttg ttg    288
   Ile Glu Asp Asp Met Ser Ile Glu His Asp Leu Lys Ile Asp Leu Leu
                   85                  90                  95
   cgt cac ttc cac gag aaa ttg ttg tta act aaa tgg agt ttc gac gga    336
   Arg His Phe His Glu Lys Leu Leu Leu Thr Lys Trp Ser Phe Asp Gly
               100                 105                 110
   aat gcc ccc gat gtg aag gac aga gcc gtt ttg aca gat ttc gaa tcg    384
   Asn Ala Pro Asp Val Lys Asp Arg Ala Val Leu Thr Asp Phe Glu Ser
           115                 120                 125
   att ctt att gaa ttc cac aaa ttg aaa cca gaa tat caa gaa gtc atc    432
   Ile Leu Ile Glu Phe His Lys Leu Lys Pro Glu Tyr Gln Glu Val Ile
       130                 135                 140
   aag gag atc acc gag aaa atg ggt aat ggt atg gcc gac tac atc tta    480
   Lys Glu Ile Thr Glu Lys Met Gly Asn Gly Met Ala Asp Tyr Ile Leu
   145                 150                 155                 160
   gat gaa aat tac aac ttg aat ggg ttg caa acc gtc cac gac tac gac    528
   Asp Glu Asn Tyr Asn Leu Asn Gly Leu Gln Thr Val His Asp Tyr Asp
                   165                 170                 175
   gtg tac tgt cac tac gta gct ggt ttg gtc ggt gat ggt ttg acc cgt    576
   Val Tyr Cys His Tyr Val Ala Gly Leu Val Gly Asp Gly Leu Thr Arg
               180                 185                 190
   ttg att gtc att gcc aag ttt gcc aac gaa tct ttg tat tct aat gag    624
   Leu Ile Val Ile Ala Lys Phe Ala Asn Glu Ser Leu Tyr Ser Asn Glu
           195                 200                 205
   caa ttg tat gaa agc atg ggt ctt ttc cta caa aaa acc aac atc atc    672
   Gln Leu Tyr Glu Ser Met Gly Leu Phe Leu Gln Lys Thr Asn Ile Ile
       210                 215                 220
   aga gat tac aat gaa gat ttg gtc gat ggt aga tcc ttc tgg ccc aag    720
   Arg Asp Tyr Asn Glu Asp Leu Val Asp Gly Arg Ser Phe Trp Pro Lys
   225                 230                 235                 240
   gaa atc tgg tca caa tac gct cct cag ttg aag gac ttc atg aaa cct    768
   Glu Ile Trp Ser Gln Tyr Ala Pro Gln Leu Lys Asp Phe Met Lys Pro
                   245                 250                 255
   gaa aac gaa caa ctg ggg ttg gac tgt ata aac cac ctc gtc tta aac    816
   Glu Asn Glu Gln Leu Gly Leu Asp Cys Ile Asn His Leu Val Leu Asn
               260                 265                 270
   gca ttg agt cat gtt atc gat gtg ttg act tat ttg gcc ggt atc cac     864
   Ala Leu Ser His Val Ile Asp Val Leu Thr Tyr Leu Ala Gly Ile His
           275                 280                 285
   gag caa tcc act ttc caa ttt tgt gcc att ccc caa gtt atg gcc att     912
   Glu Gln Ser Thr Phe Gln Phe Cys Ala Ile Pro Gln Val Met Ala Ile
       290                 295                 300
   gca acc ttg gct ttg gta ttc aac aac cgt gaa gtg cta cat ggc aat     960
   Ala Thr Leu Ala Leu Val Phe Asn Asn Arg Glu Val Leu His Gly Asn
   305                 310                 315                 320
   gta aag att cgt aag ggt act acc tgc tat tta att ttg aaa tca agg    1008
   Val Lys Ile Arg Lys Gly Thr Thr Cys Tyr Leu Ile Leu Lys Ser Arg
                   325                 330                 335
   act ttg cgt ggc tgt gtc gag att ttt gac tat tac tta cgt gat atc    1056
   Thr Leu Arg Gly Cys Val Glu Ile Phe Asp Tyr Tyr Leu Arg Asp Ile
               340                 345                 350
   aaa tct aaa ttg gct gtg caa gat cca aat ttc tta aaa ttg aac att    1104
   Lys Ser Lys Leu Ala Val Gln Asp Pro Asn Phe Leu Lys Leu Asn Ile
           355                 360                 365
   caa atc tcc aag atc gaa cag ttt atg gaa gaa atg tac cag gat aaa    1152
   Gln Ile Ser Lys Ile Glu Gln Phe Met Glu Glu Met Tyr Gln Asp Lys
       370                 375                 380
   tta cct cct aac gtg aag cca aat gaa act cca att ttc ttg aaa gtt    1200
   Leu Pro Pro Asn Val Lys Pro Asn Glu Thr Pro Ile Phe Leu Lys Val
   385                 390                 395                 400
   aaa gaa aga tcc aga tac gat gat gaa ttg gtt cca acc caa caa gaa    1248
   Lys Glu Arg Ser Arg Tyr Asp Asp Glu Leu Val Pro Thr Gln Gln Glu
                   405                 410                 415
   gaa gag tac aag ttc aat atg gtt tta tct atc atc ttg tcc gtt ctt    1296
   Glu Glu Tyr Lys Phe Asn Met Val Leu Ser Ile Ile Leu Ser Val Leu
               420                 425                 430
   ctt ggg ttt tat tat ata tac act tta cac aga gcg tga                1335
   Leu Gly Phe Tyr Tyr Ile Tyr Thr Leu His Arg Ala
           435                 440                 445
<210> 30
   <211> 444
   <212> PRT
   <213> Saccharomyces cerevisiae
<400> 30
   Met Gly Lys Leu Leu Gln Leu Ala Leu His Pro Val Glu Met Lys Ala
   1               5                   10                  15
   Ala Leu Lys Leu Lys Phe Cys Arg Thr Pro Leu Phe Ser Ile Tyr Asp
               20                  25                  30
   Gln Ser Thr Ser Pro Tyr Leu Leu His Cys Phe Glu Leu Leu Asn Leu
           35                  40                  45
   Thr Ser Arg Ser Phe Ala Ala Val Ile Arg Glu Leu His Pro Glu Leu
       50                  55                  60
   Arg Asn Cys Val Thr Leu Phe Tyr Leu Ile Leu Arg Ala Leu Asp Thr
   65                  70                  75                  80
   Ile Glu Asp Asp Met Ser Ile Glu His Asp Leu Lys Ile Asp Leu Leu
                   85                  90                  95
   Arg His Phe His Glu Lys Leu Leu Leu Thr Lys Trp Ser Phe Asp Gly
               100                 105                 110
   Asn Ala Pro Asp Val Lys Asp Arg Ala Val Leu Thr Asp Phe Glu Ser
           115                 120                 125
   Ile Leu Ile Glu Phe His Lys Leu Lys Pro Glu Tyr Gln Glu Val Ile
       130                 135                 140
   Lys Glu Ile Thr Glu Lys Met Gly Asn Gly Met Ala Asp Tyr Ile Leu
   145                 150                 155                 160
   Asp Glu Asn Tyr Asn Leu Asn Gly Leu Gln Thr Val His Asp Tyr Asp
                   165                 170                 175
   Val Tyr Cys His Tyr Val Ala Gly Leu Val Gly Asp Gly Leu Thr Arg
               180                 185                 190
   Leu Ile Val Ile Ala Lys Phe Ala Asn Glu Ser Leu Tyr Ser Asn Glu
           195                 200                 205
   Gln Leu Tyr Glu Ser Met Gly Leu Phe Leu Gln Lys Thr Asn Ile Ile
       210                 215                 220
   Arg Asp Tyr Asn Glu Asp Leu Val Asp Gly Arg Ser Phe Trp Pro Lys
   225                 230                 235                 240
   Glu Ile Trp Ser Gln Tyr Ala Pro Gln Leu Lys Asp Phe Met Lys Pro
                   245                 250                 255
   Glu Asn Glu Gln Leu Gly Leu Asp Cys Ile Asn His Leu Val Leu Asn
               260                 265                 270
   Ala Leu Ser His Val Ile Asp Val Leu Thr Tyr Leu Ala Gly Ile His
           275                 280                 285
   Glu Gln Ser Thr Phe Gln Phe Cys Ala Ile Pro Gln Val Met Ala Ile
       290                 295                 300
   Ala Thr Leu Ala Leu Val Phe Asn Asn Arg Glu Val Leu His Gly Asn
   305                 310                 315                 320
   Val Lys Ile Arg Lys Gly Thr Thr Cys Tyr Leu Ile Leu Lys Ser Arg
   325 330 335
   Thr Leu Arg Gly Cys Val Glu Ile Phe Asp Tyr Tyr Leu Arg Asp Ile
   340 345 350
   Lys Ser Lys Leu Ala Val Gln Asp Pro Asn Phe Leu Lys Leu Asn Ile
   355 360 365
   Gln Ile Ser Lys Ile Glu Gln Phe Met Glu Glu Met Tyr Gln Asp Lys
   370 375 380
   Leu Pro Pro Asn Val Lys Pro Asn Glu Thr Pro Ile Phe Leu Lys Val
   385 390 395 400
   Lys Glu Arg Ser Arg Tyr Asp Asp Glu Leu Val Pro Thr Gln Gln Glu
   405 410 415
   Glu Glu Tyr Lys Phe Asn Met Val Leu Ser Ile Ile Leu Ser Val Leu
   420 425 430
   Leu Gly Phe Tyr Tyr Ile Tyr Thr Leu His Arg Ala
   435 440
<210> 31
   <211> 1929
   <212> DNA
   <213> Saccharomyces cerevisiae
<220>
   <221> CDS
   <222> (1)..(1929)
<400> 31
   atg gac aag aag aag gat cta ctg gag aac gaa caa ttt ctc cgc atc   48
   Met Asp Lys Lys Lys Asp Leu Leu Glu Asn Glu Gln Phe Leu Arg Ile
    1               5                  10                  15
   caa aag ctc aac gct gcc gat gcg ggc aaa aga caa tct ata aca gtg   96
   Gln Lys Leu Asn Ala Ala Asp Ala Gly Lys Arg Gln Ser Ile Thr Val
               20                  25                  30
   gac gac gag ggc gaa cta tat ggg tta gac acc tcc ggc aac tca cca   144
   Asp Asp Glu Gly Glu Leu Tyr Gly Leu Asp Thr Ser Gly Asn Ser Pro
           35                  40                  45
   gcc aat gaa cac aca gct acc aca att aca cag aat cac agc gtg gtg   192
   Ala Asn Glu His Thr Ala Thr Thr Ile Thr Gln Asn His Ser Val Val
       50                  55                  60
   gcc tca aac gga gac gtc gca ttc atc cca gga act gct acc gaa ggc   240
   Ala Ser Asn Gly Asp Val Ala Phe Ile Pro Gly Thr Ala Thr Glu Gly
   65                  70                  75                  80
   aat aca gag att gta act gaa gaa gtg att gag acc gat gat aac atg   288
   Asn Thr Glu Ile Val Thr Glu Glu Val Ile Glu Thr Asp Asp Asn Met
                   85                  90                  95
   ttc aag acc cat gtg aag act tta agc tcc aaa gag aag gca cgg tat      336
   Phe Lys Thr His Val Lys Thr Leu Ser Ser Lys Glu Lys Ala Arg Tyr
               100                 105                 110
   agg caa ggg tcc tct aac ttt ata tcg tat ttc gat gat atg tca ttt      384
   Arg Gln Gly Ser Ser Asn Phe Ile Ser Tyr Phe Asp Asp Met Ser Phe
           115                 120                 125
   gaa cac agg ccc agt ata tta gat ggg tca gtt aac gag ccc ttc aag      432
   Glu His Arg Pro Ser Ile Leu Asp Gly Ser Val Asn Glu Pro Phe Lys
       130                 135                 140
   acc aaa ttc gtg gga cct act tta gaa aag gag atc aga aga agg gag      480
   Thr Lys Phe Val Gly Pro Thr Leu Glu Lys Glu Ile Arg Arg Arg Glu
   145                 150                 155                 160
   aaa gag cta atg gcc atg cgc aaa aat tta cac cac cgc aag tcc tcc      528
   Lys Glu Leu Met Ala Met Arg Lys Asn Leu His His Arg Lys Ser Ser
                   165                 170                 175
   cca gat gct gtc gac tca gta ggg aaa aat gat ggc gcc gcc cca act      576
   Pro Asp Ala Val Asp Ser Val Gly Lys Asn Asp Gly Ala Ala Pro Thr
               180                 185                 190
   act gtt cca act gcc gcc acc tca gaa acg gtg gtc acc gtt gaa acc      624
   Thr Val Pro Thr Ala Ala Thr Ser Glu Thr Val Val Thr Val Glu Thr
           195                 200                 205
   acc ata att tca tcc aat ttc tcc ggg ttg tac gtg gcg ttt tgg atg      672
   Thr Ile Ile Ser Ser Asn Phe Ser Gly Leu Tyr Val Ala Phe Trp Met
       210                 215                 220
   gct att gca ttt ggt gct gtc aag gct tta ata gac tat tat tac cag      720
   Ala Ile Ala Phe Gly Ala Val Lys Ala Leu Ile Asp Tyr Tyr Tyr Gln
   225                 230                 235                 240
   cat aat ggt agc ttc aag gat tcg gag atc ttg aaa ttt atg act acg      768
   His Asn Gly Ser Phe Lys Asp Ser Glu Ile Leu Lys Phe Met Thr Thr
                   245                 250                 255
   aat ttg ttc act gtg gca tcc gta gat ctt ttg atg tat ttg agc act      816
   Asn Leu Phe Thr Val Ala Ser Val Asp Leu Leu Met Tyr Leu Ser Thr
               260                 265                 270
   tat ttt gtc gtt gga ata caa tac tta tgc aag tgg ggg gtc ttg aaa      864
   Tyr Phe Val Val Gly Ile Gln Tyr Leu Cys Lys Trp Gly Val Leu Lys
           275                 280                 285
   tgg ggc act acc ggc tgg atc ttc acc tca att tac gag ttt ttg ttt      912
   Trp Gly Thr Thr Gly Trp Ile Phe Thr Ser Ile Tyr Glu Phe Leu Phe
       290                 295                 300
   gtt atc ttc tac atg tat tta aca gaa aac atc cta aaa cta cac tgg      960
   Val Ile Phe Tyr Met Tyr Leu Thr Glu Asn Ile Leu Lys Leu His Trp
   305                 310                 315                 320
   ctg tcc aag atc ttc ctt ttt ttg cat tct tta gtt tta ttg atg aaa    1008
   Leu Ser Lys Ile Phe Leu Phe Leu His Ser Leu Val Leu Leu Met Lys
                   325                 330                 335
   atg cat tct ttc gcc ttc tac aat ggc tat cta tgg ggt ata aag gaa    1056
   Met His Ser Phe Ala Phe Tyr Asn Gly Tyr Leu Trp Gly Ile Lys Glu
               340                 345                 350
   gaa cta caa ttt tcc aaa agc gct ctt gcc aaa tac aag gat tct ata    1104
   Glu Leu Gln Phe Ser Lys Ser Ala Leu Ala Lys Tyr Lys Asp Ser Ile
           355                 360                 365
   aat gat cca aaa gtt att ggt gct ctt gag aaa agc tgt gag ttt tgt    1152
   Asn Asp Pro Lys Val Ile Gly Ala Leu Glu Lys Ser Cys Glu Phe Cys
       370                 375                 380
   agt ttt gaa ttg agc tct cag tct tta agc gac caa act caa aaa ttc    1200
   Ser Phe Glu Leu Ser Ser Gln Ser Leu Ser Asp Gln Thr Gln Lys Phe
   385                 390                 395                 400
   ccc aac aat atc agt gca aaa agc ttt ttt tgg ttc acc atg ttt cca    1248
   Pro Asn Asn Ile Ser Ala Lys Ser Phe Phe Trp Phe Thr Met Phe Pro
                   405                 410                 415
   acc cta att tac caa att gaa tat cca aga act aag gaa atc aga tgg    1296
   Thr Leu Ile Tyr Gln Ile Glu Tyr Pro Arg Thr Lys Glu Ile Arg Trp
               420                 425                 430
   agc tac gta tta gaa aag atc tgc gcc atc ttc ggt acc att ttc tta    1344
   Ser Tyr Val Leu Glu Lys Ile Cys Ala Ile Phe Gly Thr Ile Phe Leu
           435                 440                 445
   atg atg ata gat gct caa atc ttg atg tat cct gta gca atg aga gca    1392
   Met Met Ile Asp Ala Gln Ile Leu Met Tyr Pro Val Ala Met Arg Ala
       450                 455                 460
   ttg gct gtg cgc aat tct gaa tgg act ggt ata ttg gat aga tta ttg    1440
   Leu Ala Val Arg Asn Ser Glu Trp Thr Gly Ile Leu Asp Arg Leu Leu
   465                 470                 475                 480
   aaa tgg gtt gga ttg ctc gtt gat atc gtc cca ggg ttt atc gtg atg    1488
   Lys Trp Val Gly Leu Leu Val Asp Ile Val Pro Gly Phe Ile Val Met
                   485                 490                 495
   tac atc ttg gac ttc tat ttg att tgg gat gcc att ttg aac tgt gtg    1536
   Tyr Ile Leu Asp Phe Tyr Leu Ile Trp Asp Ala Ile Leu Asn Cys Val
               500                 505                 510
   gct gaa ttg aca aga ttt ggc gac aga tat ttc tac ggt gac tgg tgg    1584
   Ala Glu Leu Thr Arg Phe Gly Asp Arg Tyr Phe Tyr Gly Asp Trp Trp
           515                 520                 525
   aat tgt gtt agt tgg gca gac ttc agt aga att tgg aac atc cca gtg    1632
   Asn Cys Val Ser Trp Ala Asp Phe Ser Arg Ile Trp Asn Ile Pro Val
       530                 535                 540
   cat aag ttt ttg tta aga cat gtt tac cat agt tca atg agt tca ttc     1680
   His Lys Phe Leu Leu Arg His Val Tyr His Ser Ser Met Ser Ser Phe
   545                 550                 555                 560
   aaa ttg aac aag agt caa gca act ttg atg acc ttt ttc tta agt tcc     1728
   Lys Leu Asn Lys Ser Gln Ala Thr Leu Met Thr Phe Phe Leu Ser Ser
                   565                 570                 575
   gtc gtt cat gaa tta gca atg tac gtt atc ttc aag aaa ttg agg ttt     1776
   Val Val His Glu Leu Ala Met Tyr Val Ile Phe Lys Lys Leu Arg Phe
               580                 585                 590
   tac ttg ttc ttc ttc caa atg ctg caa atg cca tta gta gct tta aca     1824
   Tyr Leu Phe Phe Phe Gln Met Leu Gln Met Pro Leu Val Ala Leu Thr
           595                 600                 605
   aat act aaa ttc atg agg aac aga acc ata atc gga aat gtt att ttc     1872
   Asn Thr Lys Phe Met Arg Asn Arg Thr Ile Ile Gly Asn Val Ile Phe
       610                 615                 620
   tgg ctc ggt atc tgc atg gga cca agt gtc atg tgt acg ttg tac ttg     1920
   Trp Leu Gly Ile Cys Met Gly Pro Ser Val Met Cys Thr Leu Tyr Leu
   625                 630                 635                 640
   aca ttc taa                                                         1929
   Thr Phe
<210> 32
   <211> 642
   <212> ⁻PRT
   <213> Saccharomyces cerevisiae
<400> 32
<210> 33
   <211> 60
   <212> DNA
   <213> Artificial Sequence
<220>
   <223> Die Beschreibung von Knstliche Sequenz: Primer
<220>
   <221> primer_bind
   <222> (1)..(60)
<400> 33
   atgtcgaaag ctacatataa ggaacgtgct gcatctcatc ccagctgaag cttcgtacgc 60
<210> 34
   <211> 62
   <212> DNA
   <213> Artificial Sequence
<220>
   <223> Die Beschreibung von Knstliche Sequenz:Primer
<220>
   <221> primer_bind
   <222> (1)..(62)
<400> 34
   ttagttttgc tggccgcatc ttctcaaata tgcttcccag gcataggcca ctagtggatc 60
   tg 62
<210> 35
   <211> 60
   <212> DNA
   <213> Artificial Sequence
<220>
   <223> Die Beschreibung von Knstliche Sequenz: Primer
<220>
   <221> primer_bind
   <222> (1)..(60)
<400> 35
   gaatactcag gtatcgtaag atgcaagagt tcgaatctct ccagctgaag cttcgtacgc 60
<210> 36
   <211> 62
   <212> DNA
   <213> Artificial Sequence
<220>
   <223> Die Beschreibung von Knstliche Sequenz: Primer
<220>
   <221> primer_bind
   <222> (1)..(62)
<400> 36
   tctaccctat gaacatattc cattttgtaa tttcgtgtcg gcataggcca ctagtggatc 60
   tg 62
<210> 37
   <211> 60
   <212> DNA
   <213> Artificial Sequence
<220>
   <223> Die Beschreibung von Knstliche Sequenz: Primer
<220>
   <221> primer_bind
   <222> (1)..(60)
<400> 37
   atgacagagc agaaagccct agtaaagcgt attacaaatg ccagctgaag cttcgtacgc 60
<210> 38
   <211> 62
   <212> DNA
   <213> Artificial Sequence
<220>
   <223> Die Beschreibung von Knstliche Sequenz: Primer
<220>
   <221> primer_bind
   <222> (1)..(62)
<400> 38
   ctacataaga acacctttgg tggagggaac atcgttggta gcataggcca ctagtggatc 60
   tg 62
<210> 39
   <211> 60
   <212> DNA
   <213> Artificial Sequence
<220>
   <223> Die Beschreibung von Knstliche Sequenz: Primer
<220>
   <221> primer_bind
   <222> (1)..(60)
<400> 39
   atgagtgaaa cagaattgag aaaaagacag gcccaattca ccagctgaag cttcgtacgc 60
<210> 40
   <211> 62
   <212> DNA
   <213> Artificial Sequence
<220>
   <223> Die Beschreibung von Knstliche Sequenz: Primer
<220>
   <221> primer_bind
   <222> (1)..(62)
<400> 40
   ttattgagtt gcttcttggg aagtttggga gggggtttcg gcataggcca ctagtggatc 60
   tg 62
<210> 41
   <211> 60
   <212> DNA
   <213> Artificial Sequence
<220>
   <223> Die Beschreibung von Knstliche Sequenz: Primer
<220>
   <221> primer_bind
   <222> (1)..(60)
<400> 41
   atgagttctg tcgcagaaaa tataatacaa catgccactc ccagctgaag cttcgtacgc 60
<210> 42
   <211> 62
   <212> DNA
   <213> Artificial Sequence
<220>
   <223> Die Beschreibung von Knstliche Sequenz: Primer
<220>
   <221> primer_bind
   <222> (1)..(62)
<400> 42
   ttattcgaag acttctccag taattgggtc tctctttttg gcataggcca ctagtggatc 60
   tg 62
<210> 43
   <211> 33
   <212> DNA
   <213> Artificial Sequence
<220>
   <223> Die Beschreibung von Knstliche Sequenz: Primer
<220>
   <221> primer_bind
   <222> (1)..(33)
<400> 43
   ctgcggccgc aacatgacca ccaatacggt ccc 33
<210> 44
   <211> 27
   <212> DNA
   <213> Artificial Sequence
<220>
   <223> Die Beschreibung von Knstliche Sequenz: Primer
<220>
   <221> primer_bind
   <222> (1)..(27)
<400> 44
   ttctcgagtc tttagttatg cttgctc 27
<210> 45
   <211> 33
   <212> DNA
   <213> Artificial Sequence
<220>
   <223> Die Beschreibung von Knstliche Sequenz: Primer
<220>
   <221> primer_bind
   <222> (1)..(33)
<400> 45
   ctgcggccgc aagatggacc tggttctcag tgc 33
<210> 46
   <211> 29
   <212> DNA
   <213> Artificial Sequence
<220>
   <223> Die Beschreibung von Knstliche Sequenz: Primer
<220>
   <221> primer_bind
   <222> (1)..(29)
<400> 46
   ttctcgagct acttattctt tgtaaactc 29
<210> 47
   <211> 32
   <212> DNA
   <213> Artificial Sequence
<220>
   <223> Die Beschreibung von Knstliche Sequenz: Primer
<220>
   <221> primer_bind
   <222> (1)..(32)
<400> 47
   ctgcggccgc aagatggagc ccgccgtgtc gc 32
<210> 48
   <211> 28
   <212> DNA
   <213> Artificial Sequence
<220>
   <223> Die Beschreibung von Knstliche Sequenz: Primer
<220>
   <221> primer_bind
   <222> (1)..(28)
<400> 48
   aactcgagtc agtgccttgc cgccttgc 28
<210> 49
   <211> 1833
   <212> DNA
   <213> Saccharomyces cerevisiae
<220>
   <221> CDS
   <222> (1)..(1833)
<400> 49
   atg acg gag act aag gat ttg ttg caa gac gaa gag ttt ctt aag atc    48
   Met Thr Glu Thr Lys Asp Leu Leu Gln Asp Glu Glu Phe Leu Lys Ile
    1               5                  10                  15
   cgc aga ctc aat tcc gca gaa gcc aac aaa cgg cat tcg gtc acg tac    96
   Arg Arg Leu Asn Ser Ala Glu Ala Asn Lys Arg His Ser Val Thr Tyr
               20                  25                  30
   gat aac gtg atc ctg cca cag gag tcc atg gag gtt tcg cca cgg tcg   144
   Asp Asn Val Ile Leu Pro Gln Glu Ser Met Glu Val Ser Pro Arg Ser
           35                  40                  45
   tct acc acg tcg ctg gtg gag cca gtg gag tcg act gaa gga gtg gag   192
   Ser Thr Thr Ser Leu Val Glu Pro Val Glu Ser Thr Glu Gly Val Glu
       50                  55                  60
   tcg act gag gcg gaa cgt gtg gca ggg aag cag gag cag gag gag gag   240
   Ser Thr Glu Ala Glu Arg Val Ala Gly Lys Gln Glu Gln Glu Glu Glu
   65                  70                  75                  80
   tac cct gtg gac gcc cac atg caa aag tac ctt tca cac ctg aag agc   288
   Tyr Pro Val Asp Ala His Met Gln Lys Tyr Leu Ser His Leu Lys Ser
                   85                  90                  95
   aag tct cgg tcg agg ttc cac cga aag gat gct agc aag tat gtg tcg     336
   Lys Ser Arg Ser Arg Phe His Arg Lys Asp Ala Ser Lys Tyr Val Ser
                   100                 105                 110
   ttt ttt ggg gac gtg agt ttt gat cct cgc ccc acg ctc ctg gac agc     384
   Phe Phe Gly Asp Val Ser Phe Asp Pro Arg Pro Thr Leu Leu Asp Ser
               115                 120                 125
   gcc atc aac gtg ccc ttc cag acg act ttc aaa ggt ccg gtg ctg gag     432
   Ala Ile Asn Val Pro Phe Gln Thr Thr Phe Lys Gly Pro Val Leu Glu
       130                 135                 140
   aaa cag ctc aaa aat tta cag ttg aca aag acc aag acc aag gcc acg     480
   Lys Gln Leu Lys Asn Leu Gln Leu Thr Lys Thr Lys Thr Lys Ala Thr
   145                 150                 155                 160
   gtg aag act acg gtg aag act acg gag aaa acg gac aag gca gat gcc     528
   Val Lys Thr Thr Val Lys Thr Thr Glu Lys Thr Asp Lys Ala Asp Ala
                   165                 170                 175
   ccc cca gga gaa aaa ctg gag tcg aac ttt tca ggg atc tac gtg ttc     576
   Pro Pro Gly Glu Lys Leu Glu Ser Asn Phe Ser Gly Ile Tyr Val Phe
               180                 185                 190
   gca tgg atg ttc ttg ggc tgg ata gcc atc agg tgc tgc aca gat tac     624
   Ala Trp Met Phe Leu Gly Trp Ile Ala Ile Arg Cys Cys Thr Asp Tyr
           195                 200                 205
   tat gcg tcg tac ggc agt gca tgg aat aag ctg gaa atc gtg cag tac     672
   Tyr Ala Ser Tyr Gly Ser Ala Trp Asn Lys Leu Glu Ile Val Gln Tyr
       210                 215                 220
   atg aca acg gac ttg ttc acg atc gca atg ttg gac ttg gca atg ttc     720
   Met Thr Thr Asp Leu Phe Thr Ile Ala Met Leu Asp Leu Ala Met Phe
   225                 230                 235                 240
   ctg tgc act ttc ttc gtg gtt ttc gtg cac tgg ctg gtg aaa aag cgg     768
   Leu Cys Thr Phe Phe Val Val Phe Val His Trp Leu Val Lys Lys Arg
                   245                 250                 255
   atc atc aac tgg aag tgg act ggg ttc gtt gca gtg agc atc ttc gag     816
   Ile Ile Asn Trp Lys Trp Thr Gly Phe Val Ala Val Ser Ile Phe Glu
               260                 265                 270
   ttg gct ttc atc ccc gtg acg ttc ccc att tac gtc tac tac ttt gat     864
   Leu Ala Phe Ile Pro Val Thr Phe Pro Ile Tyr Val Tyr Tyr Phe Asp
           275                 280                 285
   ttc aac tgg gtc acg aga atc ttc ctg ttc ctg cac tcc gtg gtg ttt     912
   Phe Asn Trp Val Thr Arg Ile Phe Leu Phe Leu His Ser Val Val Phe
       290                 295                 300
   gtt atg aag agc cac tcg ttt gcc ttt tac aac ggg tat ctt tgg gac     960
   Val Met Lys Ser His Ser Phe Ala Phe Tyr Asn Gly Tyr Leu Trp Asp
   305                 310                 315                 320
   ata aag cag gaa ctc gag tac tct tcc aaa cag ttg caa aaa tac aag    1008
   Ile Lys Gln Glu Leu Glu Tyr Ser Ser Lys Gln Leu Gln Lys Tyr Lys
                   325                 330                 335
   gaa tct ttg tcc cca gag acc cgc gag att ctg caa aaa agt tgc gac    1056
   Glu Ser Leu Ser Pro Glu Thr Arg Glu Ile Leu Gln Lys Ser Cys Asp
               340                 345                 350
   ttt tgc ctt ttc gaa ttg aac tac cag acc aag gat aac gac ttc ccc    1104
   Phe Cys Leu Phe Glu Leu Asn Tyr Gln Thr Lys Asp Asn Asp Phe Pro
           355                 360                 365
   aac aac atc agt tgc agc aat ttc ttc atg ttc tgt ttg ttc ccc gtc    1152
   Asn Asn Ile Ser Cys Ser Asn Phe Phe Met Phe Cys Leu Phe Pro Val
       370                 375                 380
   ctc gtg tac cag atc aac tac cca aga acg tcg cgc atc aga tgg agg    1200
   Leu Val Tyr Gln Ile Asn Tyr Pro Arg Thr Ser Arg Ile Arg Trp Arg
   385                 390                 395                 400
   tat gtg ttg gag aag gtg tgc gcc atc att ggc acc atc ttc ctc atg    1248
   Tyr Val Leu Glu Lys Val Cys Ala Ile Ile Gly Thr Ile Phe Leu Met
                   405                 410                 415
   atg gtc acg gca cag ttc ttc atg cac ccg gtg gcc atg cgc tgt atc    1296
   Met Val Thr Ala Gln Phe Phe Met His Pro Val Ala Met Arg Cys Ile
               420                 425                 430
   cag ttc cac aac acg ccc acc ttc ggc ggc tgg atc ccc gcc acg caa    1344
   Gln Phe His Asn Thr Pro Thr Phe Gly Gly Trp Ile Pro Ala Thr Gln
           435                 440                 445
   gag tgg ttc cac ctg ctc ttc gac atg att ccg ggc ttc act gtt ctg    1392
   Glu Trp Phe His Leu Leu Phe Asp Met Ile Pro Gly Phe Thr Val Leu
       450                 455                 460
   tac atg ctc acg ttt tac atg ata tgg gac gct tta ttg aat tgc gtg    1440
   Tyr Met Leu Thr Phe Tyr Met Ile Trp Asp Ala Leu Leu Asn Cys Val
   465                 470                 475                 480
   gcg gag ttg acc agg ttt gcg gac aga tat ttc tac ggc gac tgg tgg    1488
   Ala Glu Leu Thr Arg Phe Ala Asp Arg Tyr Phe Tyr Gly Asp Trp Trp
                   485                 490                 495
   aat tgc gtt tcg ttt gaa gag ttt agc aga atc tgg aac gtc ccc gtt    1536
   Asn Cys Val Ser Phe Glu Glu Phe Ser Arg Ile Trp Asn Val Pro Val
               500                 505                 510
   cac aaa ttt tta cta aga cac gtg tac cac agc tcc atg ggc gca ttg    1584
   His Lys Phe Leu Leu Arg His Val Tyr His Ser Ser Met Gly Ala Leu
           515                 520                 525
   cat ttg agc aag agc caa gct aca tta ttt act ttt ttc ttg agt gcc    1632
   His Leu Ser Lys Ser Gln Ala Thr Leu Phe Thr Phe Phe Leu Ser Ala
       530                 535                 540
   gtg ttc cac gaa atg gcc atg ttc gcc att ttc aga agg gtt aga gga    1680
   Val Phe His Glu Met Ala Met Phe Ala Ile Phe Arg Arg Val Arg Gly
   545                 550                 555                 560
   tat ctg ttc atg ttc caa ctg tcg cag ttt gtg tgg act gct ttg agc     1728
   Tyr Leu Phe Met Phe Gln Leu Ser Gln Phe Val Trp Thr Ala Leu Ser
                   565                 570                 575
   aac acc aag ttt cta cgg gca aga ccg cag ttg tcc aac gtt gtc ttt     1776
   Asn Thr Lys Phe Leu Arg Ala Arg Pro Gln Leu Ser Asn Val Val Phe
               580                 585                 590
   tcg ttt ggt gtc tgt tca ggg ccc agt atc att atg acg ttg tac ctg     1824
   Ser Phe Gly Val Cys Ser Gly Pro Ser Ile Ile Met Thr Leu Tyr Leu
           595                 600                 605
   acc tta tga                                                         1833
   Thr Leu
       610
<210> 50
   <211> 610
   <212> PRT
   <213> Saccharomyces cerevisiae
<400> 50
   Met Thr Glu Thr Lys Asp Leu Leu Gln Asp Glu Glu Phe Leu Lys Ile
    1               5                  10                  15
   Arg Arg Leu Asn Ser Ala Glu Ala Asn Lys Arg His Ser Val Thr Tyr
               20                  25                  30
   Asp Asn Val Ile Leu Pro Gln Glu Ser Met Glu Val Ser Pro Arg Ser
           35                  40                  45
   Ser Thr Thr Ser Leu Val Glu Pro Val Glu Ser Thr Glu Gly Val Glu
       50                  55                  60
   Ser Thr Glu Ala Glu Arg Val Ala Gly Lys Gln Glu Gln Glu Glu Glu
   65                  70                  75                  80
   Tyr Pro Val Asp Ala His Met Gln Lys Tyr Leu Ser His Leu Lys Ser
                   85                  90                  95
   Lys Ser Arg Ser Arg Phe His Arg Lys Asp Ala Ser Lys Tyr Val Ser
               100                 105                 110
   Phe Phe Gly Asp Val Ser Phe Asp Pro Arg Pro Thr Leu Leu Asp Ser
           115                 120                 125
   Ala Ile Asn Val Pro Phe Gln Thr Thr Phe Lys Gly Pro Val Leu Glu
       130                 135                 140
   Lys Gln Leu Lys Asn Leu Gln Leu Thr Lys Thr Lys Thr Lys Ala Thr
   145                 150                 155                 160
   Val Lys Thr Thr Val Lys Thr Thr Glu Lys Thr Asp Lys Ala Asp Ala
   165 170 175
   Pro Pro Gly Glu Lys Leu Glu Ser Asn Phe Ser Gly Ile Tyr Val Phe
   180 185 190
   Ala Trp Met Phe Leu Gly Trp Ile Ala Ile Arg Cys Cys Thr Asp Tyr
   195 200 205
   Tyr Ala Ser Tyr Gly Ser Ala Trp Asn Lys Leu Glu Ile Val Gln Tyr
   210 215 220
   Met Thr Thr Asp Leu Phe Thr Ile Ala Met Leu Asp Leu Ala Met Phe
   225 230 235 240
   Leu Cys Thr Phe Phe Val Val Phe Val His Trp Leu Val Lys Lys Arg
   245 250 255
   Ile Ile Asn Trp Lys Trp Thr Gly Phe Val Ala Val Ser Ile Phe Glu
   260 265 270
   Leu Ala Phe Ile Pro Val Thr Phe Pro Ile Tyr Val Tyr Tyr Phe Asp
   275 280 285
   Phe Asn Trp Val Thr Arg Ile Phe Leu Phe Leu His Ser Val Val Phe
   290 295 300
   Val Met Lys Ser His Ser Phe Ala Phe Tyr Asn Gly Tyr Leu Trp Asp
   305 310 315 320
   Ile Lys Gln Glu Leu Glu Tyr Ser Ser Lys Gln Leu Gln Lys Tyr Lys
   325 330 335
   Glu Ser Leu Ser Pro Glu Thr Arg Glu Ile Leu Gln Lys Ser Cys Asp
   340 345 350
   Phe Cys Leu Phe Glu Leu Asn Tyr Gln Thr Lys Asp Asn Asp Phe Pro
   355 360 365
   Asn Asn Ile Ser Cys Ser Asn Phe Phe Met Phe Cys Leu Phe Pro Val
   370 375 380
   Leu Val Tyr Gln Ile Asn Tyr Pro Arg Thr Ser Arg Ile Arg Trp Arg
   385 390 395 400
   Tyr Val Leu Glu Lys Val Cys Ala Ile Ile Gly Thr Ile Phe Leu Met
   405 410 415
   Met Val Thr Ala Gln Phe Phe Met His Pro Val Ala Met Arg Cys Ile
   420 425 430
   Gln Phe His Asn Thr Pro Thr Phe Gly Gly Trp Ile Pro Ala Thr Gln
   435 440 445
   Glu Trp Phe His Leu Leu Phe Asp Met Ile Pro Gly Phe Thr Val Leu
   450 455 460
   Tyr Met Leu Thr Phe Tyr Met Ile Trp Asp Ala Leu Leu Asn Cys Val
   465 470 475 480
   Ala Glu Leu Thr Arg Phe Ala Asp Arg Tyr Phe Tyr Gly Asp Trp Trp
   485 490 495
   Asn Cys Val Ser Phe Glu Glu Phe Ser Arg Ile Trp Asn Val Pro Val
   500 505 510
   His Lys Phe Leu Leu Arg His Val Tyr His Ser Ser Met Gly Ala Leu
   515 520 525
   His Leu Ser Lys Ser Gln Ala Thr Leu Phe Thr Phe Phe Leu Ser Ala
   530 535 540
   Val Phe His Glu Met Ala Met Phe Ala Ile Phe Arg Arg Val Arg Gly
   545 550 555 560
   Tyr Leu Phe Met Phe Gln Leu Ser Gln Phe Val Trp Thr Ala Leu Ser
   565 570 575
   Asn Thr Lys Phe Leu Arg Ala Arg Pro Gln Leu Ser Asn Val Val Phe
   580 585 590
   Ser Phe Gly Val Cys Ser Gly Pro Ser Ile Ile Met Thr Leu Tyr Leu
   595 600 605
   Thr Leu
   610
<210> 51
   <211> 33
   <212> DNA
   <213> Artificial Sequence
<220>
   <223> Die Beschreibung von Knstliche Sequenz: Primer
<220>
   <221> primer_bind
   <222> (1)..(33)
<400> 51
   ctgcggccgc atcatgtctg ctgttaacgt tgc 33
<210> 52
   <211> 30
   <212> DNA
   <213> Artificial Sequence
<220>
   <223> Die Beschreibung von Knstliche Sequenz: Primer
<220>
   <221> primer_bind
   <222> (1).. (30)
<400> 52
   ttctcgagtt aaccaatcaa ctcaccaaac 30
<210> 53
   <211> 35
   <212> DNA
   <213> Artificial Sequence
<220>
   <223> Die Beschreibung von Knstliche Sequenz: Primer
<220>
   <221> primer_bind
   <222> (1).. (35)
<400> 53
   ctgcggccgc aggatgtctg ctaccaagtc aatcg 35
<210> 54
   <211> 34
   <212> DNA
   <213> Artificial Sequence
<220>
   <223> Die Beschreibung von Knstliche Sequenz: Primer
<220>
   <221> primer_bind
   <222> (1)..(34)
<400> 54
   atctcgagct tagatctttt gttctggatt tctc 34
<210> 55
   <211> 32
   <212> DNA
   <213> Artificial Sequence
<220>
   <223> Die Beschreibung von Knstliche Sequenz: Primer
<220>
   <221> primer_bind
   <222> (1).. (32)
<400> 55
   ctgcggccgc accatgaagt ttttcccact cc 32
<210> 56
   <211> 33
   <212> DNA
   <213> Artificial Sequence
<220>
   <223> Die Beschreibung von Knstliche Sequenz: Primer
<220>
   <221> primer_bind
   <222> (1).. (33)
<400> 56
   ttctcgagtt agaacttttt gttttgcaac aag 33
<210> 57
   <211> 35
   <212> DNA
   <213> Artificial Sequence
<220>
   <221> primer_bind
   <222> (1)..(35)
<220>
   <223> Die Beschreibung von Knstliche Sequenz: Primer
<400> 57
   ctgcggccgc aatatggatt tggtcttaga agtcg 35
<210> 58
   <211> 31
   <212> DNA
   <213> Artificial Sequence
<220>
   <223> Die Beschreibung von Knstliche Sequenz: Primer
<220>
   <221> primer_bind
   <222> (1)..(31)
<400> 58
   aactcgagtc agttgttctt cttggtattt g 31
<210> 59
   <211> 34
   <212> DNA
   <213> Artificial Sequence
<220>
   <223> Die Beschreibung von Knstliche Sequenz: Primer
<220>
   <221> primer_bind
   <222> (1)..(34)
<400> 59
   ctgcggccgc actatggcaa aggataatag tgag 34
<210> 60
   <211> 32
   <212> DNA
   <213> Artificial Sequence
<220>
   <223> Die Beschreibung von Knstliche Sequenz: Primer
<220>
   <221> primer_bind
   <222> (1)..(32)
<400> 60
   ttctcgagct agaaaacata aggaataaag ac 32